# EUROPEAN PATENT SPECIFICATION

(11) **EP 2 316 940 B1**
(45) Date of publication and mention of the grant of the patent: **24.07.2013**
(21) Application number: 10182768.1
(22) Date of filing: 18.12.2001
(51) Int. Cl.: C12N 15/10, C12N 15/13, C07K 16/00, C12P 21/00, C12N 15/09, C40B 40/02

(54) **Focused libraries of genetic packages**
Gerichtete Bibliotheken, die genetisch verpackt sind
Banques ciblées de capsides génétiques

(30) Priority: 18.12.2000 US 256380 P
(43) Date of publication of application: 04.05.2011
(62) Divisional of application: 01998098.6
(73) Proprietor: Dyax Corp., Cambridge, MA 02139 (US)
(72) Inventor: Ladner, Robert Charles, Ijamsville, MD 21754 (US)
(74) Representative: Wichmann, Hendrik

(56) References cited:
- WO-A-97/08320
- WO-A2-99/06834
- KNAPPIK A ET AL: "FULLY SYNTHESIS HUMAN COMBINATORIAL ANTIBODY LIBRARIES (HUCAL) BASED ON MODULAR CONSENSUS FRAMEWORKS AND CDRS RANDOMIZED WITH TRINUCLEOTIDES", JOURNAL OF MOLECULAR BIOLOGY, LONDON, GB, vol. 296, no. 1, 2000, pages 57-86, XP000939143, ISSN: 0022-2836
- AUJAME L ET AL: "High affinity human antibodies by phage display", HUMAN ANTIBODIES, AMSTERDAM, NL, vol. 8, no. 4, 1997, pages 155-168, XP002105424, ISSN: 1093-2607
- BARBAS C F ET AL: "SEMISYNTHETIC COMBINATORIAL ANTIBODY LIBRARIES: A CHEMICAL SOLUTION TO THE DIVERSITY PROBLEM", PROCEEDINGS OF THE NATIONAL ACADEMY OF SCIENCES OF USA, NATIONAL ACADEMY OF SCIENCE. WASHINGTON, US, vol. 89, 1 May 1992 (1992-05-01), pages 4457-4461, XP002024223, ISSN: 0027-8424
- KRUIF DE J ET AL: "SELECTION AND APPLICATION OF HUMAN SINGLE CHAIN FV ANTIBODY FRAGMENTS FROM A SEMI-SYNTHETIC PHAGE ANTOBODY DISPLEY LIBRARY WITHDESIGNED CDR3 REGIONS", JOURNAL OF MOLECULAR BIOLOGY, LONDON, GB, vol. 248, no. 1, 21 April 1995 (1995-04-21), pages 97-105, XP000646544, ISSN: 0022-2836
- POWELL R ET AL: "CONSTRUCTION, ASSEMBLY AND SELECTION OF COMBINATORIAL ANTIBODY LIBRARIES", GENETIC ENGINEERING WITH PCR, XX, XX, vol. 5, 1998, pages 155-172, XP000981992,
- HOOGENBOOM H R ET AL: "Antibody phage display technology and its applications", IMMUNOTECHNOLOGY, ELSEVIER SCIENCE PUBLISHERS BV, NL, vol. 4, no. 1, 1 June 1998 (1998-06-01), pages 1-20, XP004127382, ISSN: 1380-2933

### SEQUENCE LISTING

<110> DYAX CORPORATION
<120> FOCUSED LIBRARIES OF GENETIC PACKAGES
<130> DYAX/004PCT
<140> PCT/US01/50297
   <141> 2001-12-18
<160> 99
<170> PatentIn Ver. 2.1
<210> 1
   <211> 14
   <212> PRT
   <213> Artificial sequence
<220>
   <223> Description of Artificial sequence: Heavy chain CDR1 vector
<220>
   <221> MOD_RES
   <222> (8)..(10)
   <223> Any amino acid except Cys
<220>
   <221> MOD_RES
   <222> (14)
   <223> Any amino acid except Cys
<400> 1
   Val Ser Gly Gly Ser Ile Ser Xaa Xaa Xaa Tyr Tyr Trp Xaa
   1               5                   10
<210> 2
   <211> 17
   <212> PRT
   <213> Artificial Sequence
<220>
   <223> Description of Artificial sequence: Heavy chain CDR2 vector
<220>
   <221> MOD_RES
   <222> (1)
   <223> Tyr, Arg, Trp, val Gly or Ser
<220>
   <221> MOD_RES
   <222> (3)
   <223> Tyr, Arg, Trp, Val, Gly or Ser
<220>
   <221> MOD_RES
   <222> (4)
   <223> Pro, Ser or Gly
<220>
   <221> MOD_RES
   <222> (8)
   <223> Any amino acid except Cys
<220>
   <221> MOD_RES
   <222> (10)
   <223> Any amino acid except Cys
<400> 2
   Xaa Ile Xaa Xaa Ser Gly Gly Xaa Thr Xaa Tyr Ala Asp Ser Val Lys
   1               5                   10                  15
   Gly
<210> 3
   <211> 17
   <212> PRT
   <213> Artificial sequence
<220>
   <223> Description of Artificial sequence: Heavy chain CDR2 vector
<220>
   <221> MOD_RES
   <222> (1)
   <223> Any amino acid except Cys
<220>
   <221> MOD_RES
   <222> (3)
   <223> Asp, Ile, Asn, Ser, Trp or Tyr
<220>
   <221> MOD_RES
   <222> (4)..(5)
   <223> Any amino acid except cys
<220>
   <221> MOD_RES
   <222> (7)
   <223> Ser, Gly, Asp or Asn
<220>
   <221> MOD_RES
   <222> (8)..(10)
   <223> Any amino acid except Cys
<400> 3
   Xaa Ile Xaa Xaa Xaa Gly Xaa Xaa Xaa Xaa Tyr Ala Asp Ser Val Lys
   1               5                   10                  15
   Gly
<210> 4
   <211> 16
   <212> PRT
   <213> Artificial Sequence
<220>
   <223> Description of Artificial Sequence: Heavy chain CDR2 vector
<220>
   <221> MOD_RES
   <222> (1)
   <223> Any amino acid except cys
<220>
   <221> MOD_RES
   <222> (3)
   <223> Asp, Ile, Asn, Ser, Trp or Tyr
<220>
   <221> MOD_RES
   <222> (4)..(5)
   <223> Any amino acid except Cys
<220>
   <221> MOD_RES
   <222> (7)
   <223> Ser, Gly, Asp or Asn
<220>
   <221> MOD_RES
   <222> (8)..(9)
   <223> Any amino acid except Cys
<400> 4
   Xaa Ile Xaa Xaa Xaa Gly Xaa Xaa Xaa Tyr Asn Pro Ser Leu Lys Gly
   1               5                   10                  15
<210> 5
   <211> 19
   <212> PRT
   <213> Artificial Sequence
<220>
   <223> Description of Artificial Sequence: Heavy chain CDR2 vector
<220>
   <221> MOD_RES
   <222> (1)
   <223> Any amino acid except cys
<220>
   <221> MOD_RES
   <222> (3)
   <223> Any amino acid except Cys
<220>
   <221> MOD_RES
   <222> (5)..(7)
   <223> Any amino acid except Cys
<220>
   <221> MOD_RES
   <222> (12)
   <223> Any amino acid except Cys
<400> 5
   Xaa Ile Xaa Ser Xaa Xaa Xaa Gly Gly Tyr Tyr Xaa Tyr Ala Ala Ser
   1 5 10 15
   Val Lys Gly
<210> 6
   <211> 15
   <212> PRT
   <213> Artificial Sequence
<220>
   <223> Description of Artificial sequence: Heavy chain CDR3 vector
<220>
   <221> MOD_RES
   <222> (5)
   <223> Lys or Arg
<220>
   <221> MOD_RES
   <222> (6)..(9)
   <223> Any amino acid except Cys
<400> 6
   Tyr Tyr Cys Ala Xaa Xaa Xaa Xaa Xaa Tyr Phe Asp Tyr Trp Gly
   1 5 10 15
<210> 7
   <211> 17
   <212> PRT
   <213> Artificial Sequence
<220>
   <223> Description of Artificial sequence: Heavy chain CDR3 vector
<220>
   <221> MOD_RES
   <222> (5)
   <223> Lys or Arg
<220>
   <221> MOD_RES
   <222> (6)..(11)
   <223> Any amino acid except Cys
<400> 7
   Tyr Tyr Cys Ala Xaa Xaa Xaa Xaa Xaa Xaa Xaa Tyr Phe Asp Tyr Trp
   1 5 10 15
   Gly
<210> 8
   <211> 19
   <212> PRT
   <213> Artificial Sequence
<220>
   <223> Description of Artificial Sequence: Heavy chain CDR3 vector
<220>
   <221> MOD_RES
   <222> (5)
   <223> Lys or Arg
<220>
   <221> MOD_RES
   <222> (6)..(13)
   <223> Any amino acid except Cys
<400> 8
   Tyr Tyr Cys Ala Xaa Xaa Xaa Xaa Xaa Xaa Xaa Xaa Xaa Tyr Phe Asp
   1               5                   10                  15
   Tyr Trp Gly
<210> 9
   <211> 21
   <212> PRT
   <213> Artificial Sequence
<220>
   <223> Description of Artificial Sequence: Heavy chain CDR3 vector
<220>
   <221> MOD_RES
   <222> (6)..(8)
   <223> Any amino acid except Cys
<220>
   <221> MOD_RES
   <222> (10)
   <223> Ser or Gly
<220>
   <221> MOD_RES
   <222> (12)
   <223> Tyr or Trp
<220>
   <221> MOD_RES
   <222> (13)..(15)
   <223> Any amino acid except Cys
<400> 9
   Tyr Tyr Cys Ala Arg Xaa Xaa Xaa Ser Xaa Ser Xaa Xaa Xaa Xaa Tyr
   1               5                   10                  15
   Phe Asp Tyr Trp Gly
               20
<210> 10
   <211> 22
   <212> PRT
   <213> Artificial Sequence
<220>
   <223> Description of Artificial sequence: Heavy chain CDR3 vector
<220>
   <221> MOD_RES
   <222> (5)
   <223> Lys or Arg
<220>
   <221> MOD_RES
   <222> (6)..(8)
   <223> Any amino acid except Cys
<220>
   <221> MOD_RES
   <222> (12)..(13)
   <223> Any amino acid except Cys
<220>
   <221> MOD_RES
   <222> (16)
   <223> Any amino acid except Cys
<400> 10
   Tyr Tyr Cys Ala Xaa Xaa Xaa Xaa Cys Ser Gly Xaa Xaa Cys Tyr Xaa
   1               5                   10                  15
   Tyr Phe Asp Tyr Trp Gly
               20
<210> 11
   <211> 24
   <212> PRT
   <213> Artificial Sequence
<220>
   <223> Description of Artificial Sequence: Heavy chain CDR3 vector
<220>
   <221> MOD_RES
   <222> (5)
   <223> Lys or Arg
<220>
   <221> MOD_RES
   <222> (6)..(7)
   <223> Any amino acid except cys
<220>
   <221> MOD_RES
   <222> (9)
   <223> Any amino acid except Cys
<220>
   <221> MOD_RES
   <222> (14)..(18)
   <223> Any amino acid except Cys
<400> 11
   Tyr Tyr Cys Ala Xaa Xaa Xaa Ser Xaa Thr Ile Phe Gly Xaa Xaa Xaa
   1               5                   10                  15
   Xaa Xaa Tyr Phe Asp Tyr Trp Gly
               20
<210> 12
   <211> 25
   <212> PRT
   <213> Artificial sequence
<220>
   <223> Description of Artificial Sequence: Heavy chain CDR3 vector
<220>
   <221> MOD_RES
   <222> (6)..(8)
   <223> Any amino acid except Cys
<220>
   <221> MOD_RES
   <222> (11)
   <223> Asp or Gly
<220>
   <221> MOD_RES
   <222> (13)..(14)
   <223> Ser or Gly
<220>
   <221> MOD_RES
   <222> (15)..(16)
   <223> Any amino acid
<220>
   <221> MOD_RES
   <222> (17)..(19)
   <223> Any amino acid except cys
<400> 12
   Tyr Tyr Cys Ala Arg Xaa Xaa Xaa Tyr Tyr Xaa Ser Xaa Xaa Xaa Xaa
   1               5                   10                  15
   Xaa Xaa Xaa Tyr Phe Asp Tyr Trp Gly
               20                  25
<210> 13
   <211> 26
   <212> PRT
   <213> Artificial Sequence
<220>
   <223> Description of Artificial Sequence: Heavy chain CDR3 vector
<220>
   <221> MOD_RES
   <222> (6)..(9)
   <223> Any amino acid except Cys
<220>
   <221> MOD_RES
   <222> (12)..(13)
   <223> Ser or Gly
<220>
   <221> MOD_RES
   <222> (14)
   <223> Thr, Asp or Gly
<220>
   <221> MOD_RES
   <222> (15)
   <223> Any amino acid except Cys
<220>
   <221> MOD_RES
   <222> (18)..(20)
   <223> Any amino acid except Cys
<400> 13
   Tyr Tyr Cys Ala Arg Xaa Xaa Xaa Xaa Tyr Cys Xaa Xaa Xaa Xaa Cys
    1               5                  10                  15
   Tyr Xaa Xaa Xaa Tyr Phe Asp Tyr Trp Gly
               20                  25
<210> 14
   <211> 11
   <212> PRT
   <213> Artificial sequence
<220>
   <223> Description of Artificial sequence: Kappa light chain CDR1 vector
<220>
   <221> MOD_RES
   <222> (5)
   <223> Any amino acid except Cys
<220>
   <221> MOD_RES
   <222> (7)..(8)
   <223> Any amino acid except Cys
<220>
   <221> MOD_RES
   <222> (9)
   <223> Any amino acid except Cys and Ser
<400> 14
   Arg Ala Ser Gln Xaa Val Xaa Xaa Xaa Leu Ala
   1 5 10
<210> 15
   <211> 12
   <212> PRT
   <213> Artificial sequence
<220>
   <223> Description of Artificial Sequence: Kappa light chain CDR1 vector
<220>
   <221> MOD_RES
   <222> (5)
   <223> Any amino acid except Cys
<220>
   <221> MOD_RES
   <222> (7)..(9)
   <223> Any amino acid except Cys
<220>
   <221> MOD_RES
   <222> (10)
   <223> Any amino acid except Cys and Ser
<400> 15
   Arg Ala Ser Gln Xaa Val Xaa Xaa Xaa Xaa Leu Ala
   1 5 10
<210> 16
   <211> 9
   <212> PRT
   <213> Artificial sequence
<220>
   <223> Description of Artificial Sequence: Kappa light chain CDR3 vector
<220>
   <221> MOD_RES
   <222> (3)
   <223> Any amino acid except Cys or Ser
<220>
   <221> MOD_RES
   <222> (4)..(6)
   <223> Any amino acid except Cys
<220>
   <221> MOD_RES
   <222> (8)
   <223> Any amino acid except Cys
<400> 16
   Gln Gln Xaa Xaa Xaa Xaa Pro Xaa Thr
   1 5
<210> 17
   <211> 10
   <212> PRT
   <213> Artificial Sequence
<220>
   <223> Description of Artificial Sequence: Kappa light chain CDR3 vector
<220>
   <221> MOD_RES
   <222> (3)
   <223> Any amino acid except cys or Ser
<220>
   <221> MOD_RES
   <222> (4)
   <223> Any amino acid except Cys
<220>
   <221> MOD_RES
   <222> (5)..(6)
   <223> Any amino acid except cys
<220>
   <221> MOD_RES
   <222> (9)
   <223> Any amino acid except Cys
<400> 17
   Gln Gln Xaa Xaa Xaa Xaa Pro Pro Xaa Thr
   1 5 10
<210> 18
   <211> 14
   <212> PRT
   <213> Artificial Sequence
<220>
   <223> Description of Artificial sequence: Lambda light chain CDR1 vector
<220>
   <221> MOD_RES
   <222> (3)
   <223> Any amino acid except Cys
<220>
   <221> MOD_RES
   <222> (6)
   <223> Any amino acid except Cys
<220>
   <221> MOD_RES
   <222> (9)..(12)
   <223> Any amino acid except Cys
<400> 18
   Thr Gly Xaa Ser Ser Xaa Val Gly Xaa Xaa Xaa Xaa Val Ser
   1 5 10
<210> 19
   <211> 10
   <212> PRT
   <213> Artificial Sequence
<220>
   <223> Description of Artificial Sequence: Lambda light chain CDR3 vector
<220>
   <221> MOD_RES
   <222> (1)
   <223> Any amino acid except Cys
<220>
   <221> MOD_RES
   <222> (4)..(5)
   <223> Any amino acid except cys
<220>
   <221> MOD_RES
   <222> (7)..(8)
   <223> Any amino acid except Cys
<220>
   <221> MOD_RES
   <222> (9)
   <223> Any amino acid except cys or Tyr
<400> 19
   Xaa Ser Tyr Xaa Xaa Ser Xaa Xaa Xaa Val
   1 5 10
<210> 20
   <211> 14
   <212> PRT
   <213> Homo sapiens
<400> 20
   Tyr Phe Asp Tyr Trp Gly Gln Gly Thr Leu Val Thr Ser Ser
   1 5 10
<210> 21
   <211> 15
   <212> PRT
   <213> Homo sapiens
<400> 21
   Ala Phe Asp Ile Trp Gly Gln Gly Thr Met Val Thr Val Ser Ser
   1 5 10 15
<210> 22
   <211> 5
   <212> PRT
   <213> Artificial sequence
<220>
   <223> Description of Artificial Sequence: synthetic peptide
<400> 22
   Tyr Tyr Cys Ala Arg
   1               5
<210> 23
   <211> 323
   <212> DNA
   <213> Artificial sequence
<220>
   <223> Description of Artificial Sequence: 3-23: JH4 vector with CDR1/2 diversity
<220>
   <221> CDS
   <222> (3)..(323)
<220>
   <221> modified_base
   <222> (99)..(101)
   <223> a, c, t, g, other or unknown
<220>
   <221> modified_base
   <222> (105)..(107)
   <223> a, c, t, g, other or unknown
<220>
   <221> modified_base
   <222> (111)..(113)
   <223> a, c, t, g, other or unknown
<220>
   <221> modified_base
   <222> (156)..(158)
   <223> a, c, t, g, other or unknown
<220>
   <221> modified_base
   <222> (162)..(167)
   <223> a, c, t, g, other or unknown
<220>
   <221> modified_base
   <222> (177)..(179)
   <223> a, c, t, g, other or unknown
<220>
   <221> modified_base
   <222> (183)..(185)
   <223> a, c, t, g, other or unknown
<400> 23
    cc atg gcc gaa gtt caa ttg tta gag tct ggt ggc ggt ctt gtt cag       47
   Ala Met Ala Glu Val Gln Leu Leu Glu Ser Gly Gly Gly Leu Val Gln
    1               5                  10                  15
   cct ggt ggt tct tta cgt ctt tct tgc gct gct tcc gga ttc act ttc      95
   Pro Gly Gly Ser Leu Arg Leu Ser Cys Ala Ala Ser Gly Phe Thr Phe
               20                  25                  30
   tct nnn tac nnn atg nnn tgg gtt cgc caa gct cct ggt aaa ggt ttg     143
   Ser Xaa Tyr Xaa Met Xaa Trp Val Arg Gln Ala Pro Gly Lys Gly Leu
           35                  40                  45
   gag tgg gtt tct nnn atc nnn nnn tct ggt ggc nnn act nnn tat gct     191
   Glu Trp Val Ser Xaa Ile Xaa Xaa Ser Gly Gly Xaa Thr Xaa Tyr Ala
       50                  55                  60
   gac tcc gtt aaa ggt cgc ttc act atc tct aga gac aac tct aag aat     239
   Asp Ser Val Lys Gly Arg Phe Thr Ile Ser Arg Asp Asn Ser Lys Asn
    65                  70                  75                  80
   act ctc tac ttg cag atg aac agc tta agg gct gag gac acc gct gtc     287
   Thr Leu Tyr Leu Gln Met Asn Ser Leu Arg Ala Glu Asp Thr Ala Val
                   85                  90                  95
   tac tac tgc gcc aaa gac tat gaa ggt act ggt tat                     323
   Tyr Tyr Cys Ala Lys Asp Tyr Glu Gly Thr Gly Tyr
               100                 105
<210> 24
   <211> 108
   <212> PRT
   <213> Artificial sequence
<220>
   <223> Description of Artificial sequence: 3-23: JH4 vector with CDR1/2 diversity
<220>
   <221> MOD_RES
   <222> (34)
   <223> Any amino acid
<220>
   <221> MOD_RES
   <222> (36)
   <223> Any amino acid
<220>
   <221> MOD_RES
   <222> (38)
   <223> Any amino acid
<220>
   <221> MOD_RES
   <222> (53)
   <223> Any amino acid
<220>
   <221> MOD_RES
   <222> (55)..(56)
   <223> Any amino acid
<220>
   <221> MOD_RES
   <222> (60)
   <223> Any amino acid
<220>
   <221> MOD_RES
   <222> (62)
   <223> Any amino acid
<400> 24
   Ala Met Ala Glu Val Gln Leu Leu Glu Ser Gly Gly Gly Leu Val Gln
   1               5                   10                  15
   Pro Gly Gly Ser Leu Arg Leu Ser Cys Ala Ala Ser Gly Phe Thr Phe
               20                  25                  30
   Ser Xaa Tyr Xaa Met Xaa Trp Val Arg Gln Ala Pro Gly Lys Gly Leu
           35                  40                  45
   Glu Trp Val Ser Xaa Ile Xaa Xaa Ser Gly Gly Xaa Thr Xaa Tyr Ala
       50                  55                  60
   Asp Ser Val Lys Gly Arg Phe Thr Ile Ser Arg Asp Asn Ser Lys Asn
   65                  70                  75                  80
   Thr Leu Tyr Leu Gln Met Asn Ser Leu Arg Ala Glu Asp Thr Ala Val
                   85                  90                  95
   Tyr Tyr Cys Ala Lys Asp Tyr Glu Gly Thr Gly Tyr
               100                 105
<210> 25
   <211> 45
   <212> DNA
   <213> Homo sapiens
<220>
   <221> CDS
   <222> (1)..(45)
<400> 25
   tat ttc gat tat tgg ggt caa ggt acc ctg gtc acc gtc tct agt        45
   Tyr Phe Asp Tyr Trp Gly Gln Gly Thr Leu Val Thr Val Ser Ser
   1               5                   10                  15
<210> 26
   <211> 15
   <212> PRT
   <213> Homo sapiens
<400> 26
   Tyr Phe Asp Tyr Trp Gly Gln Gly Thr Leu Val Thr Val Ser Ser
    1               5                  10                  15
<210> 27
   <211> 55
   <212> DNA
   <213> Artificial sequence
<220>
   <223> Description of Artificial Sequence: synthetic oligonucleotide
<220>
   <221> modified_base
   <222> (21)..(23)
   <223> a, c, t or g
<220>
   <221> modified_base
   <222> (27)..(29)
   <223> a, c, t or g
<220>
   <221> modified_base
   <222> (33)..(35)
   <223> a, c, t or g
<400> 27
   cttccggatt cactttctct nnntacnnna tgnnntgggt tcgccaagct cctgg 55
<210> 28
   <211> 28
   <212> DNA
   <213> Artificial sequence
<220>
   <223> Description of Artificial Sequence: Synthetic oligonucleotide
<400> 28
   cctactgtct tccggattca ctttctct 28
<210> 29
   <211> 30
   <212> DNA
   <213> Artificial Sequence
<220>
   <223> Description of Artificial sequence: Synthetic oligonucleotide
<400> 29
   tgggttcgcc aagctcctgg ttgctcactc 30
<210> 30
   <211> 61
   <212> DNA
   <213> Artificial Sequence
<220>
   <223> Description of Artificial Sequence: Synthetic oligonucleotide
<220>
   <221> modified_base
   <222> (23)..(29)
   <223> a, c, t or g
<220>
   <221> modified_base
   <222> (39)..(41)
   <223> a, c, t or g
<400> 30
   cttccggatt cactttctct wsnnnnnnnt actactggnn ntgggttcgc caagctcctg 60
   g 61
<210> 31
   <211> 82
   <212> DNA
   <213> Artificial Sequence
<220>
   <223> Description of Artificial sequence: synthetic oligonucleotide
<220>
   <221> modified_base
   <222> (42)..(50)
   <223> a, c, t or g
<220>
   <221> modified_base
   <222> (60)..(62)
   <223> a, c, t or g
<400> 31
   cttccggatt cactttctct atcagcggtg gttctatctc cnnnnnnnnn tactactggn 60
   nntgggttcg ccaagctcct gg 82
<210> 32
   <211> 68
   <212> DNA
   <213> Artificial sequence
<220>
   <223> Description of Artificial Sequence: synthetic oligonucleotide
<220>
   <221> modified_base
   <222> (19)..(21)
   <223> a, c, t or g
<220>
   <221> modified_base
   <222> (25)..(28)
   <223> a, c, t or g
<220>
   <221> modified_base
   <222> (30)
   <223> a, c, t or g
<220>
   <221> modified_base
   <222> (40)..(42)
   <223> a, c, t or g
<220>
   <221> modified_base
   <222> (46)..(48)
   <223> a, c, t or g
<400> 32
   ggtttggagt gggtttctnn natcnnnnsn tctggtggcn nnactnnnta tgctgactcc 60
   gttaaagg 68
<210> 33
   <211> 44
   <212> DNA
   <213> Artificial sequence
<220>
   <223> Description of Artificial sequence: Synthetic oligonucleotide
<400> 33
   cttgggttcg ccaagctcct ggtaaaggtt tggagtgggt ttct 44
<210> 34
   <211> 49
   <212> DNA
   <213> Artificial Sequence
<220>
   <223> Description of Artificial Sequence: Synthetic oligonucleotide
<400> 34
   tatgctgact ccgttaaagg tcgcttcact atctctagat tcctgtcac 49
<210> 35
   <211> 68
   <212> DNA
   <213> Artificial Sequence
<220>
   <223> Description of Artificial sequence: synthetic oligonucleotide
<220>
   <221> modified_base
   <222> (19)..(21)
   <223> a, c, t or g
<220>
   <221> modified_base
   <222> (26)..(33)
   <223> a, c, t or g
<220>
   <221> modified_base
   <222> (39)..(48)
   <223> a, c, t or g
<400> 35
   ggtttggagt gggtttctnn natcdnnnnn nnnggtdvnn nnnnnnnnta tgctgactcc 60
   gttaaagg 68
<210> 36
   <211> 65
   <212> DNA
   <213> Artificial Sequence
<220>
   <223> Description of Artificial Sequence: Synthetic oligonucleotide
<220>
   <221> modified_base
   <222> (19)..(21)
   <223> a, c, t or g
<220>
   <221> modified_base
   <222> (26)..(33)
   <223> a, c, t or g
<220>
   <221> modified_base
   <222> (39)..(45)
   <223> a, c, t or g
<400> 36
   ggtttggagt gggtttctnn natcdnnnnn nnnggtdvnn nnnnntataa cccttccctt 60
   aaggg 65
<210> 37
   <211> 49
   <212> DNA
   <213> Artificial Sequence
<220>
   <223> Description of Artificial sequence: Synthetic oligonucleotide
<400> 37
   tataaccctt cccttaaggg tcgcttcact atctctagat tcctgtcac 49
<210> 38
   <211> 74
   <212> DNA
   <213> Artificial sequence
<220>
   <223> Description of Artificial Sequence: synthetic oligonucleotide
<220>
   <221> modified_base
   <222> (19)..(21)
   <223> a, c, t or g
<220>
   <221> modified_base
   <222> (25)..(27)
   <223> a, c, t or g
<220>
   <221> modified_base
   <222> (31)..(39)
   <223> a, c, t or g
<220>
   <221> modified_base
   <222> (52)..(54)
   <223> a, c, t or g
<400> 38
   ggtttggagt gggtttctnn natcnnnagt nnnnnnnnng gtggtactac tnnntatgcc 60
   gcttccgtta aggg 74
<210> 39
   <211> 49
   <212> DNA
   <213> Artificial Sequence
<220>
   <223> Description of Artificial Sequence: synthetic oligonucleotide
<400> 39
   tatgccgctt ccgttaaggg tcgcttcact atctctagat tcctgtcac 49
<210> 40
   <211> 25
   <212> DNA
   <213> Artificial Sequence
<220>
   <223> Description of Artificial Sequence: Synthetic oligonucleotide
<400> 40
   gctctggtca acttaagggc tgagg 25
<210> 41
   <211> 48
   <212> DNA
   <213> Artificial Sequence
<220>
   <223> Description of Artificial Sequence: Synthetic oligonucleotide
<400> 41
   gctctggtca acttaagggc tgaggacacc gctgtctact actgcgcc 48
<210> 42
   <211> 46
   <212> DNA
   <213> Artificial sequence
<220>
   <223> Description of Artificial Sequence: Synthetic oligonucleotide
<400> 42
   tacttcgatt actggggcca aggtaccctg gtcacctcgc tccacc 46
<210> 43
   <211> 25
   <212> DNA
   <213> Artificial Sequence
<220>
   <223> Description of Artificial Sequence: Synthetic oligonucleotide
<400> 43
   ggtaccctgg tcacctcgct ccacc 25
<210> 44
   <211> 58
   <212> DNA
   <213> Artificial Sequence
<220>
   <223> Description of Artificial Sequence: synthetic oligonucleotide
<220>
   <221> modified_base
   <222> (23)..(35)
   <223> a, c, t or g
<400> 44
   ccgctgtcta ctactgcgcc mrnnnnnnnn nnnnntactt cgattactgg ggccaagg 58
<210> 45
   <211> 64
   <212> DNA
   <213> Artificial Sequence
<220>
   <223> Description of Artificial sequence: Synthetic oligonucleotide
<220>
   <221> modified_base
   <222> (23)..(41)
   <223> a, c, t or g
<400> 45
   ccgctgtcta ctactgcgcc mrnnnnnnnn nnnnnnnnnn ntacttcgat tactggggcc 60
   aagg 64
<210> 46
   <211> 70
   <212> DNA
   <213> Artificial Sequence
<220>
   <223> Description of Artificial Sequence: Synthetic oligonucleotide
<220>
   <221> modified_base
   <222> (23)..(47)
   <223> a, c, t or g
<400> 46
   ccgctgtcta ctactgcgcc mrnnnnnnnn nnnnnnnnnn nnnnnnntac ttcgattact 60
   ggggccaagg                                                         70
<210> 47
   <211> 76
   <212> DNA
   <213> Artificial sequence
<220>
   <223> Description of Artificial sequence: synthetic oligonucleotide
<220>
   <221> modified_base
   <222> (24)..(32)
   <223> a, c, t or g
<220>
   <221> modified_base
   <222> (38)
   <223> a, c, t or g
<220>
   <221> modified_base
   <222> (45)..(53)
   <223> a, c, t or g
<400> 47
   ccgctgtcta ctactgcgcc cgtnnnnnnn nntctdsntc ttrbnnnnnn nnntacttcg 60
   attactgggg ccaagg                                                  76
<210> 48
   <211> 79
   <212> DNA
   <213> Artificial Sequence
<220>
   <223> Description of Artificial Sequence: synthetic oligonucleotide
<220>
   <221> modified_base
   <222> (23)..(32)
   <223> a, c, t or g
<220>
   <221> modified_base
   <222> (42)..(47)
   <223> a, c, t or g
<220>
   <221> modified_base
   <222> (54)..(56)
   <223> a, c, t or g
<400> 48
   ccgctgtcta ctactgcgcc mrnnnnnnnn nntgctctgg tnnnnnntgc tatnnntact 60
   tcgattactg gggccaagg                                              79
<210> 49
   <211> 85
   <212> DNA
   <213> Artificial sequence
<220>
   <223> Description of Artificial Sequence: Synthetic oligonucleotide
<220>
   <221> modified_base
   <222> (23)..(29)
   <223> a, c, t or g
<220>
   <221> modified_base
   <222> (33)..(35)
   <223> a, c, t or g
<220>
   <221> modified_base
   <222> (48)..(62)
   <223> a, c, t or g
<400> 49
   ccgctgtcta ctactgcgcc mrnnnnnnnt ctnnnactat cttcggtnnn nnnnnnnnnn 60
   nntacttcga ttactggggc caagg                                       85
<210> 50
   <211> 88
   <212> DNA
   <213> Artificial Sequence
<220>
   <223> Description of Artificial Sequence: Synthetic oligonucleotide
<220>
   <221> modified_base
   <222> (24)..(32)
   <223> a, c, t or g
<220>
   <221> modified_base
   <222> (41)
   <223> a, c, t or g
<220>
   <221> modified_base
   <222> (47)
   <223> a, c, t or g
<220>
   <221> modified_base
   <222> (50)
   <223> a, c, t or g
<220>
   <221> modified_base
   <222> (57)..(65)
   <223> a, c, t or g
<400> 50
   ccgctgtcta ctactgcgcc cgtnnnnnnn nntattacgr ntctdsndsn tactatnnnn 60
   nnnnntactt cgattactgg ggccaagg                                    88
<210> 51
   <211> 91
   <212> DNA
   <213> Artificial Sequence
<220>
   <223> Description of Artificial Sequence: synthetic oligonucleotide
<220>
   <221> modified_base
   <222> (24)..(35)
   <223> a, c, t or g
<220>
   <221> modified_base
   <222> (44)
   <223> a, c, t or g
<220>
   <221> modified_base
   <222> (47)
   <223> a, c, t or g
<220>
   <221> modified_base
   <222> (50)..(53)
   <223> a, c, t or g
<220>
   <221> modified_base
   <222> (60)..(68)
   <223> a, c, t or g
<400> 51
   ccgctgtcta ctactgcgcc cgtnnnnnnn nnnnntattg cdsndsnrvn nnntgctatn 60
   nnnnnnnnta cttcgattac tggggccaag g                                91
<210> 52
   <211> 242
   <212> DNA
   <213> Artificial sequence
<220>
   <223> Description of Artificial Sequence: 3-23: JH4 vector with stuffers
<220>
   <221> CD5
   <222> (3)..(107)
<220>
   <221> CDS
   <222> (114)..(155)
<220>
   <221> CDS
   <222> (159)..(164)
<220>
   <221> CDS
   <222> (168)..(227)
<400> 52
    cc atg gcc gaa gtt caa ttg tta gag tct ggt ggc ggt ctt gtt cag      47
   Ala Met Ala Glu Val Gln Leu Leu Glu Ser Gly Gly Gly Leu Val Gln
     1               5                  10                  15
   cct ggt ggt tct tta cgt ctt tct tgc gct gct tcc gga ttc act ttc     95
   Pro Gly Gly Ser Leu Arg Leu Ser Cys Ala Ala Ser Gly Phe Thr Phe
                   20                  25                  30
   tct tcg tac gct tagtaa tgg gtt cgc caa gct cct ggt aaa ggt ttg     143
   Ser Ser Tyr Ala        Trp Val Arg Gln Ala Pro Gly Lys Gly Leu
               35                     40                  45
   gag tgg gtt tct taa cct agg tag act atc tct aga gac aac tct aag    191
   Glu Trp Val Ser     Pro Arg     Thr Ile Ser Arg Asp Asn Ser Lys
               50                          55                  60
   aat act ctc tac ttg cag atg aac agc tta agg gct tagtaaaggc cttaa   242
   Asn Thr Leu Tyr Leu Gln Met Asn Ser Leu Arg Ala
                   65                  70
<210> 53
   <211> 72
   <212> PRT
   <213> Artificial Sequence
<220>
   <223> Description of Artificial sequence: 3-23: JH4 vector with stuffers
<400> 53
   Ala Met Ala Glu Val Gln Leu Leu Glu Ser Gly Gly Gly Leu Val Gln
    1               5                  10                  15
   Pro Gly Gly Ser Leu Arg Leu Ser Cys Ala Ala Ser Gly Phe Thr Phe
               20                  25                  30
   Ser Ser Tyr Ala Trp Val Arg Gln Ala Pro Gly Lys Gly Leu Glu Trp
           35                  40                  45
   Val Ser Pro Arg Thr Ile Ser Arg Asp Asn Ser Lys Asn Thr Leu Tyr
       50                  55                  60
   Leu Gln Met Asn Ser Leu Arg Ala
   65                  70
<210> 54
   <211> 952
   <212> DNA
   <213> Homo sapiens
<220>
   <221> CDS
   <222> (206)..(907)
<220>
   <221> modified_base
   <222> (344)..(346)
   <223> a, c, t, g, other or unknown
<220>
   <221> modified_base
   <222> (350)..(361)
   <223> a, c, t, g, other or unknown
<220>
   <221> modified_base
   <222> (413)..(415)
   <223> a, c, t, g, other or unknown
<220>
   <221> modified_base
   <222> (422)..(424)
   <223> a, c, t, g, other or unknown
<220>
   <221> modified_base
   <222> (428)..(433)
   <223> a, c, t, g, other or unknown
<220>
   <221> modified_base
   <222> (536)..(547)
   <223> a, c, t, g, other or unknown
<220>
   <221> modified_base
   <222> (551)..(553)
   <223> a, c, t, g, other or unknown
<400> 54
   gaggaccatt gggccccctc cgagactctc gagcgcaacg caattaatgt gagttagctc 60
   actcattagg caccccaggc tttacacttt atgcttccgg ctcgtatgtt gtgtggaatt 120
   gtgagcggat aacaatttca cacaggaaac agctatgacc atgattacgc caagctttgg 180
   agcctttttt ttggagattt tcaac gtg aag aag ctc cta ttt gct atc ccg    232
                               Met Lys Lys Leu Leu Phe Ala Ile Pro
                               1               5
   ctt gtc gtt ccg ttt tac agc cat agt gca caa tcc gtc ctt act caa    280
   Leu Val Val Pro Phe Tyr Ser His Ser Ala Gln Ser Val Leu Thr Gln
   10                  15                  20                  25
   tct cct ggc act ctt tcg cta agc ccg ggt gaa cgt gct acc tta agt    328
   Ser Pro Gly Thr Leu Ser Leu Ser Pro Gly Glu Arg Ala Thr Leu Ser
                   30                  35                  40
   tgc cgt gct tcc cag nnn gtt nnn nnn nnn nnn ctt gct tgg tat caa    376
   Cys Arg Ala Ser Gln Xaa Val Xaa Xaa Xaa Xaa Leu Ala Trp Tyr Gln
               45                  50                  55
   cag aaa cct ggt cag gcg ccg cgt tta ctt att tat nnn gct tct nnn    424
   Gln Lys Pro Gly Gln Ala Pro Arg Leu Leu Ile Tyr Xaa Ala Ser Xaa
           60                  65                  70
   cgc nnn nnn ggg atc ccg gac cgt ttc tct ggc tct ggt tca ggt act    472
   Arg Xaa Xaa Gly Ile Pro Asp Arg Phe Ser Gly Ser Gly Ser Gly Thr
       75                  80                  85
   gac ttt acc ctt act att tct aga ttg gaa cct gaa gac ttc gct gtt    520
   Asp Phe Thr Leu Thr Ile Ser Arg Leu Glu Pro Glu Asp Phe Ala Val
   90                  95                  100                 105
   tat tat tgc caa cag nnn nnn nnn nnn cct nnn act ttc ggt caa ggt    568
   Tyr Tyr Cys Gln Gln Xaa Xaa Xaa Xaa Pro Xaa Thr Phe Gly Gln Gly
                   110                 115                 120
   acc aag gtt gaa atc aag cgt acg gtt gcc gct cct agt gtg ttt atc    616
   Thr Lys Val Glu Ile Lys Arg Thr Val Ala Ala Pro Ser Val Phe Ile
               125                 130                 135
   ttt cct cct tct gac gaa caa ttg aag tca ggt act gct tct gtc gta    664
   Phe Pro Pro Ser Asp Glu Gln Leu Lys Ser Gly Thr Ala Ser Val Val
           140                 145                 150
   tgt ttg ctc aac aat ttc tac cct cgt gaa gct aaa gtt cag tgg aaa    712
   Cys Leu Leu Asn Asn Phe Tyr Pro Arg Glu Ala Lys Val Gln Trp Lys
       155                 160                 165
   gtc gat aac gcg ttg cag tcg ggt aac agt caa gaa tcc gtc act gaa    760
   Val Asp Asn Ala Leu Gln Ser Gly Asn Ser Gln Glu Ser Val Thr Glu
   170                 175                 180                 185
   cag gat agt aag gac tct acc tac tct ttg tcc tct act ctt act tta    808
   Gln Asp Ser Lys Asp Ser Thr Tyr Ser Leu Ser Ser Thr Leu Thr Leu
                   190                 195                 200
   tca aag gct gat tat gag aag cat aag gtc tat gct tgc gaa gtt acc    856
   Ser Lys Ala Asp Tyr Glu Lys His Lys Val Tyr Ala Cys Glu Val Thr
               205                 210                 215
   cac cag ggt ctg agc tcc cct gtt acc aaa agt ttc aac cgt ggt gaa    904
   His Gln Gly Leu Ser Ser Pro Val Thr Lys Ser Phe Asn Arg Gly Glu
           220                 225                 230
   tgc taatagggcg cgccacgcat ctctaagcgg ccgcaacagg aggag              952
   Cys
<210> 55
   <211> 234
   <212> PRT
   <213> Homo sapiens
<220>
   <221> MOD_RES
   <222> (47)
   <223> Any amino acid
<220>
   <221> MOD_RES
   <222> (49)..(52)
   <223> Any amino acid
<220>
   <221> MOD_RES
   <222> (70)
   <223> Any amino acid
<220>
   <221> MOD_RES
   <222> (73)
   <223> Any amino acid
<220>
   <221> MOD_RES
   <222> (75)..(76)
   <223> Any amino acid
<220>
   <221> MOD_RES
   <222> (111)..(114)
   <223> Any amino acid
<220>
   <221> MOD_RES
   <222> (116)
   <223> Any amino acid
<400> 55
   Met Lys Lys Leu Leu Phe Ala Ile Pro Leu Val Val Pro Phe Tyr Ser
   1               5                   10                  15
   His Ser Ala Gln Ser Val Leu Thr Gln Ser Pro Gly Thr Leu Ser Leu
               20                  25                  30
   Ser Pro Gly Glu Arg Ala Thr Leu Ser Cys Arg Ala Ser Gln Xaa Val
           35                  40                  45
   Xaa Xaa Xaa Xaa Leu Ala Trp Tyr Gln Gln Lys Pro Gly Gln Ala Pro
       50                  55                  60
   Arg Leu Leu Ile Tyr Xaa Ala Ser Xaa Arg Xaa Xaa Gly Ile Pro Asp
   65                  70                  75                  80
   Arg Phe Ser Gly Ser Gly Ser Gly Thr Asp Phe Thr Leu Thr Ile Ser
                   85                  90                  95
   Arg Leu Glu Pro Glu Asp Phe Ala Val Tyr Tyr Cys Gln Gln Xaa Xaa
               100                 105                 110
   Xaa Xaa Pro Xaa Thr Phe Gly Gln Gly Thr Lys Val Glu Ile Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230
<210> 56
   <211> 732
   <212> DNA
   <213> Homo sapiens
<220>
   <221> CDS
   <222> (30)..(686)
<220>
   <221> modified_base
   <222> (108)..(110)
   <223> a, c, t, g, other or unknown
<220>
   <221> modified_base
   <222> (117)..(119)
   <223> a, c, t, g, other or unknown
<220>
   <221> modified_base
   <222> (126)..(137)
   <223> a, c, t, g, other or unknown
<220>
   <221> modified_base
   <222> (189)..(200)
   <223> a, c, t, g, other or unknown
<220>
   <221> modified_base
   <222> (306)..(320)
   <223> a, c, t, g, other or unknown
<220>
   <221> modified_base
   <222> (324)..(335)
   <223> a, c, t, g, other or unknown
<400> 56
   gaggaccatt gggcccctta ctccgtgac agt gca caa tcc gct ctc act cag     53
                                   Ser Ala Gln Ser Ala Leu Thr Gln
                                   1               5
   cct gct agc gtt tcc ggg tca cct ggt caa agt atc act att tct tgt    101
   Pro Ala Ser Val Ser Gly Ser Pro Gly Gln Ser Ile Thr Ile Ser Cys
       10                  15                  20
   aca ggt nnn tct tct nnn gtt ggc nnn nnn nnn nnn gtt tct tgg tat    149
   Thr Gly Xaa Ser Ser Xaa Val Gly Xaa Xaa Xaa Xaa Val Ser Trp Tyr
   25                  30                  35                  40
   caa caa cac ccg ggc aag gcg ccg aag ttg atg atc tac nnn nnn nnn    197
   Gln Gln His Pro Gly Lys Ala Pro Lys Leu Met Ile Tyr Xaa Xaa Xaa
                   45                  50                  55
   nnn cgt cct tct ggt gtt agc aat cgt ttc tcc gga tct aaa tcc ggt    245
   Xaa Arg Pro Ser Gly Val Ser Asn Arg Phe Ser Gly Ser Lys Ser Gly
               60                  65                  70
   aat acc gca agc tta act atc tct ggt ctg cag gct gaa gac gag gct    293
   Asn Thr Ala Ser Leu Thr Ile Ser Gly Leu Gln Ala Glu Asp Glu Ala
           75                  80                  85
   gac tac tat tgt nnn nnn nnn nnn nnn tct nnn nnn nnn nnn gtc ttc    341
   Asp Tyr Tyr Cys Xaa Xaa Xaa Xaa Xaa Ser Xaa Xaa Xaa Xaa Val Phe
       90                  95                  100
   ggc ggt ggt acc aaa ctt act gtc ctc ggt caa cct aag gct gct cct    389
   Gly Gly Gly Thr Lys Leu Thr Val Leu Gly Gln Pro Lys Ala Ala Pro
   105                 110                 115                 120
   tcc gtt act ctc ttc cct cct agt tct gaa gag ctt caa gct aac aag    437
   Ser Val Thr Leu Phe Pro Pro Ser Ser Glu Glu Leu Gln Ala Asn Lys
                   125                 130                 135
   gct act ctt gtt tgc ttg atc agt gac ttt tat cct ggt gct gtt act    485
   Ala Thr Leu Val Cys Leu Ile Ser Asp Phe Tyr Pro Gly Ala Val Thr
               140                 145                 150
   gtc gct tgg aaa gcc gat tct tct cct gtt aaa gct ggt gtt gag acg     533
   Val Ala Trp Lys Ala Asp Ser Ser Pro Val Lys Ala Gly Val Glu Thr
           155                 160                 165
   acc act cct tct aaa caa tct aac aat aag tac gct gcg agc tct tat     581
   Thr Thr Pro Ser Lys Gln Ser Asn Asn Lys Tyr Ala Ala Ser Ser Tyr
       170                 175                 180
   ctt tct ctc acc cct gaa caa tgg aag tct cat aaa tcc tat tcc tgt     629
   Leu Ser Leu Thr Pro Glu Gln Trp Lys Ser His Lys Ser Tyr Ser Cys
   185                 190                 195                 200
   caa gtt act cat gaa ggt tct acc gtt gaa aag act gtt gcc cct act     677
   Gln Val Thr His Glu Gly Ser Thr Val Glu Lys Thr Val Ala Pro Thr
                   205                 210                 215
   gag tgt tct tagtgaggcg cgccaacgat gttcaaggcg gccgcaacag gaggag      732
   Glu Cys Ser
<210> 57
   <211> 219
   <212> PRT
   <213> Homo sapiens
<220>
   <221> MOD_RES
   <222> (27)
   <223> Any amino acid
<220>
   <221> MOD_RES
   <222> (30)
   <223> Any amino acid
<220>
   <221> MOD_RES
   <222> (33)..(36)
   <223> Any amino acid
<220>
   <221> MOD_RES
   <222> (54)..(57)
   <223> Any amino acid
<220>
   <221> MOD_RES
   <222> (93)..(97)
   <223> Any amino acid
<220>
   <221> MOD_RES
   <222> (99)..(102)
   <223> Any amino acid
<400> 57
   Ser Ala Gln Ser Ala Leu Thr Gln Pro Ala Ser Val Ser Gly Ser Pro
   1               5                   10                  15
   Gly Gln Ser Ile Thr Ile Ser Cys Thr Gly Xaa Ser Ser Xaa Val Gly
               20                  25                  30
   Xaa Xaa Xaa Xaa Val Ser Trp Tyr Gln Gln His Pro Gly Lys Ala Pro
           35                  40                  45
   Lys Leu Met Ile Tyr Xaa Xaa Xaa Xaa Arg Pro Ser Gly Val Ser Asn
       50                  55                  60
   Arg Phe Ser Gly Ser Lys Ser Gly Asn Thr Ala Ser Leu Thr Ile Ser
   65                  70                  75                  80
   Gly Leu Gln Ala Glu Asp Glu Ala Asp Tyr Tyr Cys Xaa Xaa Xaa Xaa
                   85                  90                  95
   Xaa Ser Xaa Xaa Xaa Xaa Val Phe Gly Gly Gly Thr Lys Leu Thr Val
               100                 105                 110
   Leu Gly Gln Pro Lys Ala Ala Pro Ser Val Thr Leu Phe Pro Pro Ser
           115                 120                 125
   Ser Glu Glu Leu Gln Ala Asn Lys Ala Thr Leu Val Cys Leu Ile Ser
       130                 135                 140
   Asp Phe Tyr Pro Gly Ala Val Thr Val Ala Trp Lys Ala Asp Ser Ser
   145                 150                 155                 160
   Pro Val Lys Ala Gly Val Glu Thr Thr Thr Pro Ser Lys Gln Ser Asn
                   165                 170                 175
   Asn Lys Tyr Ala Ala Ser Ser Tyr Leu Ser Leu Thr Pro Glu Gln Trp
               180                 185                 190
   Lys Ser His Lys Ser Tyr Ser Cys Gln Val Thr His Glu Gly Ser Thr
           195                 200                 205
   Val Glu Lys Thr Val Ala Pro Thr Glu Cys Ser
       210                 215
<210> 58
   <211> 25
   <212> DNA
   <213> Artificial sequence
<220>
   <223> Description of Artificial Sequence: synthetic oligonucleotide
<400> 58
   gctctggtca acttaagggc tgagg 25
<210> 59
   <211> 48
   <212> DNA
   <213> Artificial Sequence
<220>
   <223> Description of Artificial sequence: synthetic oligonucleotide
<400> 59
   gctctggtca acttaagggc tgaggacacc gctgtctact actgcgcc 48
<210> 60
   <211> 46
   <212> DNA
   <213> Artificial sequence
<220>
   <223> Description of Artificial Sequence: synthetic oligonucleotide
<400> 60
   tacttcgatt acttgggcca aggtaccctg gtcacctcgc tccacc 46
<210> 61
   <211> 25
   <212> DNA
   <213> Artificial sequence
<220>
   <223> Description of Artificial Sequence: Synthetic oligonucleotide
<400> 61
   ggtaccctgg tcacctcgct ccacc 25
<210> 62
   <211> 56
   <212> DNA
   <213> Artificial sequence
<220>
   <223> Description of Artificial sequence: Synthetic oligonucleotide
<400> 62
   ggtctcagtt gctaagcccg ggtgaacgtg ctaccttaag ttgccgtgct tcccag 56
<210> 63
   <211> 24
   <212> DNA
   <213> Artificial Sequence
<220>
   <223> Description of Artificial Sequence: Synthetic oligonucleotide
<400> 63
   ggtctcagtt gctaagcccg ggtg 24
<210> 64
   <211> 45
   <212> DNA
   <213> Artificial Sequence
<220>
   <223> Description of Artificial sequence: Synthetic oligonucleotide
<400> 64
   cttgcttggt atcaacagaa acctggtcag gcgccaagtc gtgtc 45
<210> 65
   <211> 24
   <212> DNA
   <213> Artificial sequence
<220>
   <223> Description of Artificial sequence: synthetic oligonucleotide
<400> 65
   cctggtcagg cgccaagtcg tgtc 24
<210> 66
   <211> 65
   <212> DNA
   <213> Artificial Sequence
<220>
   <223> Description of Artificial Sequence: Synthetic oligonucleotide
<220>
   <221> modified_base
   <222> (28)..(30)
   <223> a, c, t or g
<220>
   <221> modified_base
   <222> (34)..(42)
   <223> a, c, t or g
<400> 66
   gctaccttaa gttgccgtgc ttcccagnnn gttnnnnnnn nncttgcttg gtatcaacag 60
   aaacc 65
<210> 67
   <211> 68
   <212> DNA
   <213> Artificial Sequence
<220>
   <223> Description of Artificial Sequence: Synthetic oligonucleotide
<220>
   <221> modified_base
   <222> (28)..(30)
   <223> a, c, t or g
<220>
   <221> modified_base
   <222> (34)..(45)
   <223> a, c, t or g
<400> 67
   gctaccttaa gttgccgtgc ttcccagnnn gttnnnnnnn nnnnncttgc ttggtatcaa 60
   cagaaacc 68
<210> 68
   <211> 22
   <212> DNA
   <213> Artificial sequence
<220>
   <223> Description of Artificiai Sequence: synthetic oligonucleotide
<400> 68
   cacgagtcct acctggtcag gc 22
<210> 69
   <211> 41
   <212> DNA
   <213> Artificial sequence
<220>
   <223> Description of Artificial Sequence: Synthetic oligonucleotide
<400> 69
   cacgagtcct acctggtcag gcgccgcgtt tacttattta t 41
<210> 70
   <211> 22
   <212> DNA
   <213> Artificial Sequence
<220>
   <223> Description of Artificial sequence: Synthetic oligonucleotide
<400> 70
   gaccgtttct ctggttctca cc 22
<210> 71
   <211> 76
   <212> DNA
   <213> Artificial Sequence
<220>
   <223> Description of Artificial sequence: synthetic oligonucleotide
<220>
   <221> modified_base
   <222> (25)..(27)
   <223> a, c, t or g
<220>
   <221> modified_base
   <222> (34)..(36)
   <223> a, c, t or g
<220>
   <221> modified_base
   <222> (40)..(45)
   <223> a, c, t or g
<400> 71
   caggcgccgc gtttacttat ttatnnngct tctnnncgcn nnnnngggat cccggaccgt 60
   ttctctggtt ctcacc 76
<210> 72
   <211> 53
   <212> DNA
   <213> Artificial Sequence
<220>
   <223> Description of Artificial Sequence: synthetic oligonucleotide
<400> 72
   gacgagtcct tctagattgg aacctgaaga cttcgctgtt tattattgcc aac 53
<210> 73
   <211> 50
   <212> DNA
   <213> Artificial sequence
<220>
   <223> Description of Artificial sequence: Synthetic oligonucleotide
<400> 73
   actttcggtc aaggtaccaa ggttgaaatc aagcgtacgt cacaggtgag 50
<210> 74
   <211> 26
   <212> DNA
   <213> Artificial Sequence
<220>
   <223> Description of Artificial sequence: synthetic oligonucleotide
<400> 74
   gaaatcaagc gtacgtcaca ggtgag 26
<210> 75
   <211> 70
   <212> DNA
   <213> Artificial sequence
<220>
   <223> Description of Artificial sequence: Synthetic oligonucleotide
<220>
   <221> modified_base
   <222> (28)..(39)
   <223> a, c, t or g
<220>
   <221> modified_base
   <222> (43)..(45)
   <223> a, c, t or g
<400> 75
   gacttcgctg tttattattg ccaacagnnn nnnnnnnnnc ctnnnacttt cggtcaaggt 60
   accaaggttg                                                        70
<210> 76
   <211> 67
   <212> DNA
   <213> Artificial sequence
<220>
   <223> Description of Artificial sequence: synthetic oligonucleotide
<220>
   <221> modified_base
   <222> (28)..(42)
   <223> a, c, t or g
<400> 76
   gacttcgctg tttattattg ccaacagnnn nnnnnnnnnn nncctttcgg tcaaggtacc 60
   aaggttg 67
<210> 77
   <211> 73
   <212> DNA
   <213> Artificial Sequence
<220>
   <223> Description of Artificial sequence: Synthetic oligonucleotide
<220>
   <221> modified_base
   <222> (28)..(39)
   <223> a, c, t or g
<220>
   <221> modified_base
   <222> (46)..(48)
   <223> a, c, t or g
<400> 77
   gacttcgctg tttattattg ccaacagnnn nnnnnnnnnc ctcctnnnac tttcggtcaa 60
   ggtaccaagg ttg 73
<210> 78
   <211> 21
   <212> DNA
   <213> Artificial Sequence
<220>
   <223> Description of Artificial sequence: synthetic oligonucleotide
<400> 78
   gacgagtcct ggtcacctgg t 21
<210> 79
   <211> 48
   <212> DNA
   <213> Artificial Sequence
<220>
   <223> Description of Artificial sequence: Synthetic oligonucleotide
<400> 79
   gacgagtcct ggtcacctgg tcaaagtatc actatttctt gtacaggt 48
<210> 80
   <211> 50
   <212> DNA
   <213> Artificial Sequence
<220>
   <223> Description of Artificial Sequence: Synthetic oligonucleotide
<400> 80
   gtttcttggt atcaacaaca cccgggcaag gcgagatctt cacaggtgag 50
<210> 81
   <211> 25
   <212> DNA
   <213> Artificial sequence
<220>
   <223> Description of Artificial Sequence: Synthetic oligonucleotide
<400> 81
   gcaaggcgag atcttcacag gtgag 25
<210> 82
   <211> 67
   <212> DNA
   <213> Artificial Sequence
<220>
   <223> Description of Artificial sequence: synthetic oligonucleotide
<220>
   <221> modified_base
   <222> (24)..(29)
   <223> a, c, t or g
<220>
   <221> modified_base
   <222> (33)..(50)
   <223> a, c, t or g
<400> 82
   gtatcactat ttcttgtaca ggtnnnnnnc tcnnnnnnnn nnnnnnnnnn tggtatcaac 60
   aacaccc 67
<210> 83
   <211> 76
   <212> DNA
   <213> Artificial sequence
<220>
   <223> Description of Artificial Sequence: synthetic oligonucleotide
<220>
   <221> modified_base
   <222> (24)..(26)
   <223> a, c, t or g
<220>
   <221> modified_base
   <222> (33)..(35)
   <223> a, c, t or g
<220>
   <221> modified_base
   <222> (42)..(53)
   <223> a, c, t or g
<400> 83
   gtatcactat ttcttgtaca ggtnnntctt ctnnngttgg cnnnnnnnnn nnngtttctt 60
   ggtatcaaca acaccc 76
<210> 84
   <211> 22
   <212> DNA
   <213> Artificial Sequence
<220>
   <223> Description of Artificial sequence: synthetic oligonucleotide
<400> 84
   gagcagagga cccgggcaag gc 22
<210> 85
   <211> 41
   <212> DNA
   <213> Artificial Sequence
<220>
   <223> Description of Artificial sequence: synthetic oligonucleotide
<400> 85
   gagcagagga cccgggcaag gcgccgaagt tgatgatcta c 41
<210> 86
   <211> 44
   <212> DNA
   <213> Artificial Sequence
<220>
   <223> Description of Artificial sequence: synthetic oligonucleotide
<400> 86
   cgtccttctg gtgtcagcaa tcgtttctcc ggatcacagg tgag 44
<210> 87
   <211> 23
   <212> DNA
   <213> Artificial Sequence
<220>
   <223> Description of Artificial Sequence: synthetic oligonucleotide
<400> 87
   cgtttctccg gatcacaggt gag 23
<210> 88
   <211> 53
   <212> DNA
   <213> Artificial sequence
<220>
   <223> Description of Artificial Sequence: synthetic oligonucleotide
<220>
   <221> modified_base
   <222> (20)..(31)
   <223> a, c, t or g
<400> 88
   gccgaagttg atgatctacn nnnnnnnnnn ncgtccttct ggtgtcagca atc 53
<210> 89
   <211> 24
   <212> DNA
   <213> Artificial sequence
<220>
   <223> Description of Artificial Sequence: synthetic oligonucleotide
<400> 89
   ctgcaggctg aagacgaggc tgac 24
<210> 90
   <211> 33
   <212> DNA
   <213> Artificial Sequence
<220>
   <223> Description of Artificial sequence: Synthetic oligonucleotide
<400> 90
   ctgcaggctg aagacgaggc tgactactat tgt 33
<210> 91
   <211> 57
   <212> DNA
   <213> Artificial Sequence
<220>
   <223> Description of Artificial sequence: Synthetic oligonucleotide
<400> 91
   gtcttcggcg gtggtaccaa acttactgtc ctcggtcaac ctaaggacac aggtgag 57
<210> 92
   <211> 25
   <212> DNA
   <213> Artificial Sequence
<220>
   <223> Description of Artificial Sequence: Synthetic oligonucleotide
<400> 92
   cggtcaacct aaggacacag gtgag 25
<210> 93
   <211> 77
   <212> DNA
   <213> Artificial sequence
<220>
   <223> Description of Artificial sequence: Synthetic oligonucleotide
<220>
   <221> modified_base
   <222> (22)..(36)
   <223> a, c, t or g
<220>
   <221> modified_base
   <222> (40)..(51)
   <223> a, c, t or g
<400> 93
   gacgaggctg actactattg tnnnnnnnnn nnnnnntctn nnnnnnnnnn ngtcttcggc 60
   ggtggtacca aacttac 77
<210> 94
   <211> 74
   <212> DNA
   <213> Artificial Sequence
<220>
   <223> Description of Artificial sequence: synthetic oligonucleotide
<220>
   <221> modified_base
   <222> (22)..(24)
   <223> a, c, t or g
<220>
   <221> modified_base
   <222> (31)..(36)
   <223> a, c, t or g
<220>
   <221> modified_base
   <222> (40)..(48)
   <223> a, c, t or g
<400> 94
   gacgaggctg actactattg tnnnagctat nnnnnntctn nnnnnnnngt cttcggcggt 60
   ggtaccaaac ttac                                                   74
<210> 95
   <211> 627
   <212> DNA
   <213> Artificial sequence
<220>
   <223> Description of Artificial Sequence: A27: JH1 Kappa light chain gene with stuffers
<220>
   <221> CDS
   <222> (206)..(328)
<220>
   <221> CDS
   <222> (357)..(377)
<220>
   <221> CDS
   <222> (405)..(470)
<220>
   <221> CDS
   <222> (501)..(596)
<400> 95
   gaggaccatt gggccccctc cgagactctc gagcgcaacg caattaatgt gagttagctc 60
   actcattagg caccccaggc tttacacttt atgcttccgg ctcgtatgtt gtgtggaatt 120
   gtgagcggat aacaatttca cacaggaaac agctatgacc atgattacgc caagctttgg 180
   agcctttttt ttggagattt tcaac gtg aag aag ctc cta ttt gct atc ccg   232
                               Met Lys Lys Leu Leu Phe Ala Ile Pro
                                1               5
   ctt gtc gtt ccg ttt tac agc cat agt gca caa tcc gtc ctt act caa   280
   Leu Val Val Pro Phe Tyr Ser His Ser Ala Gln Ser Val Leu Thr Gln
    10                  15                  20                  25
   tct cct ggc act ctt tcg cta agc ccg ggt gaa cgt gct acc tta agt   328
   Ser Pro Gly Thr Leu Ser Leu Ser Pro Gly Glu Arg Ala Thr Leu Ser
                       30                  35                  40
   tagtaagctc ccaggcctct ttgatctg aaa cct ggt cag gcg ccg cgt         377
                                  Lys Pro Gly Gln Ala Pro Arg
                                              45
   taatgaaagc gctaatggcc aacagtg act ggg atc ccg gac cgt ttc tct ggc 431
                                 Thr Gly Ile Pro Asp Arg Phe Ser Gly
                                     50                  55
   tct ggt tca ggt act gac ttt acc ctt act att tct aga taatgagtta    480
   Ser Gly Ser Gly Thr Asp Phe Thr Leu Thr Ile Ser Arg
           60                  65                  70
   actagaccta cgtaacctag ttc ggt caa ggt acc aag gtt gaa atc aag cgt 533
                         Phe Gly Gln Gly Thr Lys Val Glu Ile Lys Arg
                                         75                  80
   acg gtt gcc gct cct agt gtg ttt atc ttt cct cct tct gac gaa caa    581
   Thr Val Ala Ala Pro Ser Val Phe Ile Phe Pro Pro Ser Asp Glu Gln
               85                  90                  95
   ttg aag tca ggt act acgcatctct aagcggccgc aacaggagga g            627
   Leu Lys Ser Gly Thr
           100
<210> 96
   <211> 102
   <212> PRT
   <213> Artificial Sequence
<220>
   <223> Description of Artificial Sequence: A27: JH1 Kappa light chain gene with stuffers
<400> 96
   Met Lys Lys Leu Leu Phe Ala Ile Pro Leu Val Val Pro Phe Tyr Ser
   1               5                   10                  15
   His Ser Ala Gln Ser Val Leu Thr Gln Ser Pro Gly Thr Leu Ser Leu
               20                  25                  30
   Ser Pro Gly Glu Arg Ala Thr Leu Ser Lys Pro Gly Gln Ala Pro Arg
           35                  40                  45
   Thr Gly Ile Pro Asp Arg Phe Ser Gly Ser Gly Ser Gly Thr Asp Phe
       50                  55                  60
   Thr Leu Thr Ile Ser Arg Phe Gly Gln Gly Thr Lys Val Glu Ile Lys
   65                  70                  75                  80
   Arg Thr Val Ala Ala Pro Ser Val Phe Ile Phe Pro Pro Ser Asp Glu
                   85                  90                  95
   Gln Leu Lys Ser Gly Thr
               100
<210> 97
   <211> 413
   <212> DNA
   <213> Artificial Sequence
<220>
   <223> Description of Artificial sequence: 2a2: JH2 Human lambda-chain gene with stuffers in place of CDRS
<220>
   <221> CDS
   <222> (30)..(104)
<220>
   <221> CDS
   <222> (117)..(122)
<220>
   <221> CDS
   <222> (177)..(239)
<220>
   <221> CDS
   <222> (270)..(413)
<220>
   <221> CDS
   <222> (129)..(131)
<220>
   <221> CDS
   <222> (135)..(152)
<400> 97
   gaggaccatt gggcccctta ctccgtgac agt gca caa tcc gct ctc act cag    53
                                   Ser Ala Gln Ser Ala Leu Thr Gln
                                   1               5
   cct gct agc gtt tcc ggg tca cct ggt caa agt atc act att tct tgt    101
   Pro Ala Ser Val Ser Gly Ser Pro Gly Gln Ser Ile Thr Ile Ser Cys
       10                  15                  20
   aca tcttagtgac tc aga tct taatga ccg   tag cac ccg ggc aag gcg     149
   Thr               Arg Ser        Pro       His Pro Gly Lys Ala
   25                                             30
   ccg taatgaatct cgtacgctgg tgtt agc aat cgt ttc tcc gga tct aaa     200
   Pro                            Ser Asn Arg Phe Ser Gly Ser Lys
                                  35                  40
   tcc ggt aat acc gca agc tta act atc tct ggt ctg cag gttctgtagt     249
   Ser Gly Asn Thr Ala Ser Leu Thr Ile Ser Gly Leu Gln
           45                  50                  55
   tccaattgct ttagtgaccc ggc ggt ggt acc aaa ctt act gtc ctc ggt caa 302
                         Gly Gly Gly Thr Lys Leu Thr Val Leu Gly Gln
                                         60                  65
   cct aag gct gct cct tcc gtt act ctc ttc cct cct agt tct gaa gag    350
   Pro Lys Ala Ala Pro Ser Val Thr Leu Phe Pro Pro Ser Ser Glu Glu
               70                  75                  80
   ctt caa gct aac aag gct act ctt gtt tgc ttg atc agt gac ttt tat    398
   Leu Gln Ala Asn Lys Ala Thr Leu Val Cys Leu Ile Ser Asp Phe Tyr
           85                  90                  95
   cct ggt gct gtt act                                                413
   Pro Gly Ala Val Thr
       100
<210> 98
   <211> 103
   <212> PRT
   <213> Artificial Sequence
<220>
   <223> Description of Artificial sequence: 2a2: JH2 Human lambda-chain gene with stuffers in place of CDRS
<400> 98
   Ser Ala Gln Ser Ala Leu Thr Gln Pro Ala Ser Val Ser Gly Ser Pro
    1               5                  10                  15
   Gly Gln Ser Ile Thr Ile Ser Cys Thr Arg Ser Pro His Pro Gly Lys
               20                  25                  30
   Ala Pro Ser Asn Arg Phe Ser Gly Ser Lys Ser Gly Asn Thr Ala Ser
           35                  40                  45
   Leu Thr Ile Ser Gly Leu Gln Gly Gly Gly Thr Lys Leu Thr Val Leu
       50                  55                  60
   Gly Gln Pro Lys Ala Ala Pro Ser Val Thr Leu Phe Pro Pro Ser Ser
   65                  70                  75                  80
   Glu Glu Leu Gln Ala Asn Lys Ala Thr Leu Val Cys Leu Ile Ser Asp
                   85                  90                  95
   Phe Tyr Pro Gly Ala Val Thr
               100
<210> 99
   <211> 10
   <212> DNA
   <213> Artificial Sequence
<220>
   <223> Description of Artificial sequence: Synthetic oligonucleotide
<400> 99
   ctgtctgaac 10

## Description

The present invention relates to focused libraries of genetic packages that each display, display and express, or comprise a member of a diverse family of peptides, polypeptides or proteins and collectively display, display and express, or comprise at least a portion of the focused diversity of the family, as defined in the claims. The focused diversity of the libraries of this invention comprises both sequence diversity and length diversity. In a preferred embodiment, the focused diversity of the libraries of this invention is biased toward the natural diversity of the selected family. In a more preferred embodiment, the libraries are biased toward the natural diversity of human antibodies and are characterized by variegation in their heavy chain and light chain complementarity determining regions ("CDRs").

The present description further discloses vectors and genetic packages (e.g., cells, spores or viruses) for displaying, or displaying and expressing a focused diverse family of peptides, polypeptides or proteins. In a preferred embodiment the genetic packages are filamentous phage or phagemids or yeast. Again, the focused diversity of the family comprises diversity in sequence and diversity in length.

The present description further discloses methods of screening the focused libraries of the invention and to the peptides, polypeptides and proteins identified by such screening.

### BACKGROUND OF THE INVENTION

It is now common practice in the art to prepare libraries of genetic packages that individually display, display and express, or comprise a member of a diverse family of peptides, polypeptides or proteins and collectively display, display and express, or comprise at least a portion of the amino acid diversity of the family. In many common libraries, the peptides, polypeptides or proteins are related to antibodies (e.g., single chain Fv (scFv), Fv, Fab, whole antibodies or minibodies (i.e., dimers that consist of V_{H} linked to V_{L})). Often, they comprise one or more of the CDRs and framework regions of the heavy and light chains of human antibodies.

Peptide, polypeptide or protein libraries have been produced in several ways in the prior art. See e.g., Knappik et al., J. Mol. Biol., 296, pp. 57-86 (2000). One method is to capture the diversity of native donors, either naive or immunized. Another way is to generate libraries having synthetic diversity. A third method is a combination of the first two. Typically, the diversity produced by these methods is limited to sequence diversity, i.e., each member of the library differs from the other members of the family by having different amino acids or variegation at a given position in the peptide, polypeptide or protein chain. Naturally diverse peptides, polypeptides or proteins, however, are not limited to diversity only in their amino acid sequences. For example, human antibodies are not limited to sequence diversity in their amino acids, they are also diverse in the lengths of their amino acid chains.

For antibodies, diversity in length occurs, for example, during variable region rearrangements. See e.g., Corbett et al., J. Mol. Biol., 270, pp. 587-97 (1997). The joining of V genes to J genes, for example, results in the inclusion of a recognizable D segment in CDR3 in about half of the heavy chain antibody sequences, thus creating regions encoding varying lengths of amino acids. The following also may occur during joining of antibody gene segments: (i) the end of the V gene may have zero to several bases deleted or changed; (ii) the end of the D segment may have zero to many bases removed or changed; (iii) a number of random bases may be inserted between V and D or between D and J; and (iv) the 5' end of J may be edited to remove or to change several bases. These rearrangements result in antibodies that are diverse both in amino acid sequence and in length.

Libraries that contain only amino acid sequence diversity are, thus, disadvantaged in that they do not reflect the natural diversity of the peptide, polypeptide or protein that the library is intended to mimic. Further, diversity in length may be important to the ultimate functioning of the protein, peptide or polypeptide. For example, with regard to a library comprising antibody regions, many of the peptides, polypeptides, proteins displayed, displayed and expressed, or comprised by the genetic packages of the library may not fold properly or their binding to an antigen may be disadvantaged, if diversity both in sequence and length are not represented in the library.

An additional disadvantage of prior art libraries of genetic packages that display, display and express, or comprise peptides, polypeptides and proteins is that they are not focused on those members that are based on natural occurring diversity and thus on members that are most likely to be functional. Rather, the prior art libraries, typically, attempt to include as much diversity or variegation at every amino acid residue as possible. This makes library construction time-consuming and less efficient than possible. The large number of members that are produced by trying to capture complete diversity also makes screening more cumbersome than it needs to be. This is particularly true given that many members of the library will not be functional.

### SUMMARY OF THE INVENTION

An object of this invention is focused DNA libraries comprising variegated DNA sequences of the CDRs of human antibodies that are diverse in both their amino acid sequence and in their length (examples of such libraries include libraries of single chain Fv (scFv), Fv, Fab, whole antibodies or minibodies (i.e., dimers that consist of V_{H} linked to V_{L})). Such regions may be from the heavy or light chains or both and may include one or more of the CDRs of those chains. More preferably, the diversity or variegation occurs in all of the heavy chain and light chain CDRs.

Among the preferred embodiments of this invention are the following:
1. A focused DNA library as defined in paragraph 4 below being characterized by further comprising variegated DNA sequences that encode a heavy chain CDR1 selected from the group consisting of:
   (1) <1>₁Y₂<1>₃M₄<1>₅, wherein <1> is an equimolar mixture of each of amino acid residues A, D, E, F, G, H, I, K, L, M, N, P, Q, R, S, T, V, W, and Y;
   (2) (S/T)₁(S/G/X)₂(S/G/X)₃Y₄Y₅W₆(S/G/X)₇. wherein (S/T) is a 1:1 mixture of S and T residues, (S/G/X) is a mixture of 0.2025 S, 0.2025 G and 0.035 of each of amino acid residues A, D, E, F, H, I, K, L, M, N, P, Q, R, T, V, W, and Y;
   (3) V₁S₂G₃G₄S₅I₆S₇<1>₈<1>₉<1>₁₀Y₁₁Y₁₂W₁₃<1>₁₄, wherein <1> is an equimolar mixture of each of amino acid residues A, D, E, F, G, H, I, K, L, M, N, P, Q, R, S, T, V, W, and Y; and
   (4) mixtures of vectors or genetic packages characterized by any of the above DNA sequences, preferably in the ratio: HC CDR1s (1):(2):(3)::0.80:0.17:0.02.
2. A focused DNA library as defined in paragraph 4 below being characterized by further comprising variegated DNA sequences that encode a heavy chain CDR2 selected from the group consisting of:
   (1) <2>I<2><3>SGG<1>T<1>YADSVKG, wherein <1> is an equimolar mixture of each of amino acid residues A, D, E, F, G, H, I, K, L, M, N, P, Q, R, S, T, V, W, and Y; <2> is an equimolar mixture of each of amino acid residues Y, R, W, V, G, and S; and <3> is an equimolar mixture of each of amino acid residues P, S, and G or an equimolar mixture of P and S;
   (2) <1>I<4><1><1><G><5><1><1><1>YADSVKG, wherein <1> is an equimolar mixture of each of amino acid residues A, D, E, F, G, H, I, K, L, M, N, P, Q, R, S, T, V, W, and Y; <4> is an equimolar mixture of residues D, I, N, S, W, Y; and <5> is an equimolar mixture of residues S, G, D and N;
   (3) <1>I<4><1><1>G<5><1><1>YNPSLKG, wherein <1> is an equimolar mixture of each of amino acid residues A, D, E, F, G, H, I, K, L, M, N, P, Q, R, S, T, V, W and Y; and <4> and <5> are as defined above;
   (4) <1>I<8>S<1><1><1>GGYY<1>YAASVKG, wherein <1> is an equimolar mixture of each amino acid residues A, D, E, F, G, H, I, K, L, M, N, P, Q, R, S, T, V, W and Y; <8> is 0.27 R and 0.027 of each of ADEFGHIKLMNPQSTVWY; and
   (5) mixtures of vectors or genetic packages characterized by any of the above DNA sequences, preferably in the ratio: HC CDR2s: (1)/(2) (equimolar): (3):(4)::0.54:0.43:0.03.
3. A focused DNA library as defined in paragraph 4 below being characterized by further comprising variegated DNA sequences that encode a heavy chain CDR3 selected from the group consisting of:
   (1) YYCA21111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (2) YYCA2111111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (3) YYCA211111111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (4) YYCAR111S2S3111YFDYWG, wherein 1 is an equimolar mixture of each amino acid residues A, D, E, F, G, H, I, K, L, M, N, P, Q, R, S, T, V, W and Y; and 2 is an equimolar mixture of S and G; and 3 is an equimolar mixture of Y and W;
   (5) YYCA2111CSG11CY1YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (6) YYCA211S1TIFG11111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (7) YYCAR111YY2S3344111YFDYWG, wherein 1 is an equimolar mixture of each amino acid residues A, D, E, F, G, H, I, K, L, M, N, P, Q, R, S, T, V, W and Y; 2 is an equimolar mixture of D and G; and 3 is an equimolar mixture of S and G;
   (8) YYCAR1111YC2231CY111YFDYWG, wherein 1 is an equimolar mixture of each amino acid residues A, D, E, F, G, H, I, K, L, M, N, P, Q, R, S, T, V, W and Y; 2 is an equimolar mixture of S and G; and 3 is an equimolar mixture of T, D and G; and
   (9) mixtures of vectors or genetic packages characterized by any of the above DNA sequences, preferably the HC CDR3s (1) through (8) are in the following proportions in the mixture:
      (1) 0.10
      (2) 0.14
      (3) 0.25
      (4) 0.13
      (5) 0.13
      (6) 0.11
      (7) 0.04 and
      (8) 0.10; and more preferably the HC CDR3s (1) through (8) are in the following proportions in the mixture:
         (1) 0.02
         (2) 0.14
         (3) 0.25
         (4) 0.14
         (5) 0.14
         (6) 0.12
         (7) 0.08 and
         (8) 0.11.
   Preferably, 1 in one or all of HC CDR3s (1) through (8) is 0.095 of each of G and Y and 0.048 of each of A, D, E, F, H, I, K, L, M, N, P, Q, R, S, T, V, and W.
4. A focused DNA library encoding vectors or genetic packages that display, display and express, or comprise a member of a diverse family of human antibody related peptides, polypeptides and proteins and collectively display, display and express, or comprise at least a portion of the diversity of the antibody family, the vectors or genetic packages being characterized by variegated DNA sequences that encodes a kappa light chain CDR1 selected from the group consisting of:
   (1) RASQ<1>V<2><2><3>LA
   (2) RASQ<1>V<2><2><2><3>LA; wherein <1> is an equimolar mixture of amino acid residues ADEFGHIKLMNPQRSTVWY; <2> is 0.2 S and 0.044 of each of ADEFGHIKLMNPQRTVWY; and <3> is 0.2Y and 0.044 each of ADEFGHIKLMNPQRSTVW; and
   (3) mixtures of vectors or genetic packages characterized by any of the above DNA sequences, preferably in the ratio CDR1s (1) : (2) : :0.68:0.32; a kappa light chain CDR2 having the sequence:
      <1>AS<2>R<4><1>,
      wherein <1> is an equimolar mixture of amino acid residues ADEFGHIKLMNPQRSTVWY; <2> is 0.2 S and 0.044 of each of ADEFGHIKLMNPQRTVWY; and <4> is 0.2 A and )0.044 each of DEFGHIKLMNPQRSTVMY; and further comprising a kappa light chain CDR3 selected from the groups consisting of:
      (1) QQ<3><1><1><1>P<1>T,
         wherein <1> is an equimolar mixture of amino acid residues ADEFGHIKLMNPQRSTVWY; <3> is 0.2 Y and 0.044 each of ADEFGHIKLMNPQRTVW;
      (2) QQ33111P, wherein 1 and 3 are as defined in (1) above;
      (3) QQ3211PP1T, wherein 1 and 3 are as defined in (1) above and 2 is 0.2 S and 0.044 each of ADEFGHIKLMNPQRTVWY; and
      (4) mixtures of vectors or genetic packages characterized by any of the above DNA sequences, preferably in the ratio CDR3s (1) : (2) : (3) : :0.65: 0.1:0.25.
5. A focused DNA library as defined in paragraph 4 above being characterized by further comprising variegated DNA sequences that encode a lambda light chain CDR1 selected from the group consisting of:
   (1) TG<1>SS<2>VG<2><3><2><3>VS,
      wherein <1> is 0.27 T, 0.27 G and 0.027 each of ADEFHIKLMNPQRSVWY, <2> is 0.27 D, 0.27 N and 0.027 each of AEFGHIKLMPQRSTVWY, and <3> is 0.36 Y and 0.036 each of ADEFGHIKLMNPQRSTVW;
   (2) G<2><4>L<4><4><4><3><4><4>,
      wherein <2> is as defined in (1) above and <4> is an equimolar mixture of amino acid residues ADEFGHIKLMNPQRSTVWY; and
   (3) mixtures of vectors or genetic packages characterized by any of the above DNA sequences, preferably in the ratio CDR1s (1) : (2) : :0.67:0.33; and/or
      variegated DNA sequences that encode a lambda light chain CDR2 having the sequence:
      <4><4><4><2>RPS,
      wherein <2> is 0.27 D, 0.27 N, and 0.027 each of AEFGHIKLMPQRSTVWY and <4> is an equimolar mixture of amino acid residues ADEFGHIKLMNPQRSTVW; and/or variegated DNA sequences that encode a lambda light chain CDR3 selected from the group consisting of :
      (1) <4><5><4><2><4>S<4><4><4><4>V,
         wherein <2> is 0.27 D, 0.27 N, and 0.027 each of AEFGHIKLMPQRSTVWY; <4> is an equimolar mixture of amino acid residues ADEFGHIKLMNPQRSTVW; and <5> is 0.36 S and 0.0355 each of ADEFGHIKLMNPQRTVWY;
      (2) <5>SY<1><5>S<5><1><4>V, wherein <1> is an equimolar mixture of ADEFGHIKLMNPQRSTVWY; and <4> and <5> are as defined in (1) above; and
      (3) mixtures of vectors or genetic packages characterized by any of the above DNA sequences, preferably in the ratio CDR3s (1):(2)::1:1.
12. A population of variegated DNA sequences as described in paragraphs 1-5 above.
13. A population of vectors comprising the variegated DNA sequences as described in paragraphs 1-5 above.

### DETAILED DESCRIPTION OF THE PREFERRED EMBODIMENTS

Antibodies ("Ab") concentrate their diversity into those regions that are involved.in determining affinity and specificity of the Ab for particular targets. These regions may be diverse in sequence or in length. Generally, they are diverse in both ways. However, within families of human antibodies the diversities, both in sequence and in length, are not truly random. Rather, some amino acid residues are preferred at certain positions of the CDRs and some CDR lengths are preferred. These preferred diversities account for the natural diversity of the antibody family.

According to this invention, and as more fully described below, libraries of vectors and genetic packages that more closely mirror the natural diversity, both in sequence and in length, of antibody families, or portions thereof are prepared and used.

### Human Antibody Heavy Chain Sequence and Length Diversity

### (a) Framework

The heavy chain ("HC") Germ-Line Gene (GLG) 3-23 (also known as.VP-47) accounts for about 12% of all human Abs and is preferred as the framework in the preferred embodiment of the invention. It should, however, be understood that other well-known frameworks, such as 4-34, 3-30, 3-30.3 and 4-30.1, may also be used without departing from the principles of the focused diversities of this invention.

In addition, JH4 (YFDYWGQGTLVTUSS) occurs more often than JH3 in native antibodies. Hence, it is preferred for the focused libraries of this invention. However, JH3 (AFDIWGQGTMVTVSS) could as well be used.

### (b) Focused Length Diversity: CDR1, 2 and 3

### (i) CDR1

For CDR1, GLGs provide CDR1s only of the lengths 5, 6, and 7. Mutations during the maturation of the V-domain gene, however, can lead to CDR1s having lengths as short as 2 and as long as 16. Nevertheless, length 5 predominates. Accordingly, in the preferred embodiment of this invention, the preferred HC CDR1 is 5 amino acids, with less preferred CDR1s having lengths of 7 and 14. In the most preferred libraries of this invention, all three lengths are used in proportions similar to those found in natural antibodies.

### (ii) CDR2

GLGs provide CDR2s only of the lengths 15-19, but mutations during maturation may result in CDR2s of lengths from 16 to 28 amino acids. The lengths 16 and 17 predominate in mature Ab genes. Accordingly, length 17 is the preferred length for HC CDR2 of the present invention. Less preferred HC CDR2s of this invention have lengths 16 and 19. In the most preferred focused libraries of this invention, all three lengths are included in proportions similar to those found in natural antibody families.

### (iii) CDR3

HC CDR3s vary in length. About half of human HCs consist of the components: V::nz::D::ny::JHn where V is a V gene, nz is a series of bases (mean 12) that are essentially random, D is a D segment, often with heavy editing at both ends, ny is a series of bases (mean 6) that are essentially random, and JH is one of the six JH segments, often with heavy editing at the 5' end. The D segments appear to provide spacer segments that allow folding of the IgG. The greatest diversity is at the junctions of V with D and of D with JH.

In the preferred libraries of this invention both types of HC CDR3s are used. In HC CDR3s that have no identifiable D segment, the structure is V::nz::JHn where JH is usually edited at the 5' end. In HC CDR3s that have an identifiable D segment, the structure is V::nz::D::ny::JHn.

### (c) Focused Sequence Diversity: CDR1, 2 and 3

### (i) CDR1

In 5 amino acid length CDR1, examination of a 3D model of a humanized Ab showed that the side groups of residues 1, 3, and 5 were directed toward the combining pocket. Consequently, in the focused libraries of this invention, each of these positions may be selected from any of the native amino acid residues, except cysteine ("C"). Cysteine can form disulfide bonds, which are an important component of the canonical Ig fold. Having free thiol groups could, thus, interfere with proper folding of the HC and could lead to problems in production or manipulation of selected Abs. Thus, in the focused libraries of this invention cysteine is excluded from positions 1, 3 and 5 of the preferred 5 amino acid CDR1s. The other 19 natural amino acids residues may be used at positions 1, 3 and 5. Preferably, each is present in equimolar ratios in the variegated libraries of this invention.

3D modeling also suggests that the side groups of residue 2 in a 5 amino acid CDR1 are directed away from the combining pocket. Although this position shows substantial diversity, both in GLG and mature genes, in the focused libraries of this invention this residue is preferably Tyr (Y) because it occurs in 681/820 mature antibody genes. However, any of the other native amino acid residues, except Cys (C), could also be used at this position.

For position 4, there is also some diversity in GLG and mature antibody genes. However, almost all mature genes have uncharged hydrophobic amino acid residues: A, G, L, P, F, M, W, I, V, at this position. Inspection of a 3D model also shows that the side group of residue 4 is packed into the innards of the HC. Thus, in the preferred embodiment of this invention which uses framework 3-23, residue 4 is preferably Met because it is likely to fit very well into the framework of 3-23. With other frameworks, a similar fit consideration is used to assign residue 4.

Thus, the most preferred HC CDR1 of this invention consists of the amino acid sequence <1>Y<1>M<1> where <1> can be any one of amino acid residues: A, D, E, F, G, H, I, K, L, M, N, P, Q, R, S, T, V, W, Y (not C), preferably present at each position in an equimolar amount. This diversity is shown in the context of a framework 3-23:JH4 in Table 1. It has a diversity of 6859-fold.

The two less preferred HC CDR1s of this invention have length 7 and length 14. For length 7, a preferred variegation is (S/T)₁(S/G/<1>)₂(S/G/<1>)₃Y₄Y₅W₆(S/G/<1>)₇; where (S/T) indicates an equimolar mixture of Ser and Thr codons; (S/G/<1>) indicates a mixture of 0.2025 S, 0.2025 G, and 0.035 for each of A, D, E, F, H, I, K, L, M, N, P, Q, R, T, V, W, Y. This design gives a predominance of Ser and Gly at positions 2, 3, and 7, as occurs in mature HC genes. For length 14, a preferred variegation is VSGGSTS<1><1><1>YYW<1>, where <1> is an equimolar mixture of the 19 native amino acid residues, except Cys (C).

The DNA that encodes these preferred HC CDR1s is preferably synthesized using trinucleotide building blocks so that each amino acid residue is present in essentially equimolar or other described amounts. The preferred codons for the <1> amino acid residues are gct, gat, gag, ttt, ggt, cat, att, aag, ctt, atg, aat, cct, cag, cgt, tct, act, gtt, tgg, and tat. Of course, other codons for the chosen amino acid residue could also be used.

The diversity oligonucleotide (ON) is preferably synthesized from *Bsp*EI to BstXI (as shown in Table 1) and can, therefore, be incorporated either by PCR synthesis using overlapping ONs or introduced by ligation of BspEI/BstXI-cut fragments. Table 2 shows the oligonucleotides that embody the specified variegations of the preferred length 5 HC CDR1s of this invention. PCR using ON-R1V1vg, ON-Rltop, and ON-R1bot gives a dsDNA product of 73 base pairs, cleavage with BspEI and *Bst*XI trims 11 and 13 bases from the ends and provides cohesive ends that can be ligated to similarly cut vector having the 3-23 domain shown in Table 1. Replacement of ON-R1V1vg with either ONR1V2vg or ONR1V3vg (see Table 2) allows synthesis of the two alternative diversity patterns -- the 7 residue length and the 14 residue length HC CDR1.

The more preferred libraries of this invention comprise the 3 preferred HC CDR1 length diversities. Most preferably, the 3 lengths should be incorporated in approximately the ratios in which they are observed in antibodies selected without reference to the length of the CDRs. For example, one sample of 1095 HC genes have the three lengths present in the ratio: L=5:L=7:L=14::820:175:23::0.80:0.17:0.02. This is the preferred ratio in accordance with this invention.

### (ii) CDR2

Diversity in HC CDR2 was designed with the same considerations as for HC CDR1: GLG sequences, mature sequences and 3D structure. A preferred length for CDR2 is 17, as shown in Table 1. For this preferred 17 length CDR2, the preferred variegation in accordance with the invention is: <2>I<2><3>SGG<1>T<1>YADSVKG, where <2> indicates any amino acid residue selected from the group of Y, R, W, V, G and S (equimolar mixture), <3> is P, S and G or P and S only (equimolar mixture), and <1> is any native amino acid residue except C (equimolar mixture).

ON-R2V1vg shown in Table 3 embodies this diversity pattern. It is preferably synthesized so that fragments of dsDNA containing the *Bst*XI and *Xba*I site can be generated by PCR. PCR with ON-R2V1vg, ON-R2top,and ONR2bot gives a dsDNA product of 122 base pairs. Cleavage with BstXI and XbaI removes about 10 bases from each end and produces cohesive ends that can be ligated to similarly cut vector that contains the 3-23 gene shown in Table 1.

In an alternative embodiment for a 17 length HC CDR2, the following variegation may be used: <1>I<4><1><1>G<5><1><1><1>YADSVKG, where <1> is as described above for the more preferred alternative of HC CDR2; <4> indicates an equimolar mixture of DINSWY, and <5> indicates an equimolar mixture of SGDN. This diversity pattern is embodied in ON-R2V2vg shown in Table 3. Preferably, the two embodiments are used in equimolar mixtures in the libraries of this invention.

Other preferred HC CDR2s have lengths 16 and 19. Length 16: <1>I<4><1><1>G<5<1><1>YNPSLKG; Length 19: <1>I<8>S<1><1><1>GGYY<1>YAASVKG, wherein <1> is an equimolar mixture of all native amino acid residues except C; <4> is a equimolar mixture of DINSWY; <5> is an equimolar mixture of SGDN; and <8> is 0.27 R and 0.027 of each of residues ADEFGHIKLMNPQSTVWY. Table 3 shows ON-R2V3vg which embodies a preferred CDR2 variegation of length 16 and ON-R2V4vg which embodies a preferred CDR2 variegation of length 19. To prepare these variegations ON-R2V3vg may be PCR amplified with ON-R2top and ON-R2bo3 and ON-R2V4vg may be PCR amplified with ON-R2top and ON-R2-bo4. See Table 3. In the most preferred embodiment of this invention, all three HC CDR2 lengths are used. Preferably, they are present in a ratio 17:16:19::579:464:31::0.54:0.43:0.03.

### (iii) CDR3

The preferred libraries of this invention comprise several HC CDR3 components. Some of these will have only sequence diversity. Others will have sequence diversity with embedded D segments to extend the length, while also incorporating sequences known to allow Igs to fold. The HC CDR3 components of the preferred libraries of this invention and their diversities are depicted in Table 4: Components 1-8.

This set of components was chosen after studying the sequences of 1383 human HC sequences. The proposed components are meant to fulfill the following goals:
1) approximately the same distribution of lengths as seen in native Ab genes;
2) high level of sequence diversity at places having high diversity in native Ab genes; and
3) incorporation of constant sequences often seen in native Ab genes.

Component 1 represents all the genes having lengths 0 to 8 (counting from the YYCAR motif at the end of FR3 to the WG dipeptide motif near the start of the J region, i.e., FR4). Component 2 corresponds the all the genes having lengths 9 or 10. Component 3 corresponds to the genes having lengths 11 or 12 plus half the genes having length 13. Component 4 corresponds to those having length 14 plus half those having length 13. Component 5 corresponds to the genes having length 15 and half of those having length 16. Component 6 corresponds to genes of length 17 plus half of those with length 16. Component 7 corresponds to those with length 18. Component 8 corresponds to those having length 19 and greater. See Table 4.

For each HC CDR3 residue having the diversity <1>, equimolar ratios are preferably not used. Rather, the following ratios are used 0.095 [G and Y] and 0.048 [A, D, E, F, H, I, K, L, M, N, P, Q, R, S, T, V, and W]. Thus, there is a double dose of G and Y with the other residues being in equimolar ratios. For the other diversities, e.g., KR or SG, the residues are present in equimolar mixtures.

In the preferred libraries of this invention the eight components are present in the following fractions: 1 (0.10), 2 (0.14), 3 (0.25), 4 (0.13), 5 (0.13), 6 (0.11), 7 (0.04) and 8 (0.10). See Table 4.

In the more preferred embodiment of this invention, the amounts of the eight components is adjusted because the first component is not complex enough to justify including it as 10% of the library. For example, if the final library were to have 1 x 10⁹ members, then 1 x 10⁸ sequences would come from component 1, but it has only 2.6 x 10⁵ CDR3 sequences so that each one would occur in -385 CDR1/2 contexts. Therefore, the more preferred amounts of the eight components are 1(0.02), 2(0.14), 3(0.25), 4(0.14), 5(0.14), 6(0.12), 7(0.08), 8(0.11). In accordance with the more preferred embodiment component 1 occurs in -77 CDR1/2 contexts and the other, longer CDR3s occur more often.

Table 5 shows vgDNA that embodies each of the eight HC CDR3 components shown in Table 4. In Table 5, the oligonucleotides (ON) Ctop25, CtprmA, CBprmB, and CBot25 allow PCR amplification of each of the variegated ONs (vgDNA) : C1t08, C2t10, C3t12, C4t14, C5t15, C6t17, C7t18, and C8t19. After amplification, the dsDNA can be cleaved with *Afl*II and *Bst*EII (or *Kpn*I) and ligated to similarly cleaved vector that contains the remainder of the 3-23 domain. Preferably, this vector already contains diversity in one, or both, of CDR1 and CDR2 as disclosed herein. Most preferably, it contains diversity in both the CDR1 and CDR2 regions. It is, of course, to be understood that the various diversities can be incorporated into the vector in any order.

Preferably, the recipient vector originally contains a stuffer in place of CDR1, CDR2 and CDR3 so that there will be no parental sequence that would then occur in the resulting library. Table 6 shows a version of the V3-23 gene segment with each CDR replaced by a short segment that contains both stop codons and restriction sites that will allow specific cleavage of any vector that does not have the stuffer removed. The stuffer can either be short and contain a restriction enzyme site that will not occur in the finished library, allowing removal of vectors that are not cleaved by both *Afl*II and *Bst*EII (or *Kpn*I) and religated. Alternatively, the stuffer could be 200-400 bases long so that uncleaved or once cleaved vector can be readily separated from doubly cleaved vector.

### Human Antibody Light Chain: Sequence and Length Diversity

### (i) Kappa Chain

### (a) Framework

In this invention, the kappa light chain is built in an A27 framework with a JK1 region. These are the most common V and J regions in the native genes. Other frameworks, such as O12, L2, and A11, and other J regions, such as JK4, however, may be used without departing from the scope of this invention.

### (b) CDR1

In native human kappa chains, CDR1s with lengths of 11, 12, 13, 16, and 17 were observed with length 11 being predominant and length 12 being well represented. Thus, in this invention LC CDR1s of length 11 and 12 are used in an and mixture similar to that observed in native antibodies), length 11 being most preferred. Length 11 has the following sequence: RASQ<1>V<2><2><3>LA and Length 12 has the following sequence: RASQ<1>V<2><2><2><3>LA, wherein <1> is an equimolar mixture of all of the native amino acid residues, except C, <2> is 0.2 S and 0.044 of each of ADEFGHIKLMNPQRTVWY, and <3> is 0.2 Y and 0.044 each of A, D, E, F, G, H, I, K, L, M, N, P, Q, R, ST, V, W. In the most preferred embodiment of this invention, both CDR1 lengths are used. Preferably, they are present in a ratio of 11:12::154:73::0.68:0.32.

### (c) CDR2

In native kappa, CDR2 exhibits only length 7. This length is used in this invention. It has the sequence <1>AS<2>R<4><1>, wherein <1> is an equimolar mixture of amino acid residues ADEFGHIKLMNPQRSTVWY; <2> is 0.2 S and 0.004 of each of ADEFGHIKLMNPQRTVWY; and <4> is 0.2 A and 0.044 of each of DEFGHIKLMNPQRSTUWY.

### (d) CDR3

In native kappa, CDR3 exhibits lengths of 1, 4, 6, 7, 8, 9, 10, 11, 12, 13, and 19. While any of these lengths and mixtures of them can be employed in this invention, we prefer lengths 8, 9 and 10, length 9 being more preferred. For the preferred Length 9, the sequence is QQ<3><1><1><1>P<1>T, wherein <1> is an equimolar mixture of amino acid residues ADEFGHIKLMNPQRSTVWY and <3> is 0.2 Y and 0.044 each of ADEFGHIKLMNPQRSVW. Length 8 is preferably QQ33111P and Length 10 is preferably QQ3211PP1T, wherein 1 and 3 are as defined for Length 9 and 2 is S (0.2) and 0.044 each of ADEFGHIKLMNPQRTVWY. A mixture of all 3 lengths being most preferred (ratios as in native antibodies), i.e., 8:9:10::28:165:63::0.1:0.65:0.25.

Table 7 shows a kappa chain gene of this invention, including a PlacZ promoter, a ribosome-binding site, and signal sequence (M13 III signal). The DNA sequence encodes the GLG amino acid sequence, but does not comprise the GLG DNA sequence. Restriction sites are designed to fall within each framework region so that diversity can be cloned into the CDRs. XmaI and EspI are in FR1, SexAI is in FR2, RsrII is in FR3, and *Kpn*I (or Acc65I) are in FR4. Additional sites are provided in the constant kappa chain to facilitate construction of the gene.

Table 7 also shows a suitable scheme of variegation for kappa. In CDR1, the most preferred length 11 is depicted. However, most preferably both lengths 11 and 12 are used. Length 12 in CDR1 can be construed by introducing codon 51 as <2> (i.e. a Ser-biased mixture). CDR2 of kappa is always 7 codons. Table 7 shows a preferred variegation scheme for CDR2. Table 7 shows a variegation scheme for the most preferred CDR3 (length 9). Similar variegations can be used for CDRs of length 8 and 10. In the preferred embodiment of this invention, those three lengths (8, 9 and 10) are included in the libraries of this invention in the native ratios, as described above.

Table 9 shows series of diversity oligonucleotides and primers that may be used to construct the kappa chain diversities depicted in Table 7.

### (ii) Lambda Chain

### (a) Framework

The lambda chain is preferably built in a 2a2 framework with an L2J region. These are the most common V and J regions in the native genes. Other frameworks, such as 31, 4b, 1a and 6a, and other J regions, such as L1J, L3J and L7J, however, may be used without departing from the scope of this invention.

### (b) CDR1

In native human lambda chains, CDR1s with length 14 predominate, lengths 11, 12 and 13 also occur. While any of these can be used in this invention, lengths 11 and 14 are preferred. For length 11 the sequence is: TG<2><4>L<4><4><4><3><4><4> and for Length 14 the sequence is: TG<1>SS<2>VG<1><3><2><3>VS, wherein <1> is 0.27 T, 0.27 G and 0.027 each of ADEFHIKLMNPQRSVWY; <2> is 0.27 D, 0.27 N and 0.027 each of AEFGHIKLMPQRSTVMY; <3> is 0.36 Y and 0.0355 each of ADEFGHIKLMNPQRSTVW; and <4> is an equimolar mixture of amino acid residues ADEFGHIKLMNPQRSTVWY. Most preferably, mixtures (similar to those occurring in native antibodies) preferably, the ratio is 11:14::23:46::0.33: 0.67 of the three lengths are used.

### (c) CDR2

In native human lambda chains, CDR2s with length 7 are by far the most common. This length is preferred in this invention. The sequence of this Length 7 CDR2 is <4><4><4><2>RPS, wherein <2> is 0.27 D, 0.27 N, and 0.027 each of AEFGHIKLMPQRSTVWY and <4> is an equimolar mixture of amino acid residues ADEFGHIKLMNPQRSTVW.

### (d) CDR3

In native human lambda chains, CDR3s of length 10 and 11 predominate, while length 9 is also common. Any of these three lengths can be used in the invention. Length 11 is preferred and mixtures of 10 and 11 more preferred. The sequence of Length 11 is <4><5><4><2><4>S<4><4><4><4>V, where <2> and <4> are as defined for the lambda CDR1 and <5> is 0.36 S and 0.0355 each of ADFFGHIKLMNPQRTVWY. The sequence of Length 10 is <5>SY<1><5>S<5><1><4>V, wherein <1> is an equimolar mixture of ADEFGHIKLMNPQRSTVWY; and <4> and <5> are as defined for Length 11. The preferred mixtures of this invention comprise an equimolar mixture of Length 10 and Length 11. Table 8 shows a preferred focused lambda light chain diversity in accordance with this invention.

Table 9 shows a series of diversity oligonucleotides and primers that may be used to construct the lambda chain diversities depicted in Table 7.

### Method of Construction of the Genetic Package

The diversities of heavy chain and the kappa and lambda light chains are best constructed in separate vectors. First a synthetic gene is designed to embody each of the synthetic variable domains. The light chains are bounded by restriction sites for *Apa*LI (positioned at the very end of the signal sequence) and *Asc*I (positioned afer the stop codon). The heavy chain is bounded by *Sfi*I (positioned within the PelB signal sequence) and *Not*I (positioned in the linker between CH1 and the anchor protein). Signal sequences other than Pe1B may also need, e.g., a M13 pIII signal sequence.

The initial genes are made with "stuffer" sequences in place of the desired CDRs. A "Stuffer" is a sequence that is to be cut away and replaced by diverse DNA but which does not allow expression of a functional antibody gene. For example, the stuffer may contain several stop codons and restriction sites that will not occur in the correct finished library vector. For example, in Table 10, the stuffer for CDR1 of kappa A27 contains a *Stu*I site. The vgDNA for CDR1 is introduced as a cassette from *Esp*I, *Xma*I, or *Afl*II to either *Sex*AI or *Kas*I. After the ligation, the DNA is cleaved with *Stu*I; there should be no *Stu*I sites in the desired vectors.

The sequences of the heavy chain gene with stuffers is depicted in Table 6. The sequences of the kappa light chain gene with stuffers is depicted in Table 10. The sequence of the lambda light chain gene with stuffers is depicted in Table 11.

In another embodiment of the present intention the diversities of heavy chain and the kappa or lambda light chains are constructed in a single vector or genetic packages (e.g., for display or display and expression) having appropriate restriction sites that allow cloning of these chains. The processes to construct such vectors are well known and widely used in the art. Preferably, a heavy chain and Kappa light chain library and a heavy chain and lambda light chain library would be prepared separately. The two libraries, most preferably, will then be mixed in equimolar amounts to attain maximum diversity.

Most preferably, the display is had on the surface of a derivative of M13 phage. The most preferred vector contains all the genes of M13, an antibiotic resistance gene, and the display cassette. The preferred vector is provided with restriction sites that allow introduction and excision of members of the diverse family of genes, as cassettes. The preferred vector is stable against rearrangement under the growth conditions used to amplify phage.

In another embodiment, the diversity captured by the methods of the present disclosure may be displayed and/or expressed in a phagemid vector (e.g., pCES1) that displays and/or expresses the peptide, polypeptide or protein. Such vectors may also be used to store the diversity for subsequent display and/or expression using other vectors or phage.

In another embodiment, the diversity captured by the methods of the present disclosure may be displayed and/or expressed in a yeast vector.

Particularly disclosed are the following aspects:
1. A focused library of vectors or genetic packages that display, display and express, or comprise a member of a diverse family of human antibody related peptides, polypeptides and proteins and collectively display, display and express, or comprise at least a portion of the diversity of the antibody family, the vectors or genetic packages being characterized by variegated DNA sequences that encode a heavy chain CDR1 selected from the group consisting of:
   (1) <1>₁Y₂<1>₃M₄<1>₅, wherein <1> is an equimolar mixture of each of amino acid residues A, D, E, F, G, H, I, K, L, M, N, P, Q, R, S, T, V, W, and Y;
   (2) (S/T)1(S/G/X)₂(S/G/X)₃Y₄Y₅W₆(S/G/X)₇. wherein (S/T) is a 1:1 mixture of S and T residues, (S/G/X) is a mixture of 0.2025 S, 0.2025 G and 0.035 of each of amino acid residues A, D, E, F, H, I, K, L, M, N, P, Q, R, T, V, W, and Y;
   (3)V₁S₂G₃G₄S₅I₆S₇<1>₈<1>₉<1>₁₀Y₁₁Y₁₂W₁₃<1>₁₄, wherein <1> is an equimolar mixture of each of amino acid residues A, D, E, F, G, H, I, K, L, M, N, P, Q, R, S, T, V, W, and Y; and
   (4) mixtures of vectors or genetic packages characterized by any of the above DNA sequences.
2. The focused library according to aspect 1, wherein HC CDR1s (1), (2) and (3) are present in the library in the ratio 0.80:0.17:0.02.
3. A focused library of vectors or genetic packages that display, display and express, or comprise a member of a diverse family of human antibody related peptides, polypeptides and proteins and collectively display, display and express, or comprise at least a portion of the diversity of the antibody facility, the vectors or genetic packages being characterized by variegated DNA sequences that encode a heavy chain CDR2 selected from the group consisting of:
   (1) <2>I<2><3>SGG<1>T<1>YADSVKG, wherein <1> is an equimolar mixture of each of amino acid residues A, D, E, F, G, H, I, K, L, M, N, P, Q, R, S, T, V, W, and Y; <2> is an equimolar mixture of each of amino acid residues Y, R, W, V, G, and S; and <3> is an equimolar mixture of each of amino acid residues P, S, and G or an equimolar mixture of P and S;
   (2) <1>I<4><1><1><G><5><1><1><1>YADSVKG, wherein <1> is an equimolar mixture of each of amino acid residues A, D, E, F, G, H, I, K, L, M, N, P, Q, R, S, T, V, W, and Y; <4> is an equimolar mixture of residues D, I, N, S, W, Y; and <5> is an equimolar mixture of residues S, G, D and N;
   (3) <1>I<4><1><1><G><5><1><1>YNPSLKG, wherein <1> is an equimolar mixture of each of amino acid residues A, D, E, F, G, H, I, K, L, M, N, P, Q, R, S, T, V, W and Y; and <4> and <5> are as defined above;
   (4) <1>I<8>S<1><1><1>GGYY<1>YAASVKG, wherein <1> is an equimolar mixture of each amino acid residues A, D, E, F, G, H, I, K, L, M, N, P, Q, R, S, T, V, W and Y; <8> is 0.27 R and 0.027 of each of ADEFGHIKLMNPQSTVWY; and
   (5) mixtures of vectors or genetic packages characterized by any of the above DNA sequences.
4. The focused library according to aspect 3 wherein a mixture of HC CDR2s (1)/(2) (equimolar), (3) and (4) are present in the library in a ratio of 0.54:0.43:0.03.
5. A focused library of vectors or genetic packages that display, display and express, or comprise a member of a diverse family of human antibody related peptides, polypeptides and proteins and collectively display, display and express, or comprise at least a portion of the diversity of the antibody family, the vectors or genetic packages being characterized by variegated DNA sequences that encode a heavy chain CDR3 selected from the group consisting of:
   (1) YYCA21111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (2) YYCA2111111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (3) YYCA211111111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (4) YYCAR111S2S3111YFDYWG, wherein 1 is an equimolar mixture of each amino acid residues A, D, E, F, G, H, I, K, L, M, N, P, Q, R, S, T, V, W and Y; and 2 is an equimolar mixture of S and G; and 3 is an equimolar mixture of Y and W;
   (5) YYCA2111CSG11CY1YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (6) YYCA211S1TIFG11111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (7) YYCAR111YY2S3344111YFDYWG, wherein 1 is an equimolar mixture of each amino acid residues A, D, E, F, G, H, I, K, L, M, N, P, Q, R, S, T, V, W and Y; 2 is an equimolar mixture of D and G; and 3 is an equimolar mixture of S and G;
   (8) YYCAR1111YC2231CY111YFDYWG, wherein 1 is an equimolar mixture of each amino acid residues A, D, E, F, G, H, I, K, L, M, N, P, Q, R, S, T, V, W and Y; 2 is an equimolar mixture of S and G; and 3 is an equimolar mixture of T, D and G; and
   (9) mixtures of vectors or genetic packages characterized by any of the above DNA sequences.
6. The focused library according to aspect 5, wherein 1 in one or a11 of HC CDR3s (1) through (8) is 0.095 of each of G and Y and 0.048 of each of A, D, E, F, H, I, K, L, M, N, P, Q, R, S, T, V, and W.
7. The focused library according to aspects 5 or 6, wherein HC CDR3s (1) through (8) are present in the library in the following proportions:
   (1) 0.10
   (2) 0.14
   (3) 0.25
   (4) 0.13
   (5) 0.13
   (6) 0.11
   (7) 0.04 and
   (8) 0.10
8. The focused library according to aspects 5 or 6, wherein the HC CDR3s (1) through (8) are present in the library in the following proportions:
   (1) 0.02
   (2) 0.14
   (3) 0.25
   (4) 0.14
   (5) 0.14
   (6) 0.12
   (7) 0.08 and
   (8) 0.11
9. A focused library of vectors or genetic packages that display, display and express, or comprise a member of a diverse family of human antibody related peptides, polypeptides and proteins and collectively display, display and express, or comprise at least a portion of the diversity of the antibody family, the vectors or genetic packages being characterized by variegated DNA sequences that encodes a kappa light chain CDR1 selected from the group consisting of:
   (1) RASQ<1>V<2><2><3>LA
   (2) RASQ<1>V<2><2><2><3>LA;
   wherein <1> is an equimolar mixture of amino acid residues ADEFGHIKLMNPQRSTVWY; <2> is 0.2 S and 0.044 of each of ADEFGHIKLMNPQRTVMY; and <3> is 0.2Y and 0.044 each of ADEFGHIKLMNPQRSTVW; and (3) mixtures of vectors or genetic packages characterized by any of the above DNA sequences.
10. The focused library of aspect 9, wherein CDR1s (1) and (2) are present in the library in a ratio of 0.68:0.32.
11. A focused library of vectors or genetic packages that display, display and express, or comprise a member of a diverse family of human antibody related peptides, polypeptides and proteins and collectively display, display and express, or comprise at least a portion of the diversity of the antibody family, the vectors or genetic packages being characterized by variegated DNA sequences that encode a kappa light chain CDR2 having the sequence: <1>AS<2>R<4><1>, wherein <1> is an equimolar mixture of amino acid residues ADEFGHIKLMNPQRSTVWY; <2> is 0.2 S and 0.044 of each of ADEFGHIKLMNPQRTVWY; and <4> is 0.2 A and) 0.044 each of DEFGHIKLMPQRSTVWY.
12. A focused library of vectors or genetic packages that display, display and express, or comprise a member of a diverse family of human antibody related peptides, polypeptides and proteins and collectively display, display and express, or comprise at least a portion of the diversity of the antibody family, the vectors or genetic packages being characterized by variegated DNA sequences that encode a kappa light chain CDR3 selected from the groups consisting of:
   (1) QQ<3><1><1><1>P<1>T, wherein <1> is an equimolar mixture of amino acid residues ADEFGHIKLMNPQRSTVWY; <3> is 0.2 Y and 0.044 each of ADEFGHIKLMNPQRTVW;
   (2) QQ33111P, wherein 1 and 3 are as defined in (1) above;
   (3) QQ3211PP1T, wherein 1 and 3 are as defined in (1) above and 2 is 0.2 S and 0.044 each of ADEFGHIKLMNPQRTVWY; and
   (4) mixtures of vectors or genetic packages characterized by any of the above DNA sequences.
13. The focused library according to aspect 12, wherein CDR3s (1), (2) and (3) are present in the library in a ratio of 0.65:0.1:0.25.
14. A focused library of vectors or genetic packages that display, display and express, or comprise a member of a diverse family of human antibody related peptides, polypeptides and proteins and collectively display, display and express, or comprise at least a portion of the diversity of the antibody family, the vectors or genetic packages being characterized by variegated DNA sequences that encode a lambda light chain CDR1 selected from the group consisting of:
   (1) TG<1>SS<2>VG<1><3><2><3>VS,
      wherein <1> is 0.27 T, 0.27 G and 0.027 each of ADEFHIKLMNPQRSVWY, <2> is 0.27 D, 0.27 N and 0.027 each of AEFGHIKLMPQRSTVWY, and <3> is 0.36 Y and 0.036 each of ADEFGHIKLMNPQRSTVW;
   (2) G<2><4>L<4><4><4><3><4><4>,
      wherein <2> is as defined in (1) above and <4> is an equimolar mixture of amino acid residues ADEFGHIKLMNPQRSTVWY ; and
   (3) mixtures of vectors or genetic packages characterized by any of the above DNA sequences.
15. The focused library according to aspect 14, where CDR1s (1) and (2) are present in the library in a ratio of 0.67:0.33.
16. A focused library of vectors or genetic packages that display, display and express, or comprise a member of a diverse family of human antibody related peptides, polypeptides and proteins and collectively display, display and express, or comprise at least a portion of the diversity of the antibody family, the vectors or genetic packages being characterized by variegated DNA sequences that encode a lambda light chain CDR2 has the sequence: <4><4><4><2>RPS, wherein <2> is 0.27 D, 0.27 N, and 0.027 each of AEFGHIKLMPQRSTVWY and <4> is an equimolar mixture of amino acid residues ADEFGHIKLMNPQRSTVW.
17. A focused library of vectors or genetic packages that display, display and express, or comprise a member of a diverse family of human antibody related peptides, polypeptides and proteins and collectively display, display and express, or comprise at least a portion of the diversity of the antibody family, the vectors or genetic packages being characterized by variegated DNA sequences that encode a lambda light chain CDR3 selected from the group consisting of:
   (1) <4><5><4><2><4>S<4><4><4><4>V, wherein <2> is 0.27 D, 0.27 N, and 0.027 each of AEFGHIKLMPQRSTVWY; <4> is an equimolar mixture of amino acid residues ADEFGHIKLMNPQRSTVW; and <5> is 0.36 S and 0.0355 each of ADEFGHIKLMNPQRTVWY;
   (2) <5>SY<1><5>S<5><1><4>V, wherein <1> is an equimolar mixture of ADEFGHIKLMNPQRSTVWY; and <4> and <5> are as defined in (1) above; and
   (3) mixtures of vectors or genetic packages characterized by any of the above DNA sequences.
18. The focused library according to aspect 17, wherein CDR3s (1) and (2) are present in the library in an equimolar mixture.
19. The focused library according to aspect 1 or 2 further comprising variegated DNA sequences that encode a heavy chain CDR selected from the group consisting of:
   (1) one or more of the heavy chain CDR2s defined in aspect 3 or 4;
   (2) one or more of the heavy chain CDR3s defined in aspects 5, 6, 7, or 8; and
   (3) mixtures of vectors or genetic packages characterized by (1) and (2).
20. The focused library according to aspct 3 further comprising variegated DNA sequences that encodes one or more heavy chain CDR3s selected from the group defined in aspects 5, 6, 7 or 8.
21. The focused library according to aspect 19 or 20, further comprising variegated DNA sequences that encodes a light chain CDR selected from the group consisting of
   (1) one or more the kappa light chain CDR1s defined in aspect 9 or 10;
   (2) the kappa light chain CDR2 defined in aspect 11;
   (3) one or more of the kappa light chain CDR3s defined in aspect 12 or 13;
   (4) one or more of the kappa light chain CDR1s defined in aspect 14 or 15;
   (5) the lambda light chain CDR2 defined in aspect 16;
   (6) one or more of the lambda light chain CDR3s defined in aspect 17 or 18; and
   (7) mixtures of vectors and genetic packages characterized by one or more of (1) through (6).
22. A population of variegated DNA sequences that encode a heavy chain CDR1 selected from the group consisting of:
   (1) <1>₁Y₂<1>₃M₄<1>₅, wherein <1> is an equimolar mixture of each of amino acid residues A, D, E, F, G, H, I, K, L, M, N, P, Q, R, S, T, V, W, and Y;
   (2) (S/T)₁(S/G/X)₂(S/G/X)₃Y₄Y₅W₆(S/G/X)₇, wherein (S/T) is a 1:1 mixture of S and T residues, (S/G/X) is a mixture of 0.2025 S, 0.2025 G and 0.035 of each of amino acid residues A, D, E, F, H, I, K, L, M, N, P, Q, R, T, V, W, and Y;
   (3) V₁S₂G₃G₄S₅I₆S₇<1>₈<1>₉<1>₁₀Y₁₁Y₁₂W₁₃<1>₁₄, wherein <1> is an equimolar mixture of each of amino acid residues A, D, E, F, G, H, I, K, L, M, N, P, Q, R, S, T, V, W, and Y; and (4) mixtures of variegated DNA sequences characterized by any of the above DNA sequences.
23. The population of variegated DNA sequences according to aspect 22, wherein HC CDR1s (1), (2) and (3) are present in the population in the ratio 0.80:0.17:0.02.
24. A population of variegated DNA sequences that encode a heavy chain CDR2 selected from the group consisting of:
   (1) <2>I<2><3>SGG<1>T<1>YADSVKG, wherein <1> is an equimolar mixture of each of amino acid residues A, D, E, F, G, H, I, K, L, M, N, P, Q, R, S, T, V, W, and Y; <2> is an equimolar mixture of each of amino acid residues Y, R, W, V, G, and S; and <3> is an equimolar mixture of each of amino acid residues P, S, and G or an equimolar mixture of P and S;
   (2) <1>I<4><1><1><G><5><1><1><1>YADSVKG, wherein <1> is an equimolar mixture of each of amino acid residues A, D, E, F, G, H, I, K, L, M, N, P, Q, R, S, T, V, W, and Y; <4> is an equimolar mixture of residues D, I, N, S, W, Y; and <5> is an equimolar mixture of residues S, G, D and N;
   (3) <1>I<4><1><1>G<5><1><1>YNPSLKG, wherein <1> is an equimolar mixture of each of amino acid residues A, D, E, F, G, H, I, K, L, M, N, P, Q, R, S, T, V, W and Y; and <4> and <5> are as defined above;
   (4) <1>I<8>S<1><1><1>GGYY<1>YAASVKG, wherein <1> is an equimolar mixture of each amino acid residues A, D, E, F, G, H, I, K, L, M, N, P, Q, R, S, T, V, W and Y; <8> is 0.27 R and 0.027 of each of ADEFGHIKLMNPQSTVWY; and (5) mixtures of variegated DNA sequences characterized by any of the above DNA sequences.
25. The population of variegated DNA sequences according to aspect 24, wherein a mixture of HC CDR2s (1)/ (2) (equimolar), (3) and (4) are present in the population in a ratio of 0.54:0.43:0.03.
26. A population of variegated DNA sequences that encode a heavy chain CDR3 selected from the group consisting of:
   (1) YYCA21111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (2) YYCA2111111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (3) YYCA211111111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (4) YYCAR111S2S3111YFDYWG, wherein 1 is an equimolar mixture of each amino acid residues A, D, E, F, G, H, I, K, L, M, N, P, Q, R, S, T, V, W and Y; and 2 is an equimolar mixture of S and G; and 3 is an equimolar mixture of Y and W;
   (5) YYCA2111CSG11CY1YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (6) YYCA211S1TIFG11111YFDYWG, wherein 1 is an equimolar mixture of each amino acid residues A, D, E, F, G, H, I, K, L, M, N, P, Q, R, S, T, V, W and Y; and 2 is an equimolar mixture of K and R;
   (7) YYCAR111YY2S3344111YFDYWG, wherein 1 is an equimolar mixture of each amino acid residues A, D, E, F, G, H, I, K, L, M, N, P, Q, R, S, T, V, W and Y; 2 is an equimolar mixture of D and G; and 3 is an equimolar mixture of S and G;
   (8) YYCAR1111YC2231CY111YFDYWG, wherein 1 is an equimolar mixture of each amino acid residues A, D, E, F, G, H, I, K, L, M, N, P, Q, R, S, T, V, W and Y; 2 is an equimolar mixture of S and G; and 3 is an equimolar mixture of T, D and G; and (9) mixtures of variegated DNA sequences characterized by any of the above DNA sequences.
27. The population of variegated DNA according to aspect 26, wherein 1 in one or all of HC CDR3s (1) through (8) is 0.095 of each of G and Y and 0.048 of each of A, D, E, F, H, I, K, L, M, N, P, Q, R, S, T, V, and W.
28. The population of variegated DNA sequences according to aspect 26 or 27, wherein HC CDR3s (1) through (8) are present in the population in the following proportions:
   (1) 0.10
   (2) 0.14
   (3) 0.25
   (4) 0.13
   (5) 0.13
   (6) 0.11
   (7) 0.04 and
   (8) 0.10
29. The population of variegated DNA sequences according to aspect 26 or 27, wherein the HC CDR3s (1) through (8) are present in the population in the following proportions:
   (1) 0.02
   (2) 0.14
   (3) 0.25
   (4) 0.14
   (5) 0.14
   (6) 0.12
   (7) 0.08 and
   (8) 0.11
30. A population of variegated DNA sequences that encode a kappa light chain CDR1 selected from the group consisting of:
   (1) RASQ<1>V<2><2><3>LA
   (2) RASQ<1>V<2><2><2><3>LA;
      wherein <1> is an equimolar mixture of amino acid residues ADEFGHIKLMNPQRSTVWY; <2> is 0.2 S and 0.044 of each of ADEFGHIKLMNPQRTVWY; and <3> is 0.2Y and 0.044 each of ADEFGHIKLMNPQRSTUW; and
   (3) mixtures of variegated DNA sequences characterized by any of the above DNA sequences.
31. The population of variegated DNA sequences of aspect 30, wherein CDRls (1) and (2) are present in the population in a ratio of 0.68:0.32.
32. A population of variegated DNA sequences that encode a kappa light chain CDR2 having the sequence: <1>AS<2>R<4><1>, wherein <1> is an equimolar mixture of amino acid residues ADEFGHIKLMNPQRSTVWY; <2> is 0.2 S and 0.044 of each of ADEFGHIKLMNPQRTVWY; and <4> is 0.2 A and) 0.044 each of DEFGHIKLMNPQRSTVWY.
33. A population of variegated DNA sequences that encode a kappa light chain CDR3 selected from the groups consisting of:
   (1) QQ<3><1><1><1>P<1>T, wherein <1> is an equimolar mixture of amino acid residues ADEFGHIKLMNPQRSTVWY; <3> is 0.2 Y and 0.044 each of ADEFGHIKLMNPQRTVW;
   (2) QQ33111P, wherein 1 and 3 are as defined in (1) above;
   (3) QQ3211PP1T, wherein 1 and 3 are as defined in (1) above and 2 is 0.2 S and 0.044 each of ADEFGHIKLMNPQRTVWY; and
   (4) mixtures of variegated DNA sequences characterized by any of the above DNA sequences.
34. The population of variegated DNA sequences according to aspect 33, wherein CDR3s (1), (2) and (3) are present in the population in a ratio of 0.65:0.1:0.25.
35. A population of variegated DNA sequences that encode a lambda light chain CDR1 selected from the group consisting of:
   (1) TG<1>SS<2>VG<1><3><2><3>VS, wherein <1> is 0.27 T, 0.27 G and 0.027 each of ADEFHIKLMNPQRSVWY, <2> is 0.27 D, 0.27 N and 0.027 each of AEFGHIKLMPQRSTVWY, and <3> is 0.36 Y and 0.036 each of ADEFGHIKLMNPQRSTVW;
   (2) G<2><4>L<4><4><4><3><4><4>, wherein <2> is as defined in (1) above and <4> is an equimolar mixture of amino acid residues ADEFGHIKLMNPQRSTVWY; and (3) mixtures of variegated DNA sequences characterized by any of the above DNA sequences.
36. The population of variegated DNA sequences according to aspect 35, where CDR1s (1) and (2) are present in the population in a ratio of 0.67:0.33.
37. A population of variegated DNA sequences that encode a lambda light chain CDR2 has the sequence: <4><4><4><2>RPS, wherein <2> is 0.27 D, 0.27 N, and 0.027 each of AEFGHIKLMPQRSTVWY and <4> is an equimolar mixture of amino acid residues ADEFGHIKLMNPQRSTVW.
38. A population of variegated DNA sequences that encode a lambda light chain CDR3 selected from the group consisting of:
   (1) <4><5><4><2><4>S<4><4><4><4>V, wherein <2> is 0.27 D, 0.27 N, and 0.027 each of AEFGHIKLMPQRSTVWY; <4> is an equimolar mixture of amino acid residues ADEFGHIKLMNPQRSTVW; and <5> is 0.36 S and 0.0355 each of ADEFGHIKLMNPQRTVWY;
   (2) <5>SY<1><5>S<5><1><4>V, wherein <1> is an equimolar mixture of ADEFGHIKLMNPQRSTVWY; and <4> and <5> are as defined in (1) above; and
   (3) mixtures of variegated DNA sequence characterized by any of the above DNA sequences.
39. The population of variegated DNA sequences according to aspect 38, wherein CDR3s (1) and (2) are present in the population in an equimolar mixture.
40. The population of variegated DNA sequences according to aspect 22 or 23 further comprising variegated DNA sequences that encode a heavy chain CDR selected from the group consisting of:
   (1) one or more of the heavy chain CDR2s defined in aspect 24 or 25;
   (2) one or more of the heavy chain CDR3s defined in aspects 26, 27, 28 or 29; and
   (3) mixtures of variegated DNA sequence characterized by (1) and (2).
41. The population of variegated DNA sequences according to aspect 24 further comprising variegated DNA sequences that encodes one or more heavy chain CDR3s selected from the group defined in aspects 26, 27, 28 or 29.
42. The population of variegated DNA sequences according to aspect 40 or 41 further comprising variegated DNA sequences that encodes a light chain CDR selected from the group consisting of
   (1) one or more the kappa light chain CDR1s defined in aspect 30 or 31;
   (2) the kappa light chain CDR2 defined in aspect 32;
   (3) one or more of the kappa light chain CDR3s defined in aspect 33 or 34;
   (4) one or more of the kappa light chain CDR1s defined in aspect 35 or 36;
   (5) the lambda light chain CDR2 defined in aspect 37;
   (6) one or more of the lambda light chain CDR3s defined in aspect 38 or 39; and
   (7) mixtures of variegated DNA sequences characterized by one or more of (1) through (6).
43. A population of vectors comprising the variegated DNA sequences of any one of aspects 22-42.

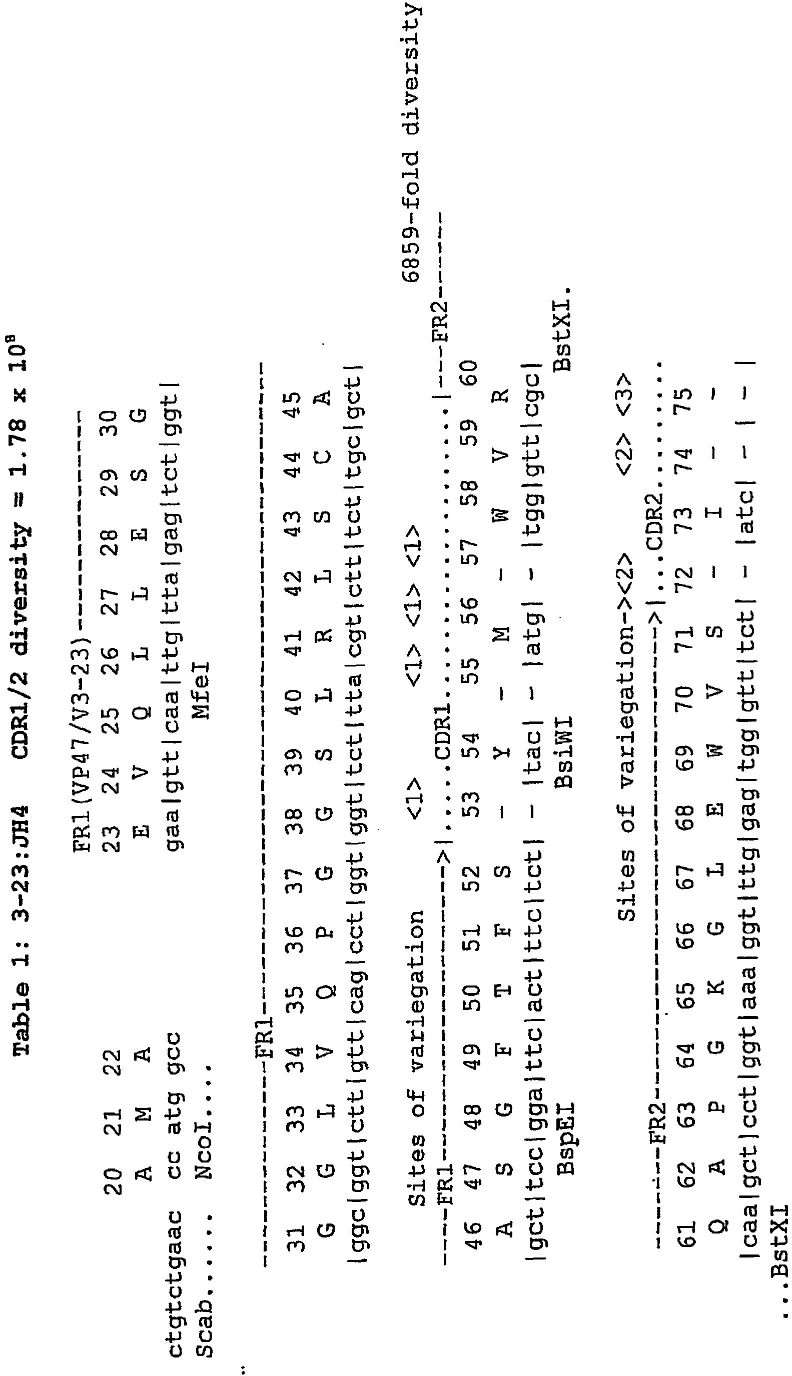

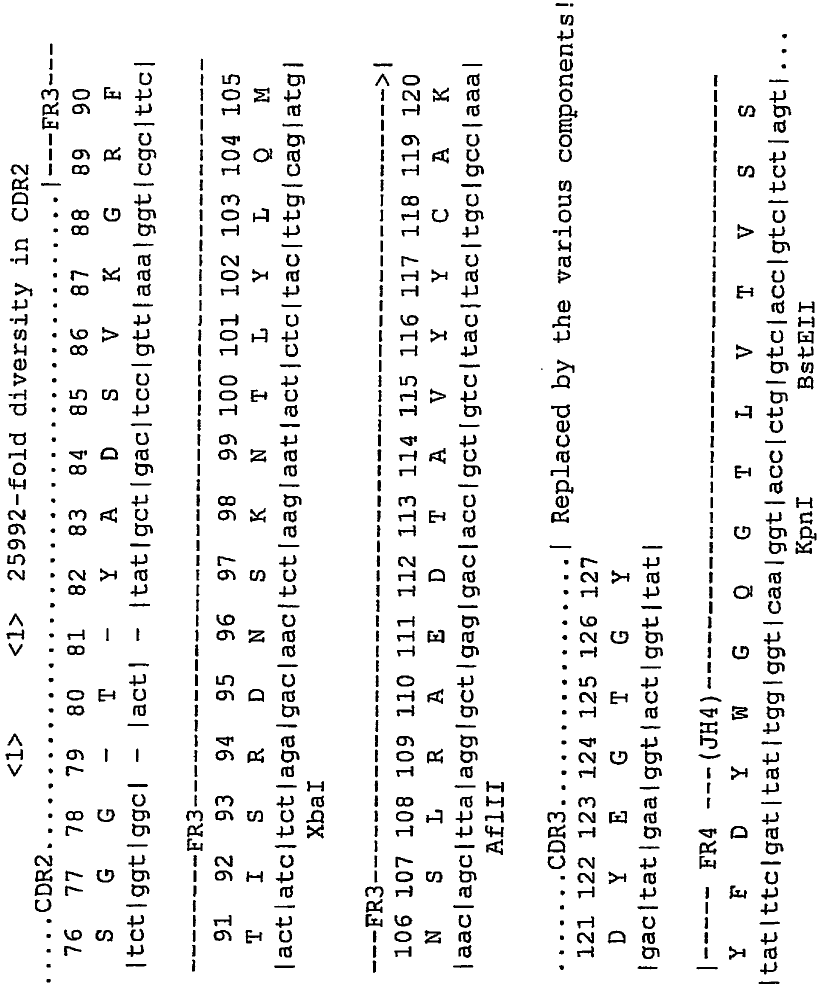

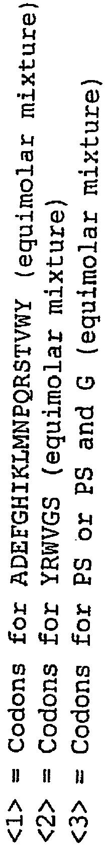

**Table 2: Oligonucleotides used to variegate CDR1 of human HC**

| **CDR1 - 5 residues** | |
|---|---|
| (ON-R1V1vg): | 5'-ct\|tcc\|gga\|ttc\|act\|ttc\|tct\|<1>\|tac\|<1>\|atg\|<1>\|tgg\|gtt\|cgc\|caa\|gct\|cct\|gg-3' |
| <1> = | Codons of ADEFGHIKLMNPQRSTVWY 1:1 |
| (ON-R1top): | 5'-cctactgtct\|tcc\|gga\|ttc\|act\|ttc\|tct-3' |
| (ON-R1bot)[RC]: | 5'-tgg\|gtt\|cgc\|caa\|gct\|cct\|ggttgctcactc-3' |

| **CDR1 - 7 residues** | |
|---|---|
| (ON-R1V2vg): | 5"-ct\|tcc\|gga\|ttc\|act\|ttc\|tct\|<6>\|<7>\|<7>\|tac\|tac\|tgg\|<7>\|tgg\|gtt\|cgc\|caa\|gct\| cct\|gg-3' |
| <6> = | Codons for ST, 1:1 |
| <7> = | 0.2025(Codons for SG) + 0.035(Codons for ADEFHIKLMNPQRTVWY) |

| **CDR1 - 14 residues** | |
|---|---|
| (ON-R1V3vg): | 5'-ct\|tcc\|gga\|ttc\|act\|ttc\|tct\|atc\|agc\|ggt\|ggt\|tct\|atc\|tcc\|<1>\|<1>\|<1>\|-tac\|tac\|tgg\|<1>\|tgg\|gtt\|cgc\|caa\|gct\|cct\|gg-3' |
| <1> = | Codons for ADEFGHIKLMNPQRSTVWY 1:1 |

**Table 3: Oligonucleotides used to variegate CDR2 of human HC**

| **CDR2 - 17 residues** | |
|---|---|
| (ON-R2V1vg): | 5'-ggt\|ttg\|gag\|tgg\|gtt\|tct\|<2>\|atc\|<2>\|<3>\|tct\|ggt\|ggc\|<1>\|act\|<1>\|tat\|gct\|-gac\|tcc\|gtt\|aaa\|gg-3' |
| (ON-R2top): | 5'-ct\|tgg\|gtt\|cgc\|caa\|gct\|cct\|ggt\|aaa\|ggt\|ttg\|gag\|tgg\|gtt\|tct-3' |
| (ON-R2bot)[RC]: | 5'-tat\|gct\|gac\|tcc\|gtt\|aaa\|ggt\|cgc\|ttc\|act\|atc\|tct\|aga\|ttcctgtcac-3' |
| <1> = | Codons for A,D,E,F,G,H,I,K,L,M,N,P,Q,R,S,T,V,W and Y (equimolar mixture) |
| <2> = | Codons for Y,R,W,V,G and S (equimolar mixture) |
| <3> = | Codons for P and S (equimolar mixture) or P,S and G (equimolar mixture) |
| (ON-R2V2vg): | 5'-ggt\|ttg\|gag\|tgg\|gtt\|tct\|<1>\|atc\|<4>\|<1>\|<1>\|ggt\|<5>\|<1>\|<1>\|<2>\|tat\|gct\|-gac\|tcc\|gtt\|aaa\|gg-3' |
| <4> = | Codons for DINSWY (equimolar mixture) |
| <5> = | Codons for SGDN, (equimolar mixture) |

| **CDR2 - 16 residues** | |
|---|---|
| (ON-R2V3vg): | 5'-ggt\|ttg\|gag\|tgg\|gtt\|tct\|<1>\|atc\|<4>\|<1>\|<1>\|ggt\| <5>\|<1>\|<1>\|tat\|aac\|cct\|tcc\|ctt\|aag\|gg-3' |
| (ON-R2bo3)[RC]: | 5'-tat\|aac\|cct\|tcc\|ctt\|aag\|ggt\|cgc\|ttc\|act\|atc\|tct\|aga\|ttcctgtcac-3' |

| **CDR2 - 19 residues** | |
|---|---|
| (ON-R2V4vg): | 5'-ggt\|ttg\|gag\|tgg\|gtt\|tct\|<1>\|atc\|<8>\|agt\|<1>\|<1>\| <1>\|ggt\|ggt\|act\|act\|<1>\|tat\|gcc\|gct\|tcc\|gtt\|aag\|gg-3' |
| (ON-R2bo4)[RC]: | 5'-tat\|gcc\|gct\|tcc\|gtt\|aag\|ggt\|cgc\|ttc\|act\|atc\|tct\|aga\|ttcctgtcac-3' <1>, <2>, <3>, <4> and <5> are as defined above |
| | <8> is 0.27 R and 0.027 each of ADEFGHIKLMNPQSTVWY |

**Table 4: Preferred Components of HC CDR3**

| Component | | Length | Complexity | Fraction of Library | Preferred Adjusted Fraction |
|---|---|---|---|---|---|
| 1 | YYCA21111YFDYWG. | 8 | 2.6 × 10⁵ | .10 | .02 |
| (1=any amino acid residue, except C; 2 = K and R) | | | | | |
| 2 | YYCA2111111YFDYWG. | 10 | 9.4 × 10⁷ | .14 | .14 |
| (1=any amino acid residue, except C; 2 = K and R) | | | | | |
| 3 | YYCA211111111YFDYWG. | 12 | 3.4 × 10¹⁰ | .25 | .25 |
| (1=any amino acid residue, except C; 2 = K and R) | | | | | |
| 4 | YYCAR111S2S3111YFDYWG. | 14 | 1.9 × 10⁸ | .13 | .14 |
| (1=any amino acid residue, except C; 2 = S and G 3 = Y and W) | | | | | |
| 5 | YYCA2111CSG11CY1YFDYWG. | 15 | 9.4 × 10⁷ | .13 | .14 |
| (1=any amino acid residue, except C; 2 = K and R) | | | | | |
| 6 | YYCA211S1TIFG11111YFDYWG. | 17 | 1.7 × 10¹⁰ | .11 | .12 |
| (1=any amino acid residue, except C; 2 = K and R) | | | | | |
| 7 | YYCAR111YY2S33YY111YFDYWG. | 18 | 3.8 × 10⁸ | .04 | .08 |
| (1=any amino acid residue, except C; 2 = D or G; 3 = S and G) | | | | | |
| 8 | YYCAR1111YC2231CY111YFDYWG. | 19 | 2.0 × 10¹¹ | .10 | .11 |
| (1=any amino acid residue, except C; 2 = S and G; 3 = T, D and G) | | | | | |

**Table 5: Oligonucleotides used to variegate the eight components of HC CDR3**

| | |
|---|---|
| (Ctop25): | 5'-gctctggtcaac\|tta\|agg\|gct\|gag\|g-3' |
| (CtprmA): | 5'-gctctggtcaac\|tta\|agg\|gct\|gag\|gac\|acc\|gct\|gtc\|tac\|tac\|tgc\|gcc-3' AfIII... |
| (CBprmB)[RC]: | 5'-\|tac\|ttc\|gat\|tac\|tgg\|ggc\|caa\|ggt\|acc\|ctg\|gtc\|acc\|tcgctccacc-3' BstEII... |
| (CBot25)[RC]: | 5'-\|ggt\|acc\|ctg\|gtc\|acc\|tcgctccacc-3' |
| The 20 bases at 3' end of CtprmA are identical to the most 5' 20 bases of each of the vgDNA molecules. | |
| Ctop25 is identical to the most 5' 25 bases of CtprmA. | |
| The 23 most 3' bases of CBprmB are the reverse complement of the most 3' 23 bases of each of the vgDNA molecules. | |
| CBot25 is identical to the 25 bases at the 5' end of CBprmB. | |
| | |

| **Component 1** | |
|---|---|
| (C1t08): | 5'-cc\|gct\|gtc\|tac\|tac\|tgc\|gcc\|<2>\|<1>\|<1>\|<1>\|<1>\|tac\|ttc\|gat\|tac\|tgg\|ggc\|caa\|gg-3' |
| <1> = 0.095 (equimolar Y + 0.095 G + 0.048 each of the residues ADEFHIKLMNPQRSTVW, no C; <2> = K and R mixture) | |

| **Component 2** | |
|---|---|
| (C2t10): | 5'-cc\|gct\|gtc\|tac\|tac\|tgc\|gcc\|<2>\|<1>\|<1>\|<1>\|<1>\|<1>\|<1>\|tac\|ttc\|gat\|tac\|tgg\|ggc\|caa\|gg-3' |
| <1> = 0.095 Y + 0.095 G + 0.048 each of ADEFHIKLMNPQRSTVW, no C; <2> = K and R (equimolar mixture) | |
| | |

| **Component 3** | |
|---|---|
| (C3t12) : | 5'-cc\|gct\|gtc\|tac\|tac\|tgc\|gcc\|<2>\|<1>\|<1>\|<1>\|<1>\|<1>\|<1>\|<1>\|<1>\|tac\|ttc\|gat\|tac\|-tgg\|ggc\|caa\|gg-3' |
| <1> = 0.095 Y + 0.095 G + 0.048 each of ADEFHIKLMNPQRSTVW, no C; <2> = K and R (equimolar mixture) | |

| **Component 4** | |
|---|---|
| (C4t140): | 5'-cc\|gct\|gtc\|tac\|tac\|tgc\|gcc\|cgt\|<1>\|<1>\|<1>\|tct\|<2>\|tct\|<3>\|<1>\|<1>\|<1>\|tac\|ttc\|gat\|-tac\|tgg\|ggc\|caa\|gg-3' |
| <1> = | 0.095 Y + 0.095 G + 0.048 each of ADEFHIKLMNPQRSTVW, no C; <2> = S and G (equimolar mixture); <3> = Y and W (equimolar mixture) |
| | |

| **Component 5** | |
|---|---|
| (C5t15) : | 5'-cc\|gct\|gtc\|tac\|tac\|tgc\|gcc\|<2>\|<1>\|<1>\|<1>\|tgc\|tct\|ggt\|<1>\|<1>\|tgc\|tat\|<1>\|tac\|-ttc\|gat\|tac\|tgg\|ggc\|caa\|gg-3' |
| <1> = | 0.095 Y + 0.095 G + 0.048 each of ADEFHIKLMNPQRSTVW, no C; <2> = K and R (equimolar mixture) |

| **Component 6** | |
|---|---|
| (C6t17): | |
| | 5'-cc\|gct\|gtc\|tac\|tac\|tgc\|gcc\|<2>\|<1>\|<1>\|tct\|<1>\|act\|atc\|ttc\|ggt\|<1>\|<1>\|<1>\|<1>\|-<1>\|tac\|ttc\|gat\|tac\|tgg\|ggc\|caa\|gg-3' |
| <1> = 0.095 Y + 0.095 G + 0.048 each of ADEFHIKLMNPQRSTVW, no C; <2> = K and R (equimolar mixture) | |
| | |

| **Component 7** | |
|---|---|
| (C7t18) : | |
| | 5'-cc\|gct\|gtc\|tac\|tac\|tgc\|gcc\|cgt\|<1>\|<1>\|<1>\|tat\|tac\|<2>\|tct\|<3>\|<3>\|tac\|tat\|-<1>\|<1>\|<1>\|tac\|ttc\|gat\|tac\|tgg\|ggc\|caa\|gg-3' |
| <1> = 0.095 Y + 0.095 G + 0.048 each of ADEFIKLMNPQRSTVW, no C; <2> = D and G (equimolar mixture); <3> = S and G (equimolar mixture) | |

| **Component 8** | |
|---|---|
| (c8t19) : | S'-cc\|gct\|gtc\|tac\|tac\|tgc\|gcc\|cgt\|<1>\|<1>\|<1>\|<1>\|tat\|tgc\|<2>\|<2>\|<3>\|<1>\|tgc\|tat\|-<1>\|<1>\|<1>\|tac\|ttc\|gat\|tac\|tgg\|ggc\|caa\|gg-3^{'} |
| <1> = 0.095 Y + 0.095 G + 0.048 each of ADEFHIKLMNPQRSTVW, no C; <2> = S and G (equimolar mixture); <3> = TDG (equimolar mixture); | |

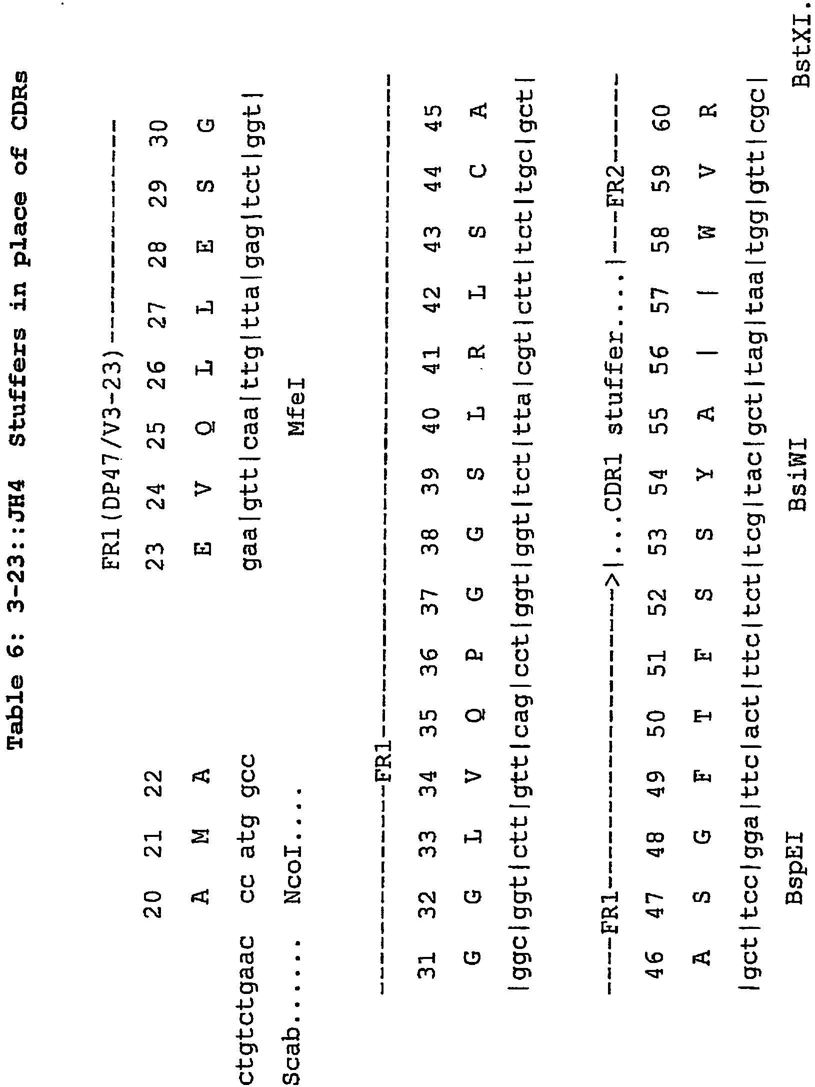

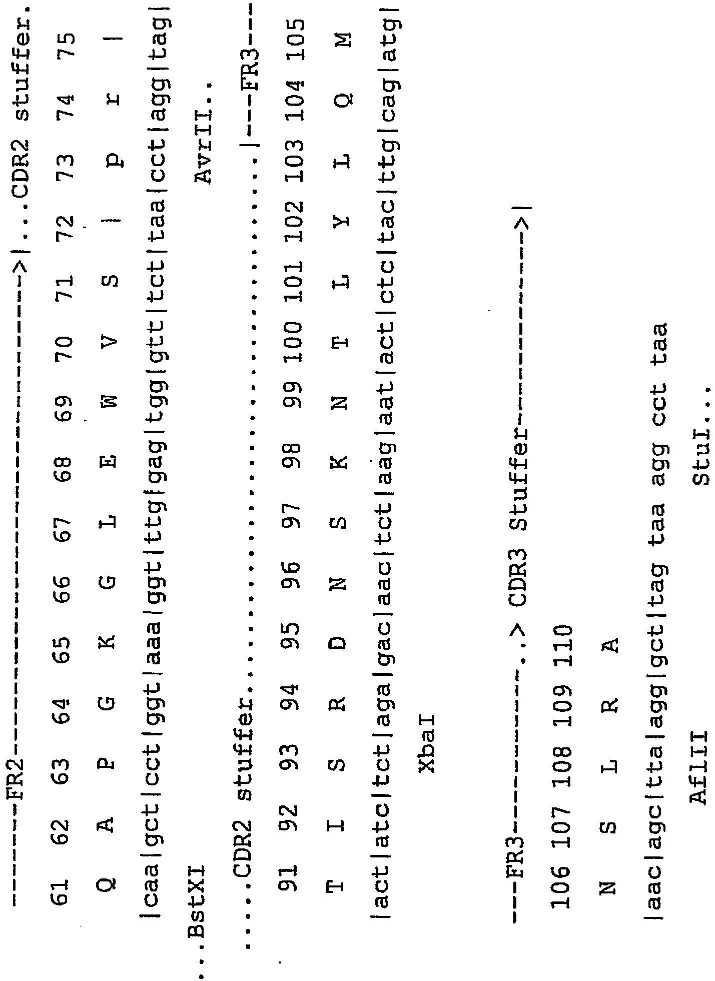

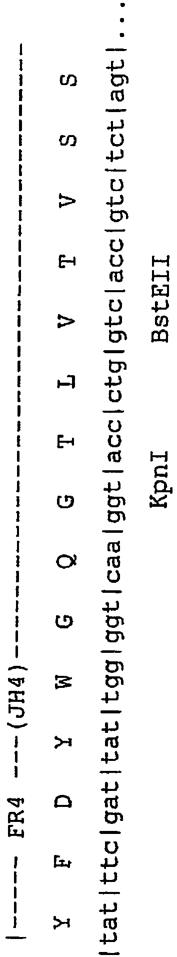

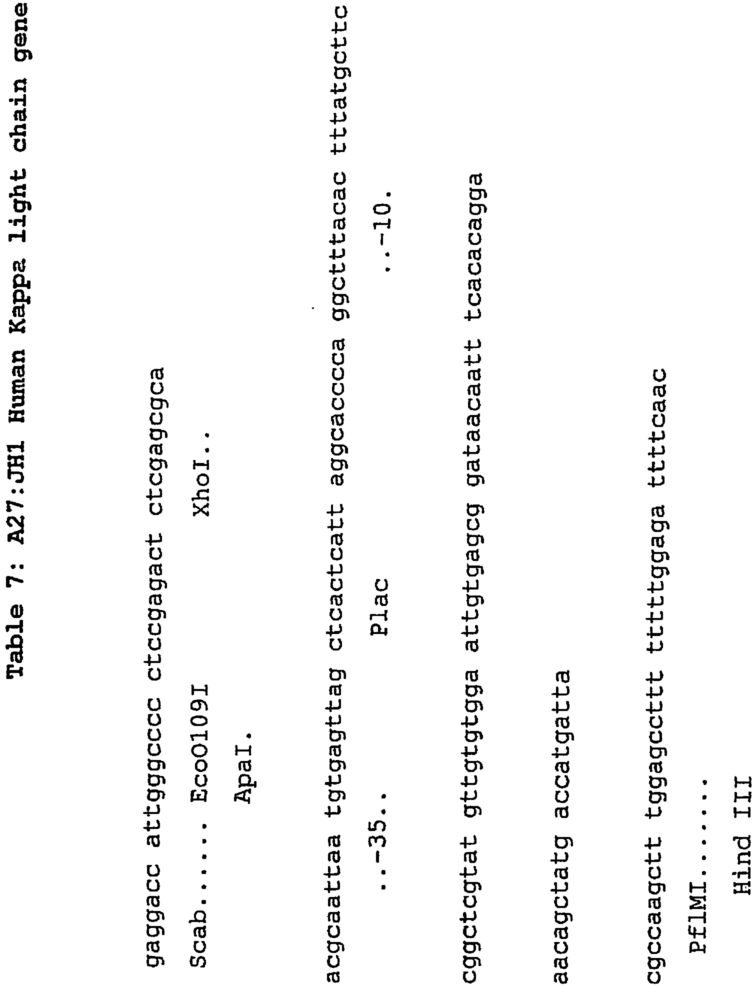

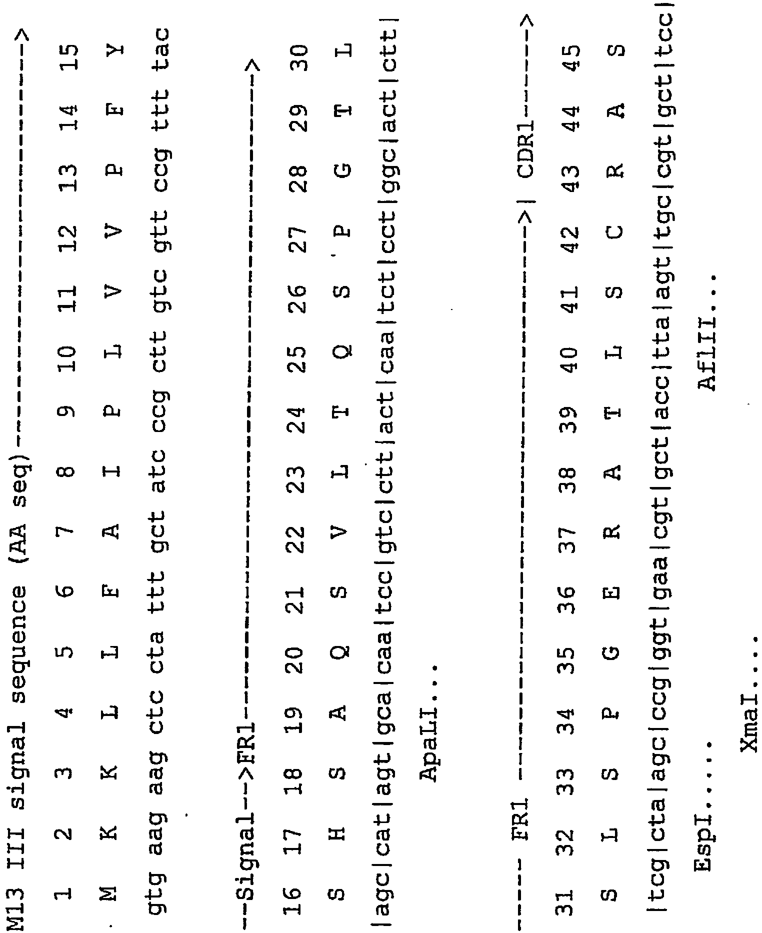

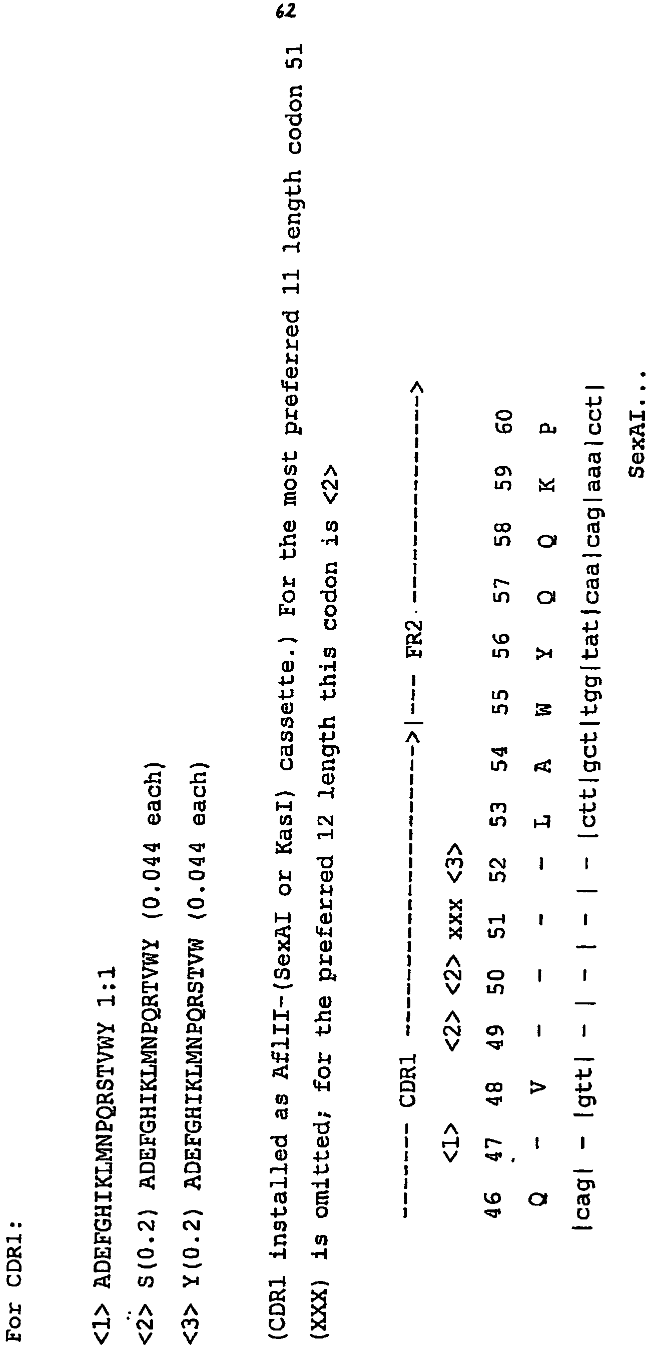

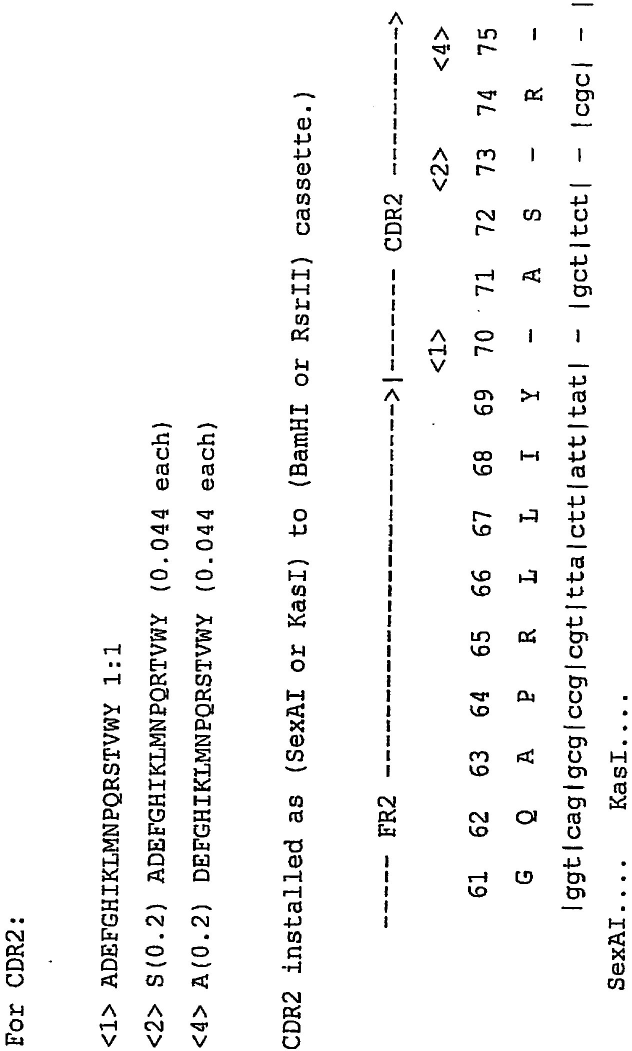

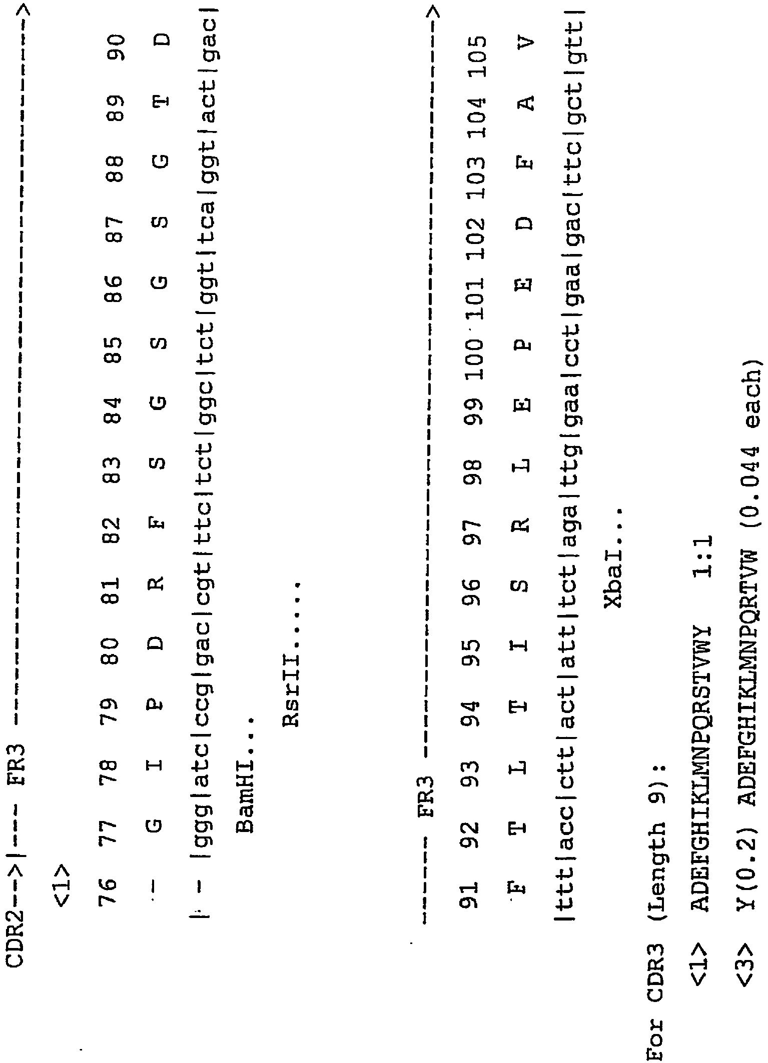

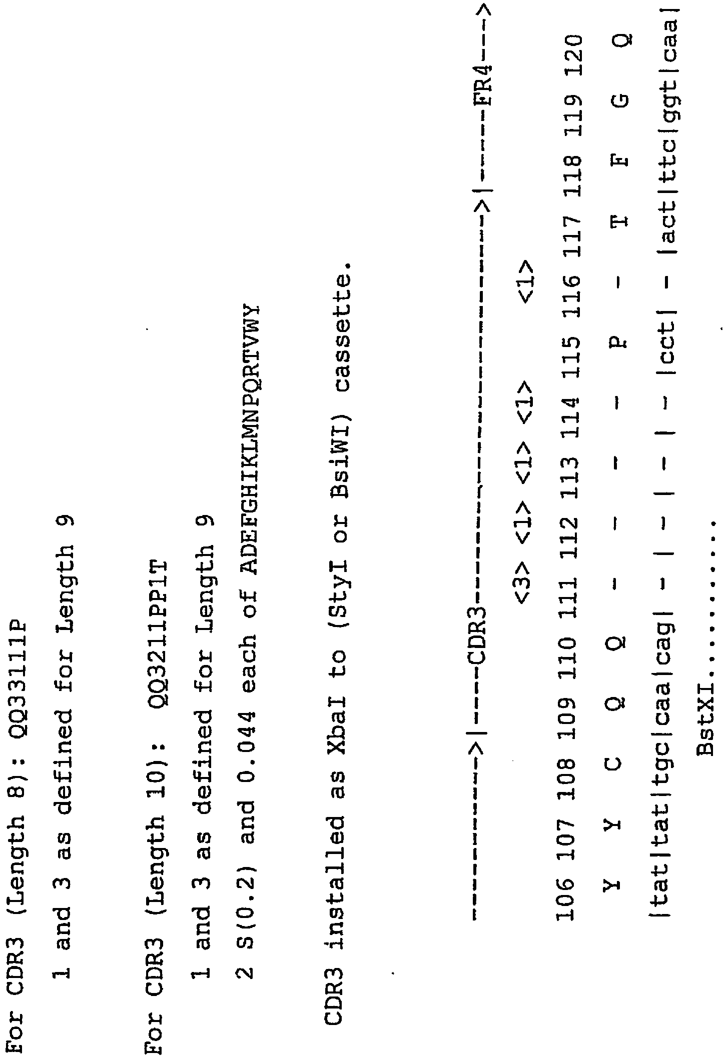

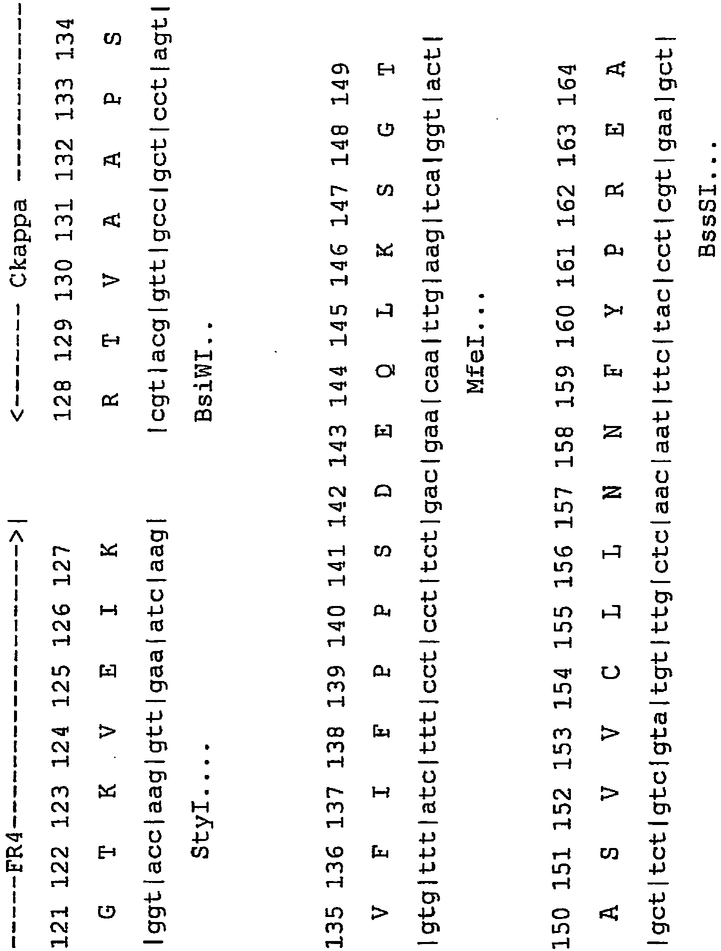

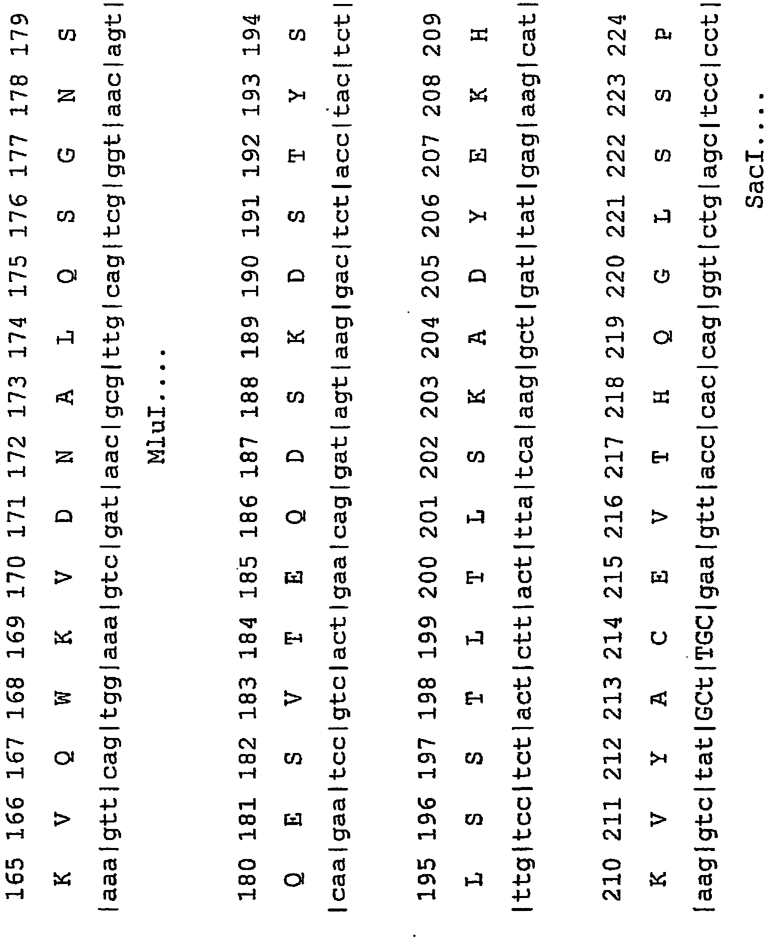

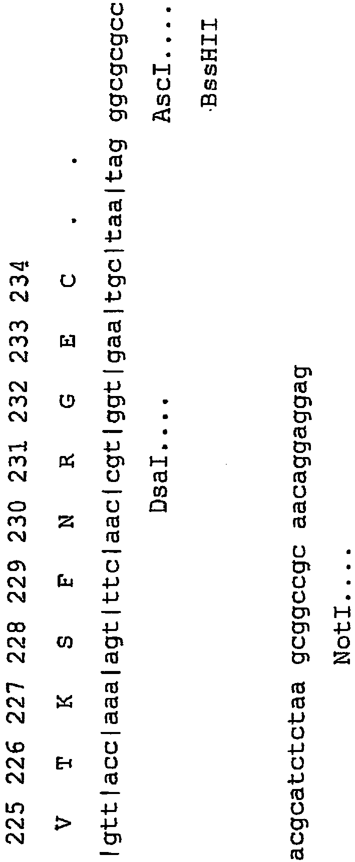

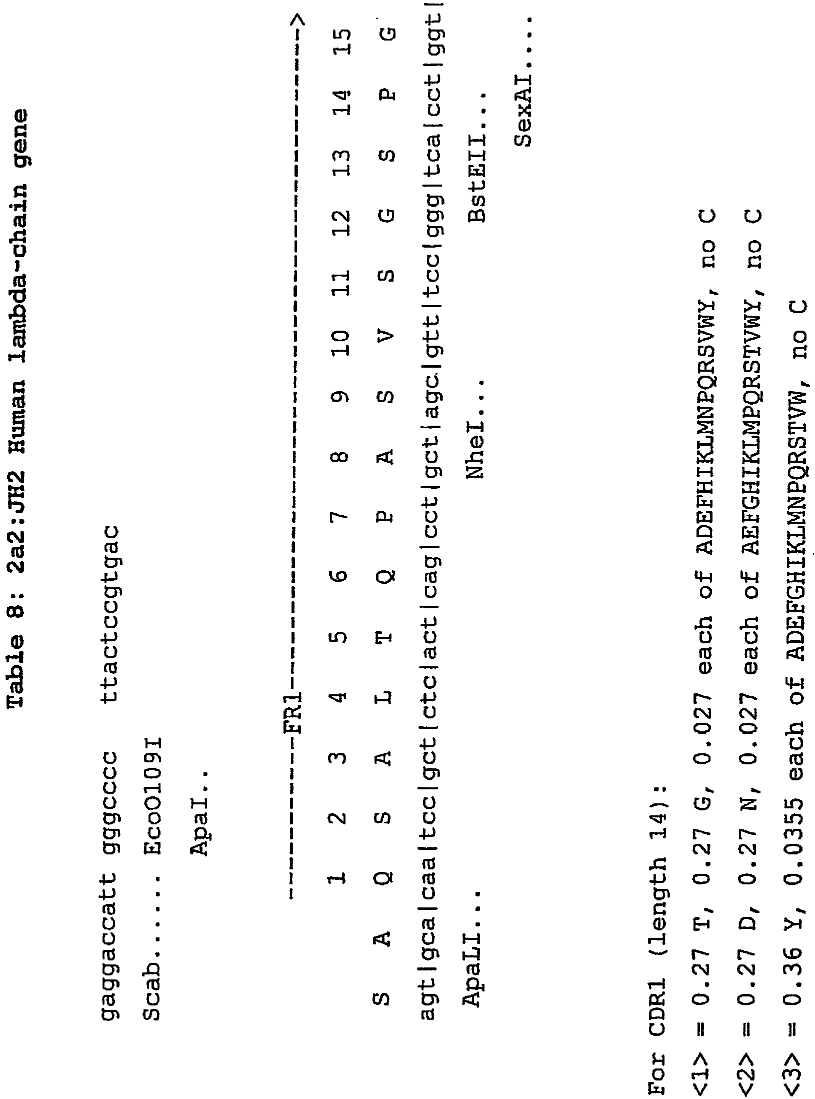

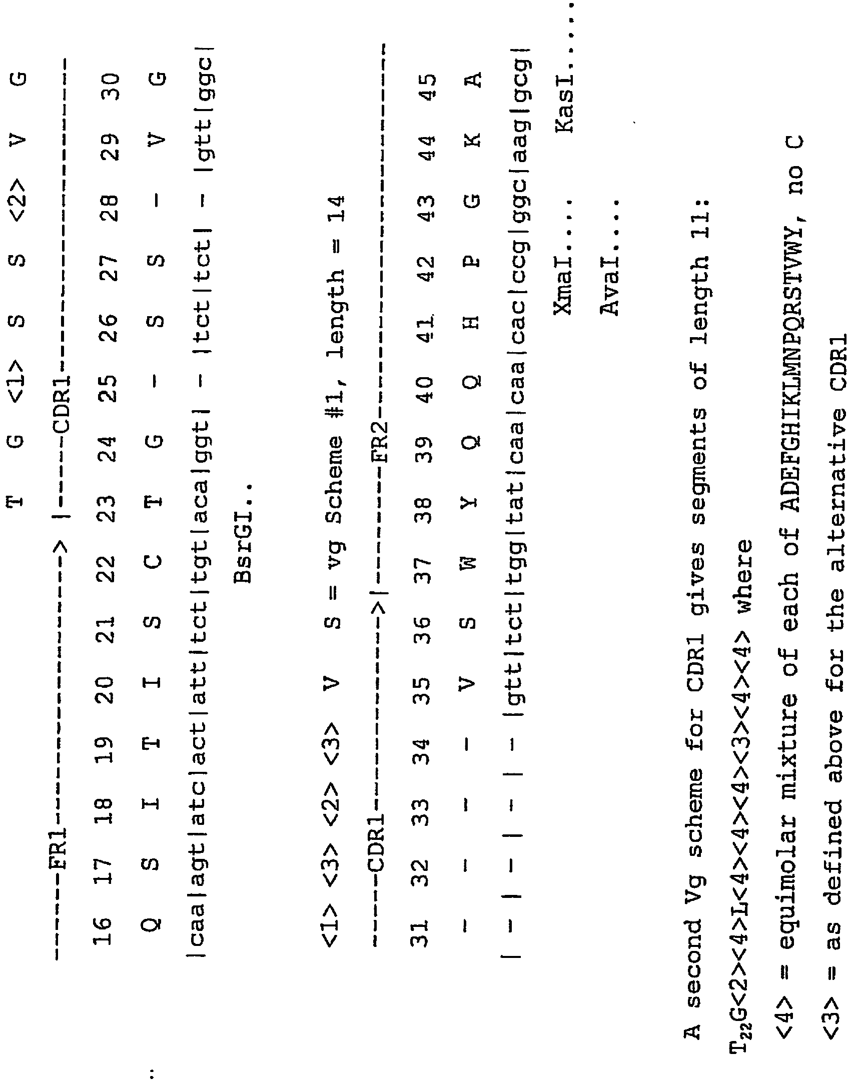

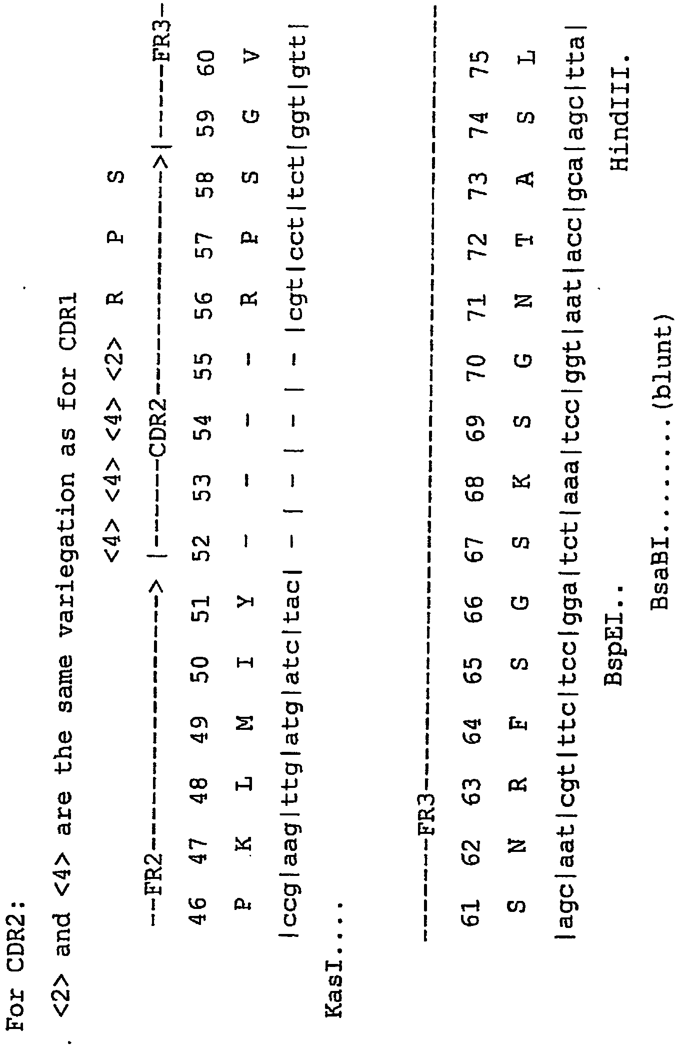

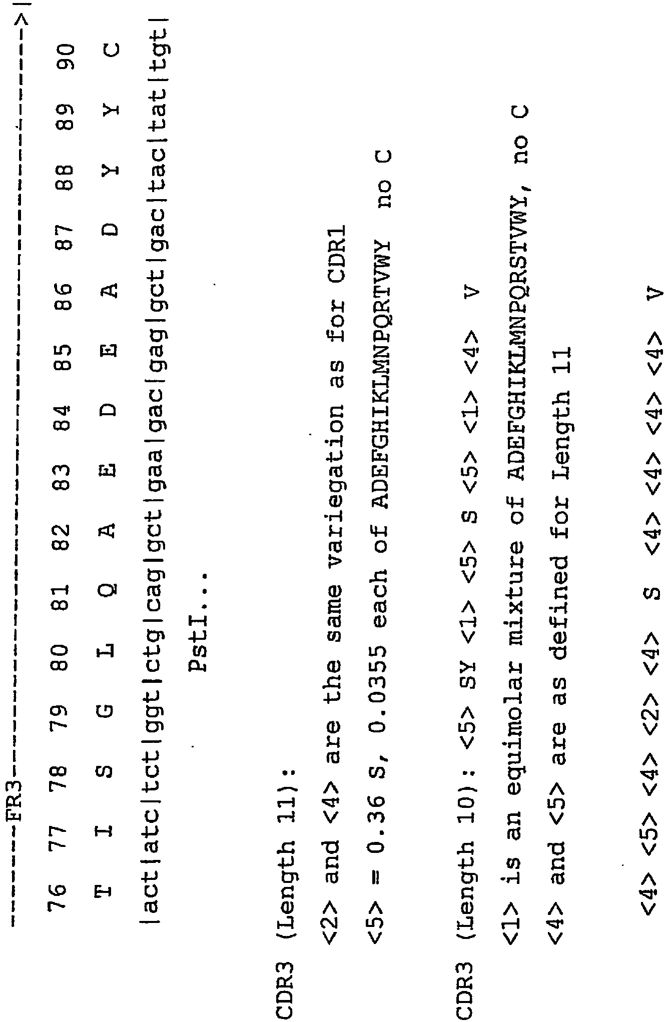

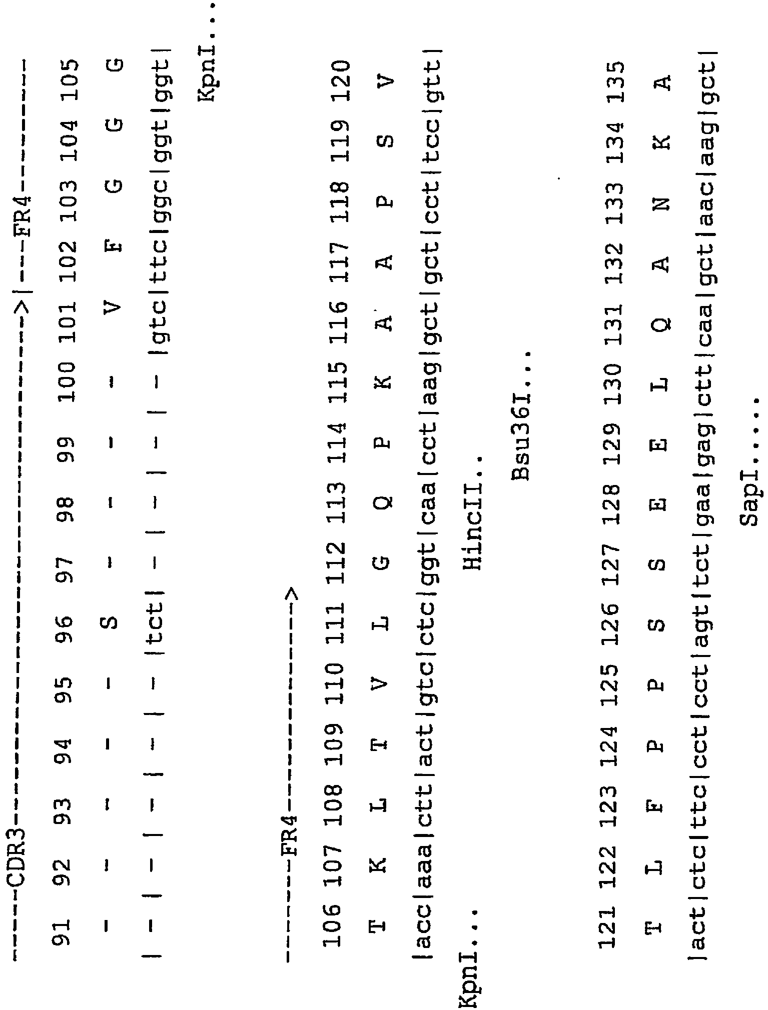

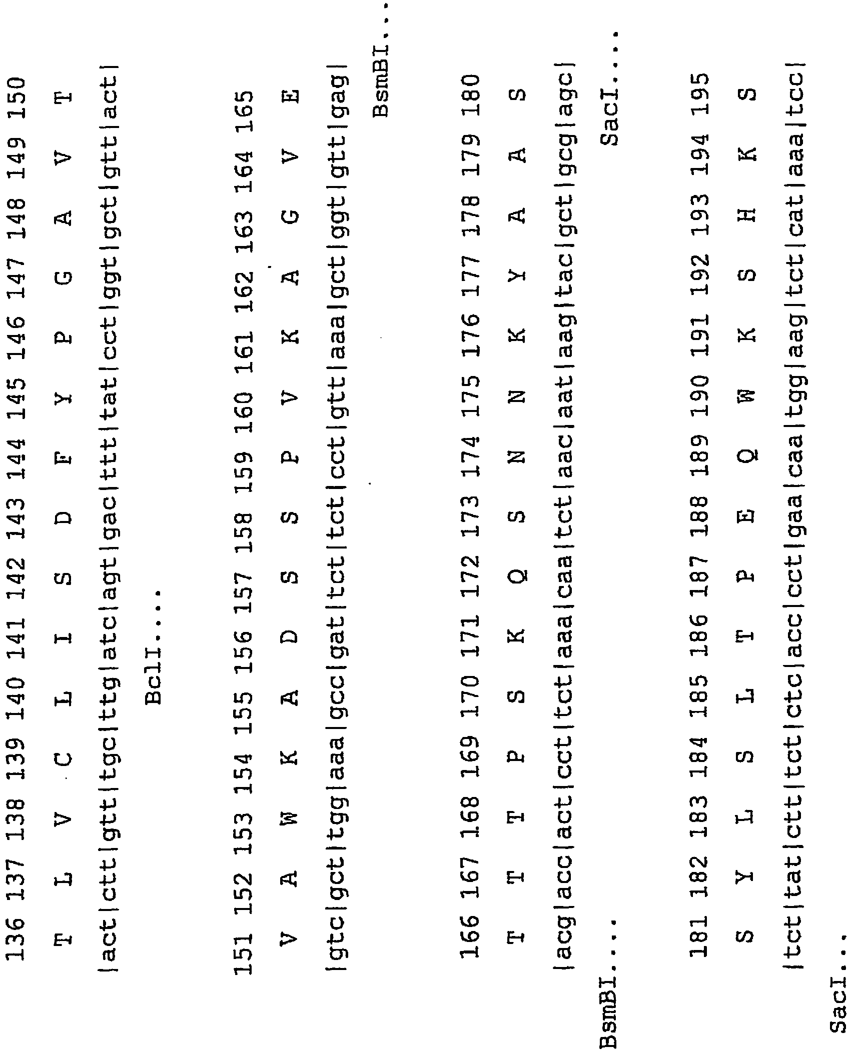

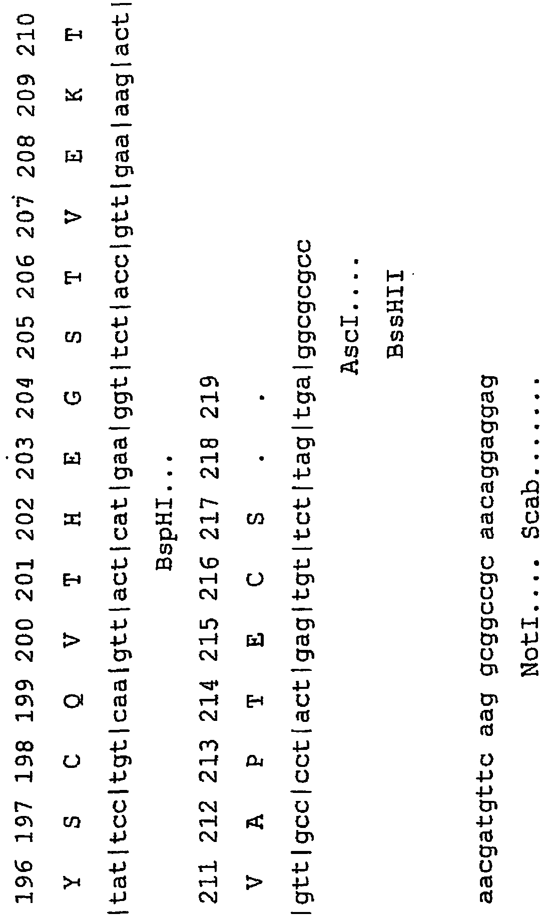

**Table 9: Oligonucleotides For Kappa and Lambda Light Chain Variegation**

| | |
|---|---|
| (Ctop25): | 5'-gctctggtcaac\|tta\|agg\|gct\|gag\|g-3' |
| (CtprmA): | 5'-gctctggtcaac\|tta\|agg\|gct\|gag\|gac\|acc\|gct\|gtc\|tac\|tac\|tgc\|gcc-3' AflII... |
| (CBprmB)[RC]: | 5'-\|tac\|ttc\|gat\|tac\|ttg\|ggc\|caa\|ggt\|acc\|ctg\|gtc\|acc\|tcgctccacc-3' BstEII... |
| (CBot25)[RC]: | 5'-\|ggt\|acc\|ctg\|gtc\|acc\|tcgctccacc-3' |

| **Kappa chains.: CDR1 ("1"), CDR2 ("2"), CDR3 ("3")** | |
|---|---|
| **CDR1** | |
| (Ka1Top610): | 5'-ggtctcagttg\|cta\|agc\|ccg\|ggt\|gaa\|cgt\|gct\|acc\|tta\|agt\|tgc\|cgt\|gct\|tcc\|cag-3' |
| (Ka1STp615): | 5'-ggtctcagttg\|cta\|agc\|ccg\|ggt\|g-3' |
| (Ka1Bot620) [RC]: | 5'-ctt\|gct\|tgg\|tat\|caa\|cag\|aaa\|cct\|ggt\|cag\|gcg\|ccaagtcgtgtc-3' |
| (Ka1SB625) [RC]: | 5'-cct\|ggt\|cag\|gcg\|ccaagtcgtgtc-3' |

| | |
|---|---|
| (Ka1vg600): | 5'-gct\|acc\|tta\|agt\|tgc\|cgt\|gct\|tcc\|cag-\|<1>\|gtt\|<2>\|<2>\|<3>\|ctt\|gct\|tgg\|tat\|caa\|cag\|aaa\|cc-3' |
| (Ka1vg600-7.2): | 5'-gct\|acc\|tta\|agt\|tgc\|cgt\|gct\|tcc\|cag-\|<1>\|gtt\|<2>\|<2>\|<2>\|<3>\|ctt\|gct\|tgg\|tat\|caa\|cag\|aaa\|cc-3' |

| **CDR2** | |
|---|---|
| (Ka2Tshort657): | 5'-cacgagtccta\|cct\|ggt\|cag\|gc-3' |
| (Ka2Tlong655): | 5'-cacgagtccta\|cct\|ggt\|cag\|gcg\|ccg\|cgt\|tta\|ctt\|att\|tat-3' |
| (Ka2Bshort660) : [RC] : | 5'-\|gac\|cgt\|ttc\|tct\|ggt\|tctcacc-3' |
| (Ka2vg650): | 5'-cag\|gcg\|ccg\|cgt\|tta\|ctt\|att\|tat\|<1>\|gct\|tct\|<2>\|-\|cgc\|<4>\|<1>\|ggg\|atc\|ccg\|gac\|cgt\|ttc\|tct\|ggt\|tctcacc-3' |

| **CDR3** | |
|---|---|
| (Ka3Tlon672): | 5'-gacgagtccttct\|aga\|ttg\|gaa\|cct\|gaa\|gac\|ttc\|gct\|gtt\|tat\|tat\|tgc\|caa\|c-3' |
| (Ka3BotL682) [RC]: | 5'-act\|ttc\|ggt\|caa\|ggt\|acc\|aag\|gtt\|gaa\|atc\|aag\|cgt\|acg\|tcacaggtgag-3' |
| (Ka3Bsho694) [RC]: | 5'-gaa\|atc\|aag\|cgt\|acg\|tcacaggtgag-3' |

| | |
|---|---|
| (Ka3vg670): | 5'-gac\|ttc\|gct\|gtt\|-\|tat\|tat\|tgc\|caa\|cag\|<3>\|<1>\|<1>\|<1>\|cct\|<1>\|act\|ttc\|ggt\|caa\|-\|ggt\|acc\|aag\|gtt\|g-3' |
| (Ka3vg670-8): | 5'-gac\|ttc\|gct\|gtt\|-\|tat\|tat\|tgc\|caa\|cag\|<3>\|<3>\|<1>\|<1>\|<1>\|cct\|ttc\|ggt\|caa\|-\|ggt\|acc\|aag\|gtt\|g-3' |
| (Ka3vg670-10): | 5'-gac\|ttc\|gct\|gtt\|tat\|-\|tat\|tgc\|caa\|cag\|<3>\|<2>\|<1>\|<1>\|cct\|cct\|<1>\|act\|ttc\|ggt\|caa\|-\|ggt\|acc\|aag\|gtt\|g-3' |
| | |

| **Lambda Chains: CDR1 ("1"), CDR2 ("2"), CDR3 ("3")** | |
|---|---|
| | |

| **CDR1** | |
|---|---|
| (Lm1TPri75): | 5'-gacgagtcctgg\|tca\|cct\|ggt\|-3' |
| (Lm1tlo715): | 5'-gacgagtcctgg\|tca\|cct\|ggt\|caa\|agt\|atc\|act\|att\|tct\|tgt\|aca\|ggt-3' |
| (Lm1blo724) [rc]: | 5'-gtt\|tct\|tgg\|tat\|caa\|caa\|cac\|ccg\|ggc\|aag\|gcg\|agatcttcacaggtgag-3' |
| (Lmlbsh737) [rc]: | 5'-gc\|aag\|gcg\|agatcttcacaggtgag-3' |
| | (Lm1vg710b):5'-gt\|atc\|act\|att\|tct\|tgt\|aca\|ggt\|<2>\|<4>\|ctc\|<4>\|<4>\|<4>\|-\|<3>\|<4>\|<4>\|tgg\|tat\|caa\|caa\|cac\|cc-3' |

| | |
|---|---|
| (Lm1vg710): | 5'-gt\|atc\|act\|att\|tct\|tgt\|aca\|ggt\|<1>\|tct\|tct\|<2>\|gtt\|ggc\|-\|<1>\|<3>\|<2>\|<3>\|gtt\|tct\|tgg\|tat\|caa\|caa\|cac\|cc-3' |
| | |

| **CDR2** | |
|---|---|
| (Lm2TSh757): | 5'-gagcagaggac\|ccg\|ggc\|aag\|gc-3' |
| (Lm2TLo753): | 5'-gagcagaggac\|ccg\|ggc\|aag\|gcg\|ccg\|aag\|ttg\|atg\|atc\|tac\|-3' |
| (Lm2BLo762) [RC]: | 5'-cgt\|cct\|tct\|ggt\|gtc\|agc\|aat\|cgt\|ttc\|tcc\|gga\|tcacaggtgag-3' |
| (Lm2BSh765) [RC]: | 5'-cgt\|ttc\|tcc\|gga\|tcacaggtgag-3' |
| (Lm2vg750): | 5'-g\|ccg\|aag\|ttg\|atg\|atc\|tac\|-<4>\|<4>\|<4>\|<2>\|cgt\|cct\|tct\|ggt\|gtc\|agc\|aat\|c-3' |
| | |

| **CDR3** | |
|---|---|
| (Lm3TSh822): | 5'-ctg\|cag\|gct\|gaa\|gac\|gag\|gct\|gac-3' |
| (Lm3TLo819): | 5'-ctg\|cag\|gct\|gaa\|gac\|gag\|gct\|gac\|tac\|tat\|tgt\|-3' |
| (Lm3BLo825) [RC]: | 5'-gtc\|ttc\|ggc\|ggt\|ggt\|acc\|aaa\|ctt\|act\|gtc\|ctc\|ggt\|caa\|cct\|aag\|g-acacaggtgag-3' |
| (Lm3BSh832) [RC]: | 5'-c\|ggt\|caa\|cct\|aag\|gacacaggtgag-3' |

| | |
|---|---|
| (Lm3vg817): | 5'-gac\|gag\|gct\|gac\|tac\|tat\|tgt\|-\|<4>\|<5>\|<4>\|<2>\|<4>\|tct\|<4>\|<4>\|<4>\|<4>\|-Gtc\|ttc\|ggc\|ggt\|ggt\|acc\|aaa\|ctt\|ac-3' |
| (Lm3vg817-10): | 5'- gac\|gag\|gct\|gac\|tac\|tat\|tgt\|-\|<5>\|agc\|tat\|<1>\|<5>\|tct\|<5>\|<1>\|<4>\|gtc\|ttc\|ggc\|ggt\|ggt\|-\|acc\|aaa\|ctt\|ac-3' |

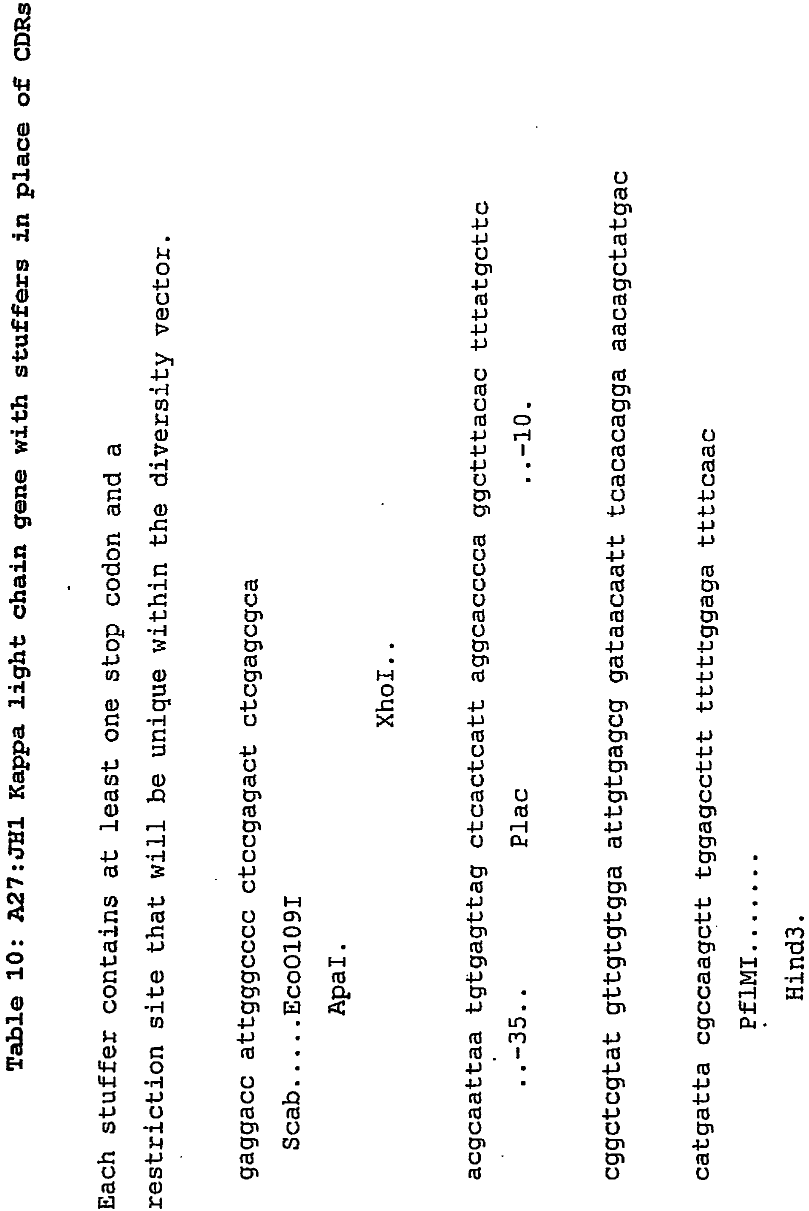

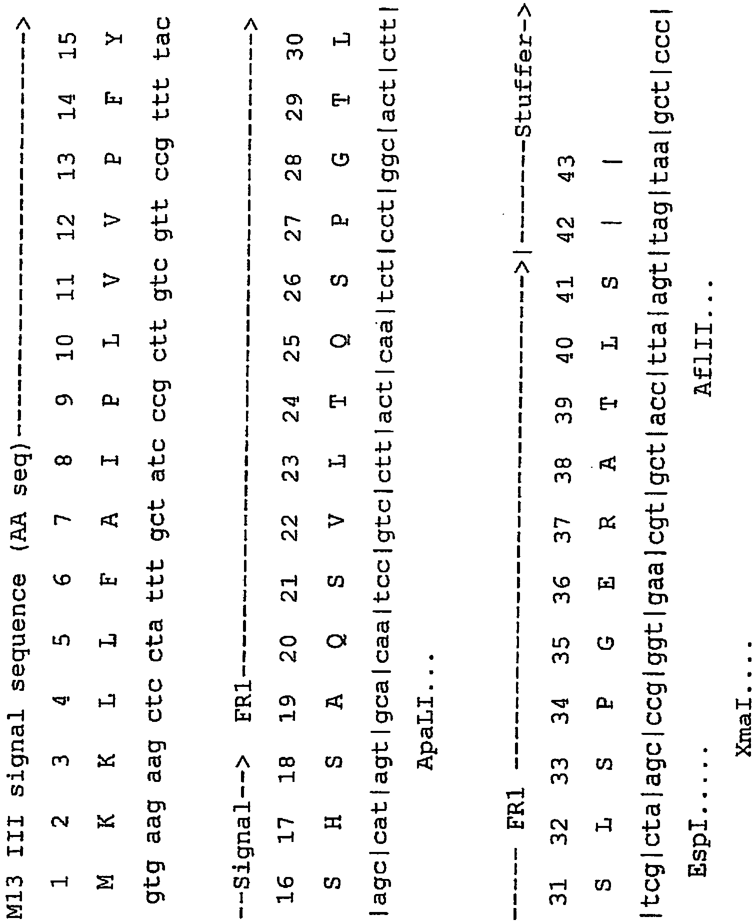

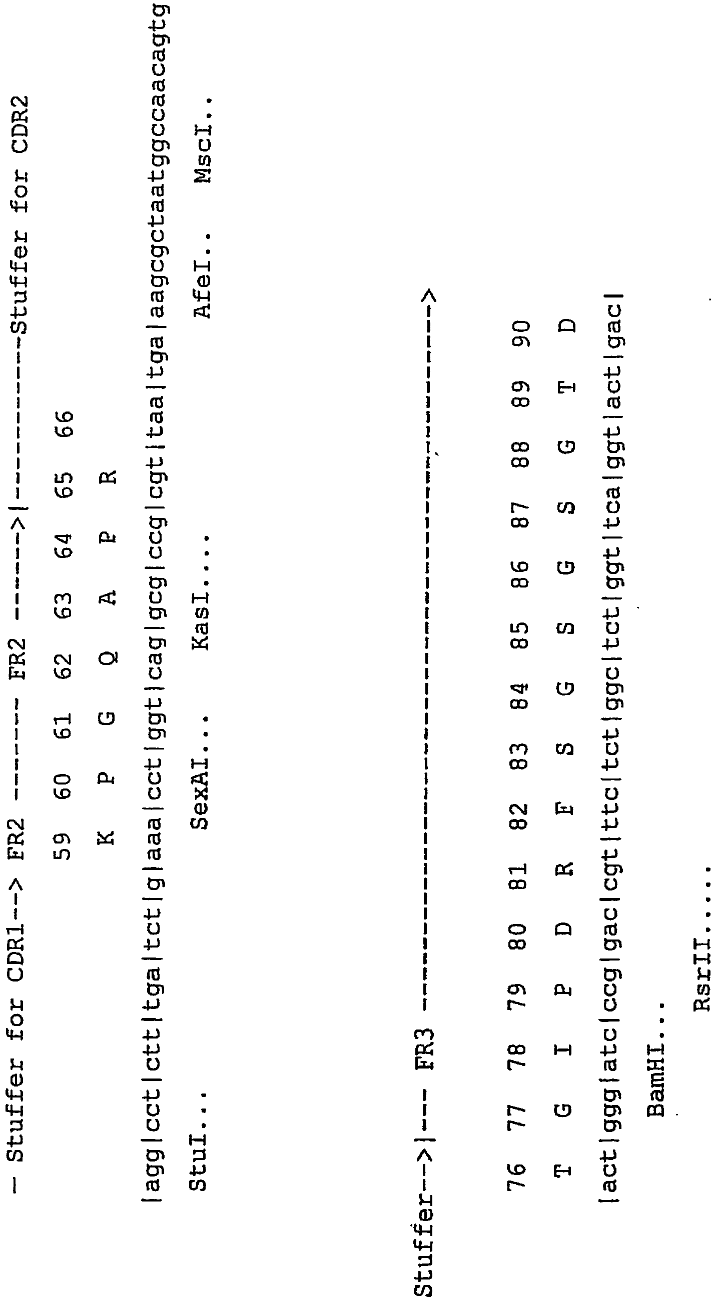

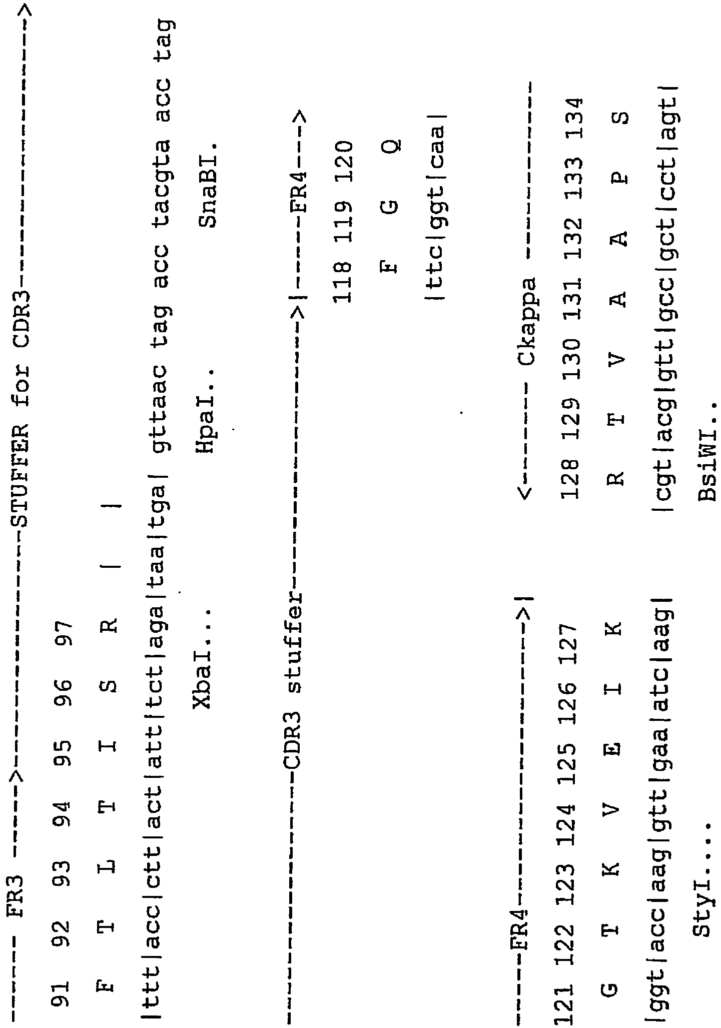

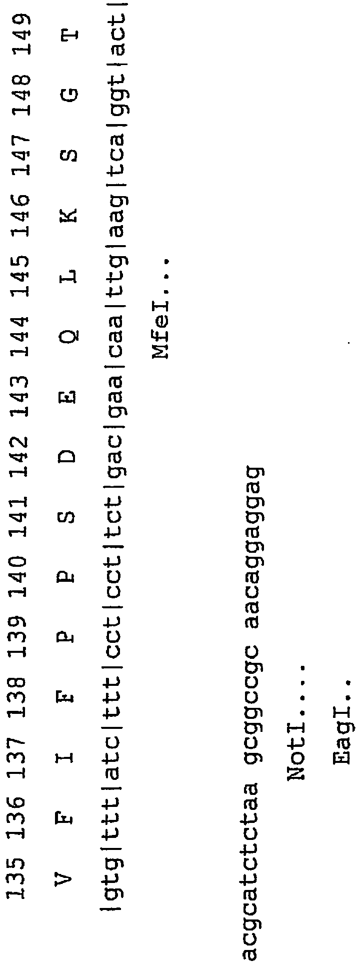

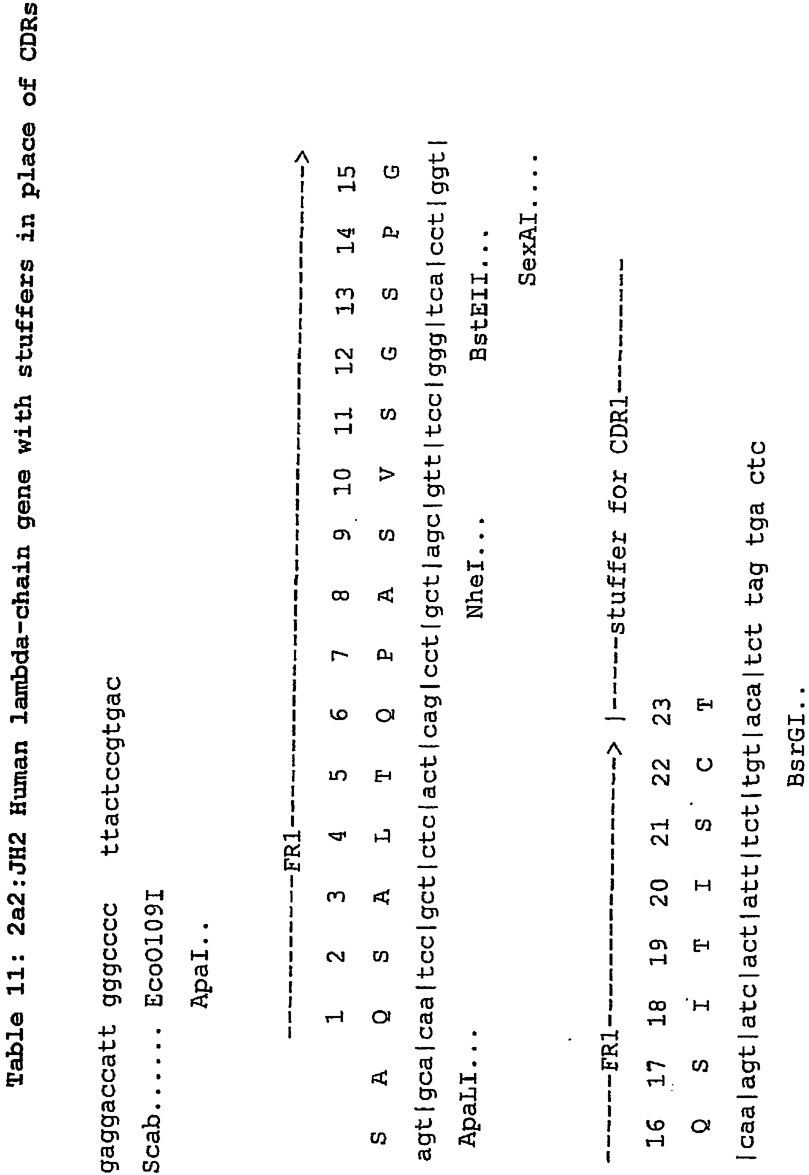

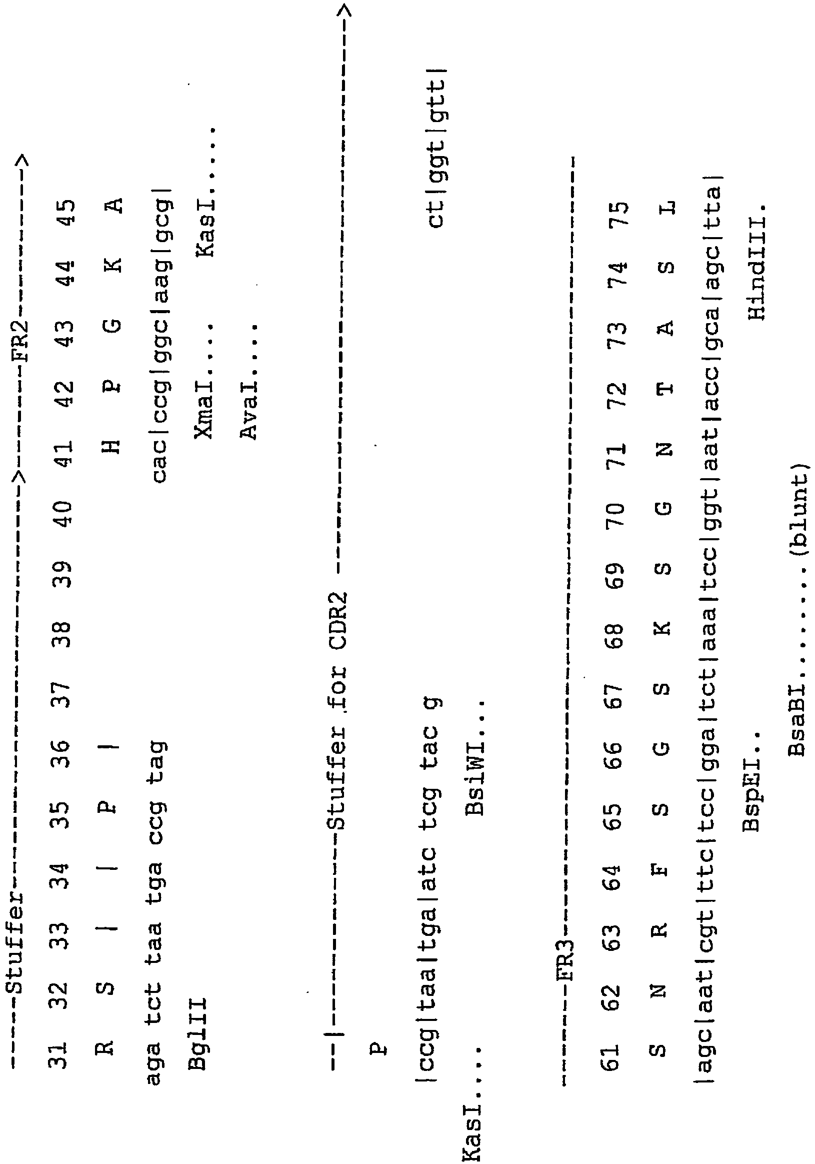

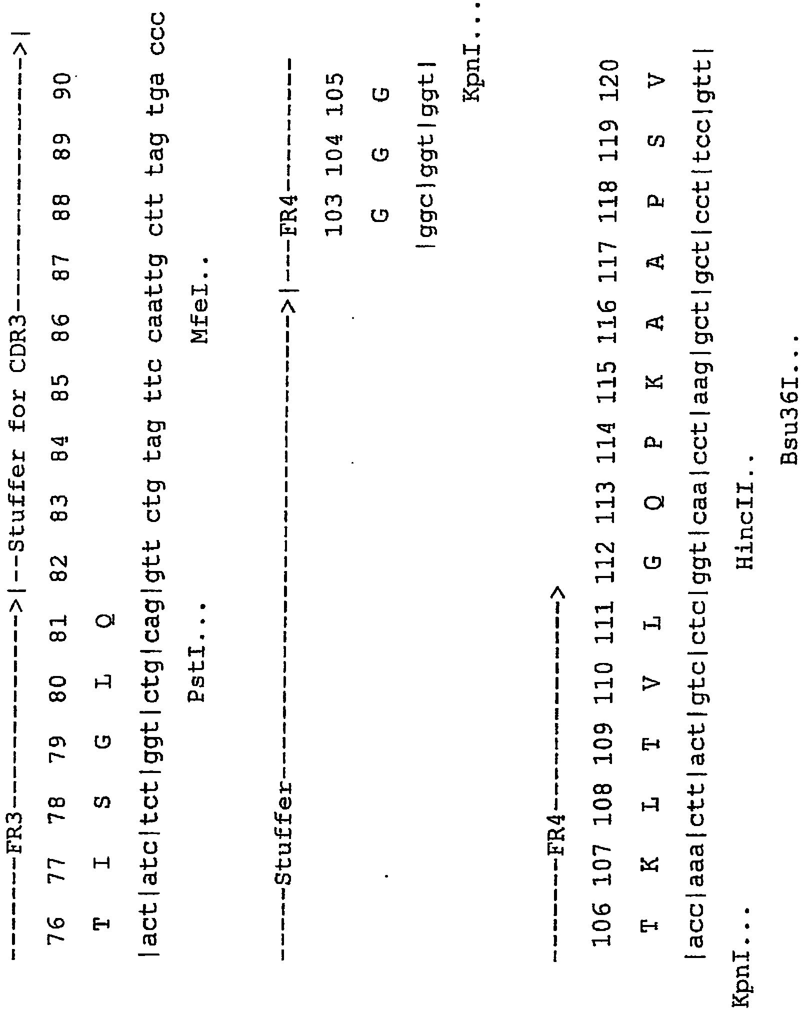

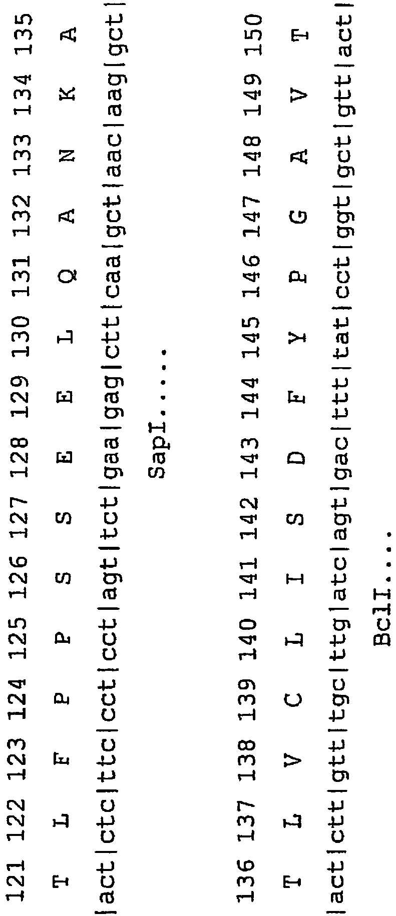

## Claims

1. A focused DNA library comprising variegated DNA sequences of antibodies that encode collectively:
(1) kappa light chain CDR1 regions selected from the group consisting of:
(a) RASQ<1>V<2><2><3>LA
(b) RASQ<1>V<2><2><2><3>LA;
wherein <1> is an equimolar mixture of amino acid residues ADEFGHIKLMNPQRSTVWY; <2> is 0.2 S and 0.044 of each of ADEFGHIKLMNPQRTVWY; and <3> is 0.2 Y and 0.044 each of ADEFGHIKLMNPQRSTVW; and
(c) a mixture of (a) and (b) set forth above;
the members of this group being the only kappa light chain CDR1s present in the library;
(2) kappa light chain CDR2 regions having the sequence:
<1>AS<2>R<4><1>,
wherein <1> is an equimolar mixture of amino acid residues ADEFGHIKLMNPQRSTVWY; <2> is 0.2 S and 0.044 of each of ADEFGHIKLMNPQRTWVY; and <4> is 0.2 A and 0.044 each of DEFGHIKLMNPQRSTVWY;
the members of this group being the only kappa light chain CDR2s present in the library; and
(3) kappa light chain CDR3 regions selected from the groups consisting of:
(a) QQ<3><1><1><1>P<1>T,
wherein <1> is an equimolar mixture of amino acid residues ADEFGHIKLMNPQRSTVWY; <3> is 0.2 Y and 0.044 each of ADEFGHIKLMNPQRTVW;
(b) QQ33111P,
wherein 1 and 3 are as defined in (1) above;
(c) QQ3211PP1T,
wherein 1 and 3 are as defined in (1) above and 2 is 0.2 S and 0.044 each of ADEFGHIKLMNPQRTVWY; and
(d) a mixture of any of (a) to (c) set forth above;
the members of this group being the only kappa light chain CDR3s present in the library.

2. The focused DNA library according to claim 1, wherein CDRIs (a) and (b) are present in the library in a ratio of 0.68:0.32.

3. The focused DNA library according to claim 1, wherein CDR3s (a), (b) and (c) are present in the library in a ratio of 0.65: 0.1: 0.25.

4. The focused DNA library of any of claims 1-3, further comprising variegated DNA sequences that encode collectively
(1) lambda light chain CDR1 regions selected from the group consisting of:
(a) TG<1>SS<2>VG<1><3><2><3>VS,
wherein <1> is 0.27 T, 0.27 G and 0.027 each of ADEFHIKLMNPQRSVWY, <2> is 0.27 D, 0.27 N and 0.027 each of AEFGHIKLMPQRSTVWY, and <3> is 0.36 Y and 0.036 each of ADEFGHIKLMNPQRSTVW;
(b) G<2><4>L<4><4><4><3><4><4>, wherein <2> is as defined in (1) above and <4> is an equimolar mixture of amino acid residues ADEFGHIKLMNPQRSTVWY; and
(c) a mixture of (a) and (b) set forth above;
the members of this group being the only lambda light chain CDR1s present in the library;
(2) lambda light chain CDR2 regions having the sequence:
<4><4><4><2>RPS,
wherein <2> is 0.27 D, 0.27 N, and 0.027 each of AEFGHIKLMPQRSTVWY and <4> is an equimolar mixture of amino acid residues ADEFGHIKLMNPQRSTVW; the members of this group being the only lambda light chain CDR2s present in the library; and
(3) lambda light chain CDR3 regions selected from the group consisting of:
(a) <4><5><4><2><4>S<4><4><4><4>V,
wherein <2> is 0.27 D, 0.27 N, and 0.027 each of AEFGHIKLMPQRSTVWY; <4> is an equimolar mixture of amino acid residues ADEFGHIKLMNPQRSTVW; and <5> is 0.36 S and 0.0355 each of ADEFGHIKLMNPQRTVWY;
(b) <5>SY<1><5>S<5><1><4>V,
wherein <1> is an equimolar mixture of ADEFGHIKLMNPQRSTVWY; and <4> and <5> are as defined in (1) above; and
(c) a mixture of (a) and (b);
the members of this group being the only kappa light chain CDR3s present in the library.

5. The focused DNA library according to claim 4, where CDR1s (a) and (b) are present in the library in a ratio of 0.67: 0.33.

6. The focused DNA library according to claim 4, wherein CDR3s (a) and (b) are present in the library in an equimolar mixture.

7. The focused DNA library according to any of claims 1 to 6, further comprising variegated DNA sequences that collectively encode at least one of:
(1) heavy chain CDR1 regions selected from the group consisting of:
(a) <I>₁Y₂<1>₃M₄<1>₅,
wherein <1> is an equimolar mixture of each of amino acid residues A, D, E, F, G, H, I, K, L, M, N, P, Q, R, S, T, V, W, and Y;
(b) (S/T)₁ (S/G/X) ₂ (S/G/X)₃Y₄Y₅W₆ (S/G/X)₇,
wherein (S/T) is a 1: 1 mixture of S and T residues, (S/G/X) is a mixture of 0.2025 S, 0.2025 G and 0.035 of each of amino acid residues A, D, E, F, H, I, K, L, M, N, P, Q, R, T, V, W, and Y;
(c) V₁S₂G₃G₄S_{S}I₆S₇<I>₈<I>₉<I>₁₀Y₁₁Y₁₂W₁₃<I>₁₄,
wherein <1> is an equimolar mixture of each of amino acid residues A, D, E, F, G, H, I, K, L, M, N, P, Q, R, S, T, V, W, and Y; and
(d) a mixture of any of (a)-(c) set forth above;
the members of this group being the only heavy chain CDR1s present in the library;
(2) heavy chain CDR2 regions selected from the group consisting of:
(a) <2>I<2><3>SGG<1>T<1>YADSVKG,
wherein <1> is an equimolar mixture of each of amino acid residues A, D, E, F, G, H, I, K, L, M, N, P, Q, R, S, T, V, W, and Y; <2> is an equimolar mixture of each of amino acid residues Y, R, W, V, G, and S; and <3> is an equimolar mixture of each of amino acid residues P, S, and G or an equimolar mixture of P and S;
(b) <1>I<4><1><1><G><5><1><1><1>YADSVKG,
wherein <1> is an equimolar mixture of each of amino acid residues A, D, E, F, G, H, I, K, L, M, N, P, Q, R, S, T, V, W, and Y; <4> is an equimolar mixture of residues D, I, N, S, W, Y; and <5> is an equimolar mixture of residues S, G, D and N;
(c) <1>I<4><1><1>G<S><I><1>YNPSLKG,
wherein <1> is an equimolar mixture of each of amino acid residues A, D, E, F, G, H, I, K, L, M, N, P, Q, R, S, T, V, W and Y; and <4> and <5> are as defined above;
(d) <1>I<8>S<1><1><1>GGYY<1>YAASVKG,
wherein <1> is an equimolar mixture of each amino acid residues A, D, E, F, G, H, I, K, L, M, N, P, Q, R, S, T, V, W and Y; <8> is 0.27 R and 0.027 of each of ADEFGHIKLMNPQSTVWY; and
(e) a mixture of any of (a) to (d) set forth above;
the members of this group being the only heavy chain CDR2s present in the library; and
(3) heavy chain CDR3 regions selected from the group consisting of:
(a) YYCA21111YFDYWG,
wherein 1 is an equimolar mixture of each amino acid residues A, D, E, F, G, H, I, K, L, M, N, P, Q, R, S, T, V, W and Y; and 2 is an equimolar mixture of K and R;
(b) YYCA2111111YFDYWG,
wherein 1 is an equimolar mixture of each amino acid residues A, D, E, F, G, H, I, K, L, M, N, P, Q, R, S, T, V, W and Y; and 2 is an equimolar mixture of K and R;
(c) YYCA211111111YFDYWG,
wherein 1 is an equimolar mixture of each amino acid residues A, D, E, F, G, H, I, K, L, M, N, P, Q, R, S, T, V, W and Y; and 2 is an equimolar mixture of K and R;
(d) YYCAR111S2S3111YFDYWG,
wherein 1 is an equimolar mixture of each amino acid residues A, D, E, F, G, H, I, K, L, M, N, P, Q, R, S, T, V, W and Y; and 2 is an equimolar mixture of S and G; and 3 is an equimolar mixture of Y and W;
(e) YYCA2111CSG11CY1YFDYWG,
wherein 1 is an equimolar mixture of each amino acid residues A, D, E, F, G, H, I, K, L, M, N, P, Q, R, S, T, V, W and Y; and 2 is an equimolar mixture of K and R;
(f) YYCA211S1TIFG11111YFDYWG,
wherein 1 is an equimolar mixture of each amino acid residues A, D, E, F, G, H, I, K, L, M, N, P, Q, R, S, T, V, W and Y; and 2 is an equimolar mixture of K and R;
(g) YYCAR111YY2S3344111YFDYWG,
wherein 1 is an equimolar mixture of each amino acid residues A, D, E, F, G, H, I, K, L, M, N, P, Q, R, S, T, V, W and Y; 2 is an equimolar mixture of D and G; and 3 is an equimolar mixture of S and G;
(h) YYCAR1111YC2231CY111YFDYWG,
wherein 1 is an equimolar mixture of each amino acid residues A, D, E, F, G, H, I, K, L, M, N, P, Q, R, S, T, V, W and Y; 2 is an equimolar mixture of S and G ; and 3 is an equimolar mixture of T, D and G; and
(i) a mixture of any of the (a) to (h) set forth above;
the members of this group being the only heavy chain CDR3s present in the library.

8. The focused DNA library according to claim 7, wherein heavy chain CDR1s (a), (b) and (c) are present in the library in the ratio 0.80:0.17:0.02.

9. The focused DNA library according to claim 7, wherein a mixture of heavy chain CDR2s (a)/ (b) (equimolar), (c) and (d) are present in the library in a ratio of 0.54: 0.43: 0.03.

10. The focused DNA library of any of claims 1-9, which is a library of vectors.

11. The focused DNA library of claim 10, wherein the vectors are yeast vectors.

12. The focused DNA library of any one of claims 1-9, which is a library of genetic packages.

13. The focused DNA library of claim 12, wherein the genetic packages are filamentous phages or yeast cells.

14. The focused DNA library of claim 13, wherein the genetic packages are yeast cells that display the polypeptides encoded by the variegated DNA sequences in the library.

15. The focused DNA library of any of claims 1-14, wherein the library is for use in a screen for human antibodies, which optionally are single chain Fv (scFv) antibodies, Fv antibodies, Fab antibodies, whole antibodies, or miniantibodies.

## Patentansprüche

1. Fokussierte DNA-Bibliothek, umfassend variierte DNA-Sequenzen von Antikörpern, die kollektiv kodieren:
(1) kappa leichte Kette CDR1-Regionen, ausgewählt aus der Gruppe bestehend aus:
(a) RASQ<1>V<2><2><3>LA
(b) RASQ<1>V<2><2><2><3>LA;
wobei <1> ein äquimolares Gemisch von Aminosäureresten ADEFGHIKLMNPQRSTVWY ist; <2> 0,2 S und 0,044 von jedem von ADEFGHIKLMNPQRTVWY ist; und <3> 0,2 Y und 0,044 von jedem von ADEFGHIKLMNPQRSTVW ist; und
(c) einem Gemisch von vorstehend dargelegtem (a) und (b);
wobei die Mitglieder dieser Gruppe die einzigen in der Bibliothek vorhandenen kappa leichte Ketten CDR1 sind;
(2) kappa leichte Kette CDR2-Regionen mit der Sequenz:
<1>AS<2>R<4><1>,
wobei <1> ein äquimolares Gemisch von Aminosäureresten ADEFGHIKLMNPQRSTVWY ist; <2> 0,2 S und 0,044 von jedem von ADEFGHIKLMNPQRTVWY ist; und <4> 0,2 A und 0,044 von jedem von DEFGHIKLMNPQRSTVWY ist;
wobei die Mitglieder dieser Gruppe die einzigen in der Bibliothek vorhandenen kappa leichte Ketten CDR2 sind; und
(3) kappa leichte Kette CDR3-Regionen, ausgewählt aus den Gruppen bestehend aus:
(a) QQ<3><1><1><1>P<1>T,
wobei <1> ein äquimolares Gemisch der Aminosäurereste ADEFGHIKLMNPQRSTVWY ist; <3> 0,2 Y und 0,044 von jedem von ADEFGHIKLMNPQRTVW ist;
(b) QQ33111P,
wobei 1 und 3 wie vorstehend in (1) definiert sind;
(c) QQ3211PP1T,
wobei 1 und 3 wie vorstehend in (1) definiert sind und 2 0,2 S und 0,044 von jedem von ADEFGHIKLMNPQRTVWY ist; und
(d) einer Mischung von beliebigen der vorstehend dargelegten (a) bis (c); wobei die Mitglieder dieser Gruppe die einzigen in der Bibliothek vorhandenen kappa leichte Ketten CDR3 sind.

2. Fokussierte DNA-Bibliothek nach Anspruch 1, wobei CDRI (a) und (b) in der Bibliothek in einem Verhältnis von 0,68:0,32 vorhanden sind.

3. Fokussierte DNA-Bibliothek nach Anspruch 1, wobei CDR3 (a), (b) und (c) in der Bibliothek in einem Verhältnis von 0,65: 0,1: 0,25 vorhanden sind.

4. Fokussierte DNA-Bibliothek nach einem beliebigen der Ansprüche 1-3, des Weiteren umfassend variierte DNA-Sequenzen, die kollektiv kodieren
(1) lambda leichte Kette CDR1-Regionen, ausgewählt aus der Gruppe bestehend aus:
(a) TG<1>SS<2>VG<1><3><2><3>VS,
wobei <1> 0,27 T, 0,27 G und 0,027 von jedem von ADEFHIKLMNPQRSVWY ist, <2> 0,27 D, 0,27 N und 0,027 von jedem von AEFGHIKLMPQRSTVWY ist, und <3> 0,36 Y und 0,036 von jedem von ADEFGHIKLMNPQRSTVW ist;
(b) G<2><4>L<4><4><4><3><4><4>, wobei <2> wie vorstehend in (1) definiert ist und <4> eine äquimolare Mischung von Aminosäureresten ADEFGHIKLMNPQRSTVWY ist; und
(c) einer Mischung von vorstehend dargelegtem (a) und (b);
wobei die Mitglieder dieser Gruppe die einzigen in der Bibliothek vorhandenen lambda leichte Ketten CDR1 sind;
(2) lambda leichte Kette CDR2-Regionen mit der Sequenz:
<4><4><4><2>RPS,
wobei <2> 0,27 D, 0,27 N und 0,027 von jedem von AEFGHIKLMPQRSTVWY ist und <4> eine äquimolare Mischung von Aminosäureresten ADEFGHIKLMNPQRSTVW ist;
wobei die Mitglieder dieser Gruppe die einzigen in der Bibliothek vorhandenen lambda leichte Ketten CDR2 sind; und
(3) lambda leichte Kette CDR3-Regionen, ausgewählt aus der Gruppe bestehend aus:
(a) <4><5><4><2><4>S<4><4><4><4>V,
wobei <2> 0,27 D, 0,27 N, und 0,027 von jedem von AEFGHIKLMPQRSTVWY ist; <4> eine äquimolare Mischung von Aminosäureresten ADEFGHIKLMNPQRSTVW ist; und <5> 0,36 S und 0,0355 von jedem von ADEFGHIKLMNPQRTVWY ist;
(b) <5>SY<1><5>S<5><1><4>V,
wobei <1> eine äquimolare Mischung von ADEFGHIKLMNPQRSTVWY ist; und <4> und <5> wie vorstehend in (1) definiert sind; und
(c) einer Mischung von (a) und (b);
wobei die Mitglieder dieser Gruppe die einzigen in der Bibliothek vorhandenen lambda leichte Ketten CDR3 sind.

5. Fokussierte DNA-Bibliothek nach Anspruch 4, wobei CDR1 (a) und (b) in der Bibliothek in einem Verhältnis von 0,67: 0,33 vorhanden sind.

6. Fokussierte DNA-Bibliothek nach Anspruch 4, wobei CDR3 (a) und (b) in der Bibliothek in einer äquimolaren Mischung vorhanden sind.

7. Fokussierte DNA-Bibliothek nach einem beliebigen der Ansprüche 1 bis 6, des Weiteren umfassend variierte DNA-Sequenzen, die kollektiv mindestens eines kodieren von:
(1) schwere Kette CDR1-Regionen, ausgewählt aus der Gruppe bestehend aus:
(a) <I>₁Y₂<1>₃M₄<1>₅,
wobei <1> eine äquimolare Mischung von jedem der Aminosäurereste A, D, E, F, G, H, I, K, L, M, N, P, Q, R, S, T, V, W, und Y ist;
(b) (S/T) ₁ (S/G/X) ₂ (S/G/X)₃Y₄Y₅W₆ (S/G/X)₇,
wobei (S/T) eine 1: 1 Mischung von S und T-Resten ist, (S/G/X) eine Mischung von 0,2025 S, 0,2025 G und 0,035 von jedem der Aminosäurereste A, D, E, F, H, I, K, L, M, N, P, Q, R, T, V, W, und Y ist;
(c) V₁S₂G₃G₄S₅I₆S₇<I>₈<I>₉<I>₁₀Y₁₁Y₁₂W₁₃<I>₁₄,
wobei <1> eine äquimolare Mischung von jedem der Aminosäurereste A, D, E, F, G, H, I, K, L, M, N, P, Q, R, S, T, V, W, und Y ist; und
(d) einer Mischung von beliebigen der vorstehend dargelegten (a)-(c);
wobei die Mitglieder dieser Gruppe die einzigen in der Bibliothek vorhandenen schwere Ketten CDR1 sind;
(2) schwere Kette CDR2-Regionen, ausgewählt aus der Gruppe bestehend aus:
(a) <2>I<2><3>SGG<1>T<1>YADSVKG,
wobei <1> eine äquimolare Mischung von jedem der Aminosäurereste A, D, E, F, G, H, I, K, L, M, N, P, Q, R, S, T, V, W, und Y ist; <2> eine äquimolare Mischung von jedem der Aminosäurereste Y, R, W, V, G, und S ist; und <3> eine äquimolare Mischung von jedem der Aminosäurereste P, S, und G oder eine äquimolare Mischung von P und S ist;
(b) <1>I<4><1><1><G><5><1><1><1>YADSVKG,
wobei <1> eine äquimolare Mischung von jedem der Aminosäurereste A, D, E, F, G, H, I, K, L, M, N, P, Q, R, S, T, V, W, und Y ist; <4> eine äquimolare Mischung der Reste D, I, N, S, W, Y ist; und <5> eine äquimolare Mischung der Reste S, G, D und N ist;
(c) <1>I<4><1><1>G<5><I><1>YNPSLKG,
wobei <1> eine äquimolare Mischung von jedem der Aminosäurereste A, D, E, F, G, H, I, K, L, M, N, P, Q, R, S, T, V, W und Y ist; und <4> und <5> wie vorstehend definiert sind;
(d) <1>I<8>S<1><1><1>GGYY<1>YAASVKG,
wobei <1> eine äquimolare Mischung von jedem der Aminosäurereste A, D, E, F, G, H, I, K, L, M, N, P, Q, R, S, T, V, W und Y ist; <8> 0,27 R und 0,027 von jedem von ADEFGHIKLMNPQSTVWY ist; und
(e) einer Mischung von beliebigen der vorstehend dargelegten (a) bis (d);
wobei die Mitglieder dieser Gruppe die einzigen in der Bibliothek vorhandenen schwere Ketten CDR2 sind; und
(3) schwere Kette CDR3-Regionen, ausgewählt aus der Gruppe bestehend aus:
(a) YYCA21111YFDYWG,
wobei 1 eine äquimolare Mischung von jedem Aminosäurerest A, D, E, F, G, H, I, K, L, M, N, P, Q, R, S, T, V, W und Y ist; und 2 eine äquimolare Mischung von K und R ist;
(b) YYCA2111111YFDYWG,
wobei 1 eine äquimolare Mischung von jedem der Aminosäurereste A, D, E, F, G, H, I, K, L, M, N, P, Q, R, S, T, V, W und Y ist; und 2 eine äquimolare Mischung von K und R ist;
(c) YYCA211111111YFDYWG,
wobei 1 eine äquimolare Mischung von jedem der Aminosäurereste A, D, E, F, G, H, I, K, L, M, N, P, Q, R, S, T, V, W und Y ist; und 2 eine äquimolare Mischung von K und R ist;
(d) YYCAR111S2S3111YFDYWG,
wobei 1 eine äquimolare Mischung von jedem der Aminosäurereste A, D, E, F, G, H, I, K, L, M, N, P, Q, R, S, T, V, W und Y ist; und 2 eine äquimolare Mischung von S und G ist; und 3 eine äquimolare Mischung von Y und W ist;
(e) YYCA2111CSG11CY1YFDYWG,
wobei 1 eine äquimolare Mischung von jedem der Aminosäurereste A, D, E, F, G, H, I, K, L, M, N, P, Q, R, S, T, V, W und Y; und 2 eine äquimolare Mischung von K und R ist;
(f) YYCA211S1TIFG11111YFDYWG,
wobei 1 eine äquimolare Mischung von jedem der Aminosäurereste A, D, E, F, G, H, I, K, L, M, N, P, Q, R, S, T, V, W und Y ist; und 2 eine äquimolare Mischung von K und R ist;
(g) YYCAR111YY2S3344111YFDYWG,
wobei 1 eine äquimolare Mischung von jedem der Aminosäurereste A, D, E, F, G, H, I, K, L, M, N, P, Q, R, S, T, V, W und Y ist; 2 eine äquimolare Mischung von D und G ist; und 3 eine äquimolare Mischung von S und G ist;
(h) YYCAR1111YC2231CY111YFDYWG,
wobei 1 eine äquimolare Mischung von jedem der Aminosäurereste A, D, E, F, G, H, I, K, L, M, N, P, Q, R, S, T, V, W und Y ist; 2 eine äquimolare Mischung von S und G ist; und 3 eine äquimolare Mischung von T, D und G ist; und
(i) einer Mischung von beliebigen der vorstehend dargelegten (a) bis (h);
wobei die Mitglieder dieser Gruppe die einzigen in der Bibliothek vorhandenen schwere Ketten CDR3 sind.

8. Fokussierte DNA-Bibliothek nach Anspruch 7, wobei schwere Kette CDR1 (a), (b) und (c) in der Bibliothek in dem Verhältnis 0,80:0,17:0,02 vorhanden sind.

9. Fokussierte DNA-Bibliothek nach Anspruch 7, wobei eine Mischung der schweren Kette CDR2 (a)/ (b) (äquimolar), (c) und (d) in der Bibliothek in einem Verhältnis von 0,54: 0,43: 0,03 vorhanden sind.

10. Fokussierte DNA-Bibliothek nach einem beliebigen der Ansprüche 1-9, die eine Bibliothek von Vektoren ist.

11. Fokussierte DNA-Bibliothek nach Anspruch 10, wobei die Vektoren Hefevektoren sind.

12. Fokussierte DNA-Bibliothek nach einem beliebigen der Ansprüche 1-9, die eine Bibliothek von genetischen Verpackungen ist.

13. Fokussierte DNA-Bibliothek nach Anspruch 12, wobei die genetischen Verpackungen filamentöse Phagen oder Hefezellen sind.

14. Fokussierte DNA-Bibliothek nach Anspruch 13, wobei die genetischen Verpackungen Hefezellen sind, die die durch die variierten DNA-Sequenzen in der Bibliothek kodierten Polypeptide präsentieren.

15. Fokussierte DNA-Bibliothek nach einem beliebigen der Ansprüche 1-14, wobei die Bibliothek für die Verwendung in einem Screening auf humane Antikörper ist, die gegebenenfalls single chain Fv (scFv) Antikörper, Fv Antikörper, Fab Antikörper, ganze Antikörper, oder Miniantikörper sind.

## Revendications

1. Banque d'ADN ciblée comprenant des séquences d'ADN bigarrées d'anticorps qui codent collectivement pour :
(1) des régions CDR1 de chaîne légère kappa choisies dans le groupe constitué par :
(a) RASQ<1>V<2><2><3>LA
(b) RASQ<1>V<2><2><2><3>LA ;
dans laquelle <1> est un mélange équimolaire de résidus d'acide aminé ADEFGHIKLMNPQRSTVWY ; <2> est 0,2 S et 0,044 de chacun de ADEFGHIKLMNPQRTVWY ; et <3> est 0,2 Y et 0,044 de chacun de ADEFGHIKLMNPQRSTVW ; et
(c) un mélange de (a) et (b) indiqués ci-dessus ;
les membres de ce groupe étant les seules CDR1 de chaîne légère kappa présentes dans la banque ;
(2) des régions CDR2 de chaîne légère kappa ayant la séquence :
<1>AS<2>R<4><1>,
dans laquelle <1> est un mélange équimolaire de résidus d'acide aminé ADEFGHIKLMNPQRSTVWY ; <2> est 0,2 S et 0,044 de chacun de ADEFGHIKLMNPQRTVWY ; et <4> est 0,2 A et 0,044 de chacun de DEFGHIKLMNPQRSTVWY ;
les membres de ce groupe étant les seules CDR2 de chaîne légère kappa présentes dans la banque ; et
(3) des régions CDR3 de chaîne légère kappa choisies dans les groupes constitués par :
(a) QQ<3><1><1><1>P<1>T,
dans laquelle <1> est un mélange équimolaire de résidus d'acide aminé ADEFGHIKLMNPQRSTVWY ; <3> est 0,2 Y et 0,044 de chacun de ADEFGHIKLMNPQRTVW ;
(b) QQ33111P,
dans laquelle 1 et 3 sont tels que définis en (1) ci-dessus ;
(c) QQ3211PP1T,
dans laquelle 1 et 3 sont tels que définis en (1) ci-dessus et 2 est 0,2 S et 0,044 de chacun de ADEFGHIKLMNPQRTVWY ; et
(d) un mélange de l'un quelconque de (a) à (c) indiqués ci-dessus ;
les membres de ce groupe étant les seules CDR3 de chaîne légère kappa présentes dans la banque.

2. Banque d'ADN ciblée selon la revendication 1, dans laquelle les CDRI (a) et (b) sont présentes dans la banque dans un rapport de 0,68:0,32.

3. Banque d'ADN ciblée selon la revendication 1, dans laquelle les CDR3 (a), (b) et (c) sont présentes dans la banque dans un rapport de 0,65:0,1:0,25.

4. Banque d'ADN ciblée selon l'une quelconque des revendications 1 à 3, comprenant en outre des séquences d'ADN bigarrées codant collectivement pour
(1) des régions CDR1 de chaîne légère lambda choisies dans le groupe constitué par :
(a) TG<1>SS<2>VG<1><3><2><3>VS,
dans laquelle < 1 > est 0,27 T, 0,27 G et 0,027 de chacun de ADEFHIKLMNPQRSVWY, <2> est 0,27 D, 0,27 N et 0,027 de chacun de AEFGHIKLMPQRSTVWY, et <3> est 0,36 Y et 0,036 de chacun de ADEFGHIKLMNPQRSTVW ;
(b) G<2><4>L<4><4><4><3><4><4>, dans laquelle <2> est tel que défini en (1) ci-dessus et <4> est un mélange équimolaire de résidus d'acide aminé ADEFGHIKLMNPQRSTVWY ; et
(c) un mélange de (a) et (b) indiqués ci-dessus ;
les membres de ce groupe étant les seules CDR1 de chaîne légère lambda présentes dans la banque ;
(2) des régions CDR2 de chaîne légère lambda, ayant la séquence :
<4><4><4><2>RPS,
dans laquelle <2> est 0,27 D, 0,27 N, et 0,027 de chacun de AEFGHIKLMPQRSTVWY et <4> est un mélange équimolaire de résidus d'acide aminé ADEFGHIKLMNPQRSTVW ;
les membres de ce groupe étant les seules CDR2 de chaîne légère lambda présentes dans la banque ; et
(3) des régions CDR3 de chaîne légère lambda choisies dans le groupe constitué par :
(a) <4><5><4><2><4>S<4><4><4><4>V,
dans laquelle <2> est 0,27 D, 0,27 N, et 0,027 de chacun de AEFGHIKLMPQRSTVWY ; <4> est un mélange équimolaire de résidus d'acide aminé ADEFGHIKLMNPQRSTVW ; et <5> est 0,36 S et 0,0355 de chacun de ADEFGHIKLMNPQRTVWY ;
(b) <5>SY<1><5>S<5><1><4>V,
dans laquelle <1> est un mélange équimolaire de ADEFGHIKLMNPQRSTVWY ; et <4> et <5> sont tels que définis en (1) ci-dessus ; et
(c) un mélange de (a) et (b) ;
les membres de ce groupe étant les seules CDR3 de chaîne légère kappa présentes dans la banque.

5. Banque d'ADN ciblée selon la revendication 4, où les CDR1 (a) et (b) sont présentes dans la banque dans un rapport de 0,67:0,33.

6. Banque d'ADN ciblée selon la revendication 4, dans laquelle les CDR3 (a) et (b) sont présentes dans la banque en un mélange équimolaire.

7. Banque d'ADN ciblée selon l'une quelconque des revendications 1 à 6, comprenant en outre des séquences d'ADN bigarrées qui codent collectivement pour au moins une parmi :
(1) des régions CDR1 de chaîne lourde choisies dans le groupe constitué par :
(a) <I>₁Y₂<1>₃M₄<1>₅,
dans laquelle <1> est un mélange équimolaire de chacun des résidus d'acide aminé A, D, E, F, G, H, I, K, L, M, N, P, Q, R, S, T, V, W, et Y ;
(b) (S/T)₁ (S/G/X)₂ (S/G/X)₃Y₄Y₅W₆ (S/G/X)₇,
dans laquelle (S/T) est un mélange 1:1 1 de résidus S et T, (S/G/X) est un mélange de 0,2025 S, 0,2025 G et 0,035 de chacun des résidus d'acide aminé A, D, E, F, H, I, K, L, M, N, P, Q, R, T, V, W, et Y ;
(c) V₁S₂G₃G₄S₅I₆S₇<I>₈<I>₉<I>₁₀Y₁₁Y₁₂W₁₃<I>₁₄,
dans laquelle <1> est un mélange équimolaire de chacun des résidus d'acide aminé A, D, E, F, G, H, I, K, L, M, N, P, Q, R, S, T, V, W et Y ; et
(d) un mélange de l'un quelconque de (a) à (c) indiqués ci-dessus ;
les membres de ce groupe étant les seules CDR1 de chaîne lourde présentes dans la banque ;
(2) des régions CDR2 de chaîne lourde choisies dans le groupe constitué par :
(a) <2>I<2><3>SGG<1>T<1>YADSVKG,
dans laquelle < 1 > est un mélange équimolaire de chacun des résidus d'acide aminé A, D, E, F, G, H, I, K, L, M, N, P, Q, R, S, T, V, W, et Y ; <2> est un mélange équimolaire de chacun des résidus d'acide aminé Y, R, W, V, G, et S ; et <3> est un mélange équimolaire de chacun des résidus d'acide aminé P, S, et G, ou un mélange équimolaire de P et S ;
(b) <1> 1 <4> <1> <1> <G> <5> <1> <1><1> YADSVKG,
dans laquelle <1> est un mélange équimolaire de chacun des résidus d'acide aminé A, D, E, F, G, H, I, K, L, M, N, P, Q, R, S, T, V, W, et Y ; <4> est un mélange équimolaire de résidus D, I, N, S, W, Y ; et <5> est un mélange équimolaire de résidus S, G, D et N ;
(c) <1>I<4><1><1>G<5><1><1>YNPSLKG,
dans laquelle <1> est un mélange équimolaire de chacun des résidus d'acide aminé A, D, E, F, G, H, I, K, L, M, N, P, Q, R, S, T, V, W et Y ; et <4> et <5> sont tels que définis ci-dessus ;
(d) <1>1<8>S<1><1><1>GGYY<1>YAASVKG,
dans laquelle <1> est un mélange équimolaire de chacun des résidus d'acide aminé A, D, E, F, G, H, I, K, L, M, N, P, Q, R, S, T, V, W et Y ; <8> est 0,27 R et 0,027 de chacun de ADEFGHIKLMNPQSTVWY ; et
(e) un mélange de l'un quelconque de (a) à (d) indiqués ci-dessus ;
les membres de ce groupe étant les seules CDR2 de chaîne lourde présentes dans la banque ; et
(3) des régions CDR3 de chaîne lourde choisies dans le groupe constitué par :
(a) YYCA21111YFDYWG,
dans laquelle 1 est un mélange équimolaire de chacun des résidus d'acide aminé A, D, E, F, G, H, I, K, L, M, N, P, Q, R, S, T, V, W et Y ; et 2 est un mélange équimolaire de K et R ;
(b) YYCA2111111YFDYWG,
dans laquelle 1 est un mélange équimolaire de chacun des résidus d'acide aminé A, D, E, F, G, H, I, K, L, M, N, P, Q, R, S, T, V, W et Y ; et 2 est un mélange équimolaire de K et R ;
(c) YYCA211111111YFDYWG,
dans laquelle 1 est un mélange équimolaire de chacun des résidus d'acide aminé A, D, E, F, G, H, I, K, L, M, N, P, Q, R, S, T, V, W et Y ; et 2 est un mélange équimolaire de K et R ;
(d) YYCAR111S2S3111YFDYWG,
dans laquelle 1 est un mélange équimolaire de chacun des résidus d'acide aminé A, D, E, F, G, H, I, K, L, M, N, P, Q, R, S, T, V, W et Y ; et 2 est un mélange équimolaire de S et G ; et 3 est un mélange équimolaire de Y et W ;
(e) YYCA2111CSG11CY1YFDYWG,
dans laquelle 1 est un mélange équimolaire de chacun des résidus d'acide aminé A, D, E, F, G, H, I, K, L, M, N, P, Q, R, S, T, V, W et Y ; et 2 est un mélange équimolaire de K et R ;
(f) YYCA211S1TIFG11111YFDYWG,
dans laquelle 1 est un mélange équimolaire de chacun des résidus d'acide aminé A, D, E, F, G, H, I, K, L, M, N, P, Q, R, S, T, V, W et Y ; et 2 est un mélange équimolaire de K et R ;
(g) YYCAR111YY2S3344111YFDYWG,
dans laquelle 1 est un mélange équimolaire de chacun des résidus d'acide aminé A, D, E, F, G, H, I, K, L, M, N, P, Q, R, S, T, V, W et Y ; 2 est un mélange équimolaire de D et G ; et 3 est un mélange équimolaire de S et G ;
(h) YYCAR1111YC2231CY111YFDYWG,
dans laquelle 1 est un mélange équimolaire de chacun des résidus d'acide aminé A, D, E, F, G, H, I, K, L, M, N, P, Q, R, S, T, V, W et Y ; 2 est un mélange équimolaire de S et G ; et 3 est un mélange équimolaire de T, D et G ; et
(i) un mélange de l'un quelconque de (a) à (h) indiqués ci-dessus ;
les membres de ce groupe étant les seules CDR3 de chaîne lourde présentes dans la banque.

8. Banque d'ADN ciblée selon la revendication 7, dans laquelle les CDR1 de chaîne lourde (a), (b) et (c) sont présentes dans la banque dans le rapport de 0,80:0,17:0,02.

9. Banque d'ADN ciblée selon la revendication 7, dans laquelle un mélange de CDR2 de chaîne lourde (a)/(b) (équimolaire), (c) et (d) sont présentes dans la banque dans un rapport de 0,54:0,43:0,03.

10. Banque d'ADN ciblée selon l'une quelconque des revendications 1 à 9, qui est une banque de vecteurs.

11. Banque d'ADN ciblée selon la revendication 10, dans laquelle les vecteurs sont des vecteurs de type levure.

12. Banque d'ADN ciblée selon l'une quelconque des revendications 1 à 9, qui est une banque d'empaquetages génétiques.

13. Banque d'ADN ciblée selon la revendication 12, dans laquelle les empaquetages génétiques sont des phages filamenteux ou des cellules de levure.

14. Banque d'ADN ciblée selon la revendication 13, dans laquelle les empaquetages génétiques sont des cellules de levure qui affichent les polypeptides codés par les séquences d'ADN bigarrées dans la banque.

15. Banque d'ADN ciblée selon l'une quelconque des revendications 1 à 14, dans laquelle la banque est destinée à une utilisation dans un criblage d'anticorps humains, qui sont éventuellement des anticorps Fv à chaîne unique (scFv), des anticorps Fv, des anticorps Fab, des anticorps entiers, ou des minianticorps.
